(19) **Europäisches Patentamt / European Patent Office / Office européen des brevets**

(11) **EP 2 912 160 B1**

(12) **EUROPEAN PATENT SPECIFICATION**

(45) Date of publication and mention of the grant of the patent:
**29.07.2020 Bulletin 2020/31**

(21) Application number: **13786587.9**

(22) Date of filing: **23.10.2013**

(51) Int Cl.:
***C12M 1/24*** *(2006.01)*   ***C12M 1/00*** *(2006.01)*

(86) International application number:
**PCT/US2013/066410**

(87) International publication number:
**WO 2014/066519 (01.05.2014 Gazette 2014/18)**

(54) **PERFUSION CULTURING METHODS AND USES THEREOF**

PERFUSIONSKULTIVIERUNGSVERFAHREN UND VERWENDUNGEN DAVON

PROCÉDÉS DE CULTURE SOUS PERFUSION ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **23.10.2012 US 201261717486 P**

(43) Date of publication of application:
**02.09.2015 Bulletin 2015/36**

(73) Proprietor: **Genzyme Corporation**
**Cambridge, MA 02142 (US)**

(72) Inventors:
• **YANG, Jianguo**
**Bridgewater, New Jersey 08807 (US)**
• **VILLIGER-OBERBEK, Agata**
**Bridgewater, New Jersey 08807 (US)**
• **YANG, Yang**
**Bridgewater, New Jersey 08807 (US)**

(74) Representative: **Lavoix**
**Bayerstrasse 83**
**80335 München (DE)**

(56) References cited:
**WO-A1-2006/138143    WO-A2-2009/034186**

• **JURE STRNAD ET AL: "Optimization of cultivation conditions in spin tubes for Chinese hamster ovary cells producing erythropoietin and the comparison of glycosylation patterns in different cultivation vessels", BIOTECHNOLOGY PROGRESS, vol. 26, no. 3, 29 January 2010 (2010-01-29), pages 653-663, XP055091106, ISSN: 8756-7938, DOI: 10.1002/btpr.390**

**Description**

**CROSS-REFERENCE TO RELATED APPLICATIONS**

This application claims priority to U.S. Provisional Patent Application Serial No. 61/717,486, filed October 23, 2012.

**TECHNICAL FIELD**

This application relates to methods of molecular biology, cell culture process development, and the manufacture of recombinant proteins.

**BACKGROUND**

Mammalian cells containing a nucleic acid that encodes a recombinant protein are often used to produce therapeutically or commercially important proteins. Although several high throughput (HT) cell culture systems have been used within the biotechnology industry for fed-batch processes for years, no HT model for a perfusion-based cell culture is known to exist.

The current shake tube methods of culturing mammalian cells use a shaking diameter of 50 mm orbit (throw), a shaking speed of 180 RPM, a shaking angle of 90° (i.e., 90° from horizontal or a reactor angle of 90°), and a 10 mL working volume (w.v.). These current shake tube methods have been reported to promote growth of Chinese hamster ovary (CHO) cells up to 20 x 10$^6$ cells/mL in a non-instrumented (without controllers) culture under batch conditions (e.g., Sartorius website).

Document WO2006/138143 discloses a cell culture vessel comprising a housing defining a chamber that includes an inverted frusto-conical bottom or cone-shaped roundbottom configured lower section, the chamber having a vertical axis, and the use thereof in a method of culturing living cells.

Document WO2009/034186 discloses a process for use in the production of a structurally stable porous matrix material, the inner surfaces of which are at least partially colonized by adhering cells, the process involving the cultivation of mammalian cells in a bioreactor.

Jure Strnad (2010, Biotechnol. Prog., vol. 26, no. 3, 653-663) discloses the optimization of cultivation condition in spin tubes for Chinese Hamster Ovary cells producing erythropoietin and the comparison of glycosylation patterns in differents cultivation vessels.

**SUMMARY**

The present invention is defined in the claims.

The present invention is based, at least in part, on the discovery that culturing a mammalian cell in the specific manner described herein results in a substantially improved viable cell density and recombinant protein production. Thus, the present specification discloses (in vitro) methods of culturing a mammalian cell that include providing a conical container containing a mammalian cell suspended in about 4% to about 80% (e.g., in about 4% to about 70%, in about 4% to about 60%, or in about 4% to about 30%) of the volume of the container (e.g., a container having a volume greater than 2 mL, e.g., a volume of between about 2 mL to about 600 mL, between about 2 mL and about 2 L, or between about 2 mL and about 3 L) of a first liquid culture medium, incubating the container over a period of time at about 31°C to about 40 °C at a reactor angle of about 5 degrees to about 85 degrees (e.g., about 10 degrees to about 85 degrees, about 15 degrees to about 85 degrees, about 5 degrees to about 65 degrees, or about 35 degrees to about 50 degrees) from horizontal and with an agitation of about 20 RPM to about 1000 RPM (e.g., about 20 RPM to about 400 RPM, about 120 RPM to about 240 RPM, about 140 RPM to about 220 RPM, about 160 RPM to about 180 RPM, about 400 RPM to about 600 RPM, about 600 RPM to about 800 RPM, or about 800 RPM to about 1000 RPM) (e.g., in an incubator, such as a shake incubator with throw (orbit) diameter from about 3 mm to about 50 mm), and continuously or periodically, during the period of time, removing a first volume of the first liquid culture medium (having any mammalian cell density, e.g., substantially free of mammalian cells) and adding to the first liquid culture medium a second volume of a second liquid culture medium, e.g., wherein the first and second volumes are about 70% to about 90% equal, where the first and second volumes are about equal (e.g., within about 0.1% to about 3% equal, within about 0.1% to about 2% equal, within about 1% to about 5% equal, within about 5% to about 10% equal, within about 10% equal, or absolutely equal). In some disclosed embodiments, the first and second liquid culture medium can be the same medium. In some disclosed embodiments, the first and second liquid culture medium can be different (e.g., a different medium or a different concentration). As is appreciated in the art, the level of agitation (e.g., RPM speed) can be varied depending upon the size and shape of the container (e.g., the diameter of the container) and the throw (orbit) diameter of the incubator that is used to perform the incubating. For example, a smaller throw (orbit) diameter can require a higher level of agitation (e.g., a higher RPM speed), while a larger throw (orbit) diameter can require a lower level of agitation (e.g., a lower RPM speed) to achieve a similar level of fluid sheer force and dissolved $O_2$ concentration. In another example, a container having a larger diameter can require a lower RPM speed, while a container having a smaller diameter can require a higher RPM speed to achieve a similar level of fluid sheer force and dissolved $O_2$ concentration. In some disclosed embodiments, the incubating is performed using a shake tube incubator with a throw (orbit) diameter of between about 25 mm to about 50 mm and an agitation of between about 20 RPM to about 400 RPM (e.g., about 120 RPM to about 240 RPM, about 140 RPM to about 220 RPM, about 160 RPM to about 180 RPM). In some disclosed embodiments, the incubating is performed using a shake tube incubator with a throw (orbit) diameter of about 1 mm to about 25 mm and an agitation of about 20 RPM to about 1000 RPM (e.g., about 100 RPM to about 1000 RPM, about 200 RPM to about 1000 RPM, about 100 RPM to about 200 RPM, about 200 RPM to about 300 RPM, about 300 RPM to about 400 RPM, about 400 RPM to about 500 RPM, about 500 RPM to about 600 RPM, about 600 RPM to about 700 RPM, about 700 RPM to about 800 RPM, about 800 RPM to about 900 RPM, about 900 RPM to about 1000 RPM).

In some disclosed examples, the container is a conical container with a volume of about 40 mL to about 60 mL, the reactor angle is about 3° to about 7°, the agitation is about 65 RPM to about 105 RPM, and the volume of liquid culture medium is about 15% to about 25% of the container volume. In other disclosed examples, the container is a conical container with a volume of about 40 mL to about 60 mL, the reactor angle is about 3° to about 7°, the agitation is about 240 RPM to about 280 RPM, and the volume of liquid culture medium is about 2% to about 9% of the container volume.

In other disclosed methods, the container is a conical container with a volume of about 40 mL to about 60 mL, the reactor angle is about 15° to about 25°, the agitation is about 310 RPM to about 350 RPM, and the volume of liquid culture medium is about 35% to about 45% of the container volume.

In some disclosed examples, the container is a conical container with a volume of about 40 mL to about 60 mL, the reactor angle is about 25° to about 35°, the agitation is about 100 RPM to about 140 RPM, and the volume of liquid culture medium is about 1% to about 9% of the container volume. In other disclosed examples, the container is a conical container with a volume of about 40 mL to about 60 mL, the reactor angle is about 25° to about 35°, the agitation is about 235 RPM to about 275 RPM, and the volume of liquid culture medium is about 27% to about 37% of the container volume.

In some disclosed methods, the container is a conical container with a volume of about 40 mL to about 60 mL, the reactor angle is about 40° to about 50°, the agitation is about 140 RPM to about 180 RPM, and the volume of liquid culture medium is about 15% to about 25% of the container volume.

In other disclosed examples, the container is a conical container with a volume of about 40 mL to about 60 mL, the reactor angle is about 55° to about 65°, the agitation is about 235 RPM to about 275 RPM, and the volume of liquid culture medium is about 1% to about 9% of the container volume. In disclosed other examples, the container is a conical container with a volume of about 40 mL to about 60 mL, the reactor angle is about 55° to about 65°, the agitation is about 168 RPM to about 208 RPM, and the volume of liquid culture medium is about 55% to about 65% of the container volume.

In some disclosed methods, the container is a conical container with a volume of about 40 mL to about 60 mL, the reactor angle is about 60° to about 70°, the agitation is about 230 RPM to about 270 RPM, and the volume of liquid culture medium is about 75% to about 85% of the container volume.

In other disclosed examples, the container is a conical container with a volume of about 40 mL to about 60 mL, the reactor angle is about 75° to about 85°, the agitation is about 310 RPM to about 350 RPM, and the volume of liquid culture medium is about 35% to about 45% of the container volume. In other disclosed examples, the container is a conical container with a volume of about 40 mL to about 60 mL, the reactor angle is about 75° to about 85°, the agitation is about 310 RPM to about 350 RPM, and the volume of liquid culture medium is about 65% to about 75% of the container volume.

In some disclosed embodiments, the second volume of the second liquid culture medium added is less (e.g., at most 1%, 2%, 3%, 4%, 5%, 6%, 7%, 8%, 9%, or 10% less) than the first volume of the first liquid culture medium removed. Also disclosed are methods of culturing a mammalian cell that include culturing in a gradient perfusion process a mammalian cell suspended in a liquid culture medium under conditions that generate in the medium a fluid sheer force and dissolved oxygen ($O_2$) concentration that is the same as (or essentially the same as) that achieved in about 4% to about 80% (e.g., about 4% to about 70%, about 4% to about 60%, or about 4% to about 30%) volume of the container (e.g., a container having a volume of greater than 2 mL or a volume of between about 2 mL to about 600 mL, between about 2 mL to about 2 L, or between about 2 mL to about 3L) of liquid culture medium in a gas-permeable conical container positioned at a reactor angle of about 5 degrees to about 85 degrees (e.g., about 10 degrees to about 85 degrees, about 15 degrees to about 85 degrees, about 5 degrees to about 65 degrees, or about 35 degrees to about 50 degrees) from horizontal, incubated at a temperature of about 31 °C to about 40 °C, and agitated at a frequency of about 20 RPM to about 1000 RPM (e.g., about 20 RPM to about 400 RPM, about 120 RPM to about 240 RPM, about 140 RPM to about 220 RPM, about 160 RPM to about 180 RPM, about 400 RPM to about 600 RPM, about 600 RPM to about 800 RPM, or about 800 RPM to about 1000 RPM) (e.g., in an incubator, such as a shake incubator with throw (orbit) diameter from about 3 mm to about 50 mm). Methods of producing a recombinant protein and methods of testing a manufacturing process for making a recombinant protein that utilize any of the exemplary culturing methods described herein are also disclosed.

Disclosed herein are methods of culturing a mammalian cell that include: providing a conical container containing a mammalian cell suspended in a first liquid culture medium that occupies about 4% to about 80% of the volume of the container; incubating the container for a period of time at about 31 °C to about 40 °C at a reactor angle of about 5 degrees to about 85 degrees from horizontal and with a rotary agitation of about 20 revolutions per minute (RPM) to about 1000 RPM; and continuously or periodically, during the period of time, removing a first volume of the first liquid culture medium and adding to the first liquid culture medium a second volume of a second liquid culture medium, wherein the first and second volumes are about equal. In some embodiments, the first volume of the first liquid culture medium is substantially free of mammalian cells. In some disclosed embodiments, the first liquid culture medium occupies about 4% to about 30% of the volume of the container.

In some disclosed embodiments, the mammalian cell is a Chinese hamster ovary (CHO) cell. In some embodiments, the CHO cell contains a nucleic acid encoding a recombinant protein (e.g., an immunoglobulin, an enzyme, a growth factor, a protein fragment, or an engineered protein).

In some disclosed embodiments, the container is incubated at about 40 degrees to about 55 degrees from horizontal. In some embodiments, the removing of the first volume of the first liquid culture medium and the adding of the second volume of the second liquid culture medium is performed simultaneously. In some embodiments, the removing of the first volume of the first liquid culture medium and the adding of the second volume of the second liquid culture medium is performed continuously. In some embodiments, the removing of the first volume of the first liquid culture medium and the adding of the second volume of the second liquid culture medium is performed periodically. In some embodiments, the first volume of the first liquid culture medium removed and the second volume of the second liquid culture medium added are increased over time.

In some embodiments embodiments, the container is incubated for a period of time greater than 7 days, and on days 1 through 3 of incubation, in each 24-hour period, the first volume of the first liquid culture medium removed and the second volume of the second liquid culture medium added is about 50% of the volume of the first liquid culturing medium; on days 4 through 6 of incubation, in each 24-hour period, the first volume of the first liquid culture medium removed and the second volume of the second liquid culture medium added is about 70% of the volume of the first liquid culture medium; and on day 7 and onwards of incubation, in each 24-hour period, the first volume of the first liquid culture medium removed and the second volume of the second liquid culture medium added is about 100% of the volume of the first liquid culture medium. In some disclosed embodiments, the conical container is a gas-permeable 50-mL to 600-mL conical container. In some embodiments, the mammalian cell is suspended in about 2 mL to about 15 mL of the first liquid culture medium. In some embodiments, the first liquid culture medium and/or second liquid culture medium is selected from the group of: a chemically-defined liquid culture medium, a serum-free liquid culture medium, a serum-containing liquid culture medium, an animal-derived component free liquid culture medium, and a protein-free medium.

Also disclosed are methods of culturing a mammalian cell that include culturing in a gradient perfusion process a mammalian cell suspended in a liquid culture medium under conditions that generate in the medium a fluid sheer force and dissolved oxygen ($O_2$) concentration that is essentially the same as that achieved in a medium occupying 4% to 40% of the volume of a gas-permeable conical container when the container is positioned at a reactor angle of about 5 degrees to about 85 degrees from horizontal, incubated at a temperature of about 31 °C to about 40 °C, and agitated at a frequency of about 20 revolutions per minute (RPM) to about 1000 RPM.

In some disclosed embodiments, the conical container is a gas-permeable 50-mL to 600-mL conical container. In some disclosed embodiments, the mammalian cell is a Chinese hamster ovary (CHO) cell. In some embodiments, the CHO cell contains a nucleic acid encoding a recombinant protein (e.g., an immunoglobulin, an enzyme, a growth factor, a protein fragment, or an engineered protein). In some embodiments, the liquid culture medium is selected from the group consisting of: a chemically-defined liquid culture medium, a serum-free liquid culture medium, a serum-containing liquid culture medium, an animal-derived component free liquid culture medium, or a protein-free medium.

Also disclosed are methods of producing a recombinant protein that include: providing a conical container containing a mammalian cell containing a nucleic acid that encodes a recombinant protein, wherein the cell is suspended in a first liquid culture medium that occupies about 4% to about 80% of the volume of the container; incubating the container for a period of time at about 31 °C to about 40°C at a reactor angle of about 5 degrees to about 85 degrees from horizontal and with a rotary agitation of about 20 revolutions per minute (RPM) to about 1000 RPM; continuously or periodically, during the period of time, removing a first volume of the first liquid culture medium and adding to the first liquid culture medium a second volume of a second liquid culture medium, wherein the first and second volumes are about equal; and recovering the recombinant protein from the mammalian cell or from the first or second culture medium.

In some embodiments, the first volume of the first liquid culture medium is substantially free of mammalian cells. In some disclosed embodiments, the first liquid culture medium occupies about 4% to about 30% of the volume of the container. In some disclosed embodiments, the conical container is a gas-permeable 50-mL to 600-mL conical container. In some embodiments, the mammalian cell is suspended in about 2 mL to about 15 mL of the first liquid culture medium.

In some disclosed embodiments, the mammalian cell is a Chinese hamster ovary (CHO) cell. In some embodiments, the recombinant protein is a secreted immunoglobulin, a secreted enzyme, a secreted growth factor, a secreted protein fragment, or a secreted engineered protein and wherein the recombinant protein is recovered from the first or second culture medium.

In some embodiments, the recombinant protein is recovered from the mammalian cell. In some embodiments, the recombinant protein recovered from the mammalian cell is an immunoglobulin, an enzyme, a growth factor, a protein fragment, or an engineered protein.

In some disclosed embodiments, the container is incubated at about 40 degrees to about 55 degrees from horizontal. In some embodiments, the removing of the first volume of the first liquid culture medium and the adding of the second volume of the second liquid culture medium is performed simultaneously. In some embodiments, the removing of the first volume of the first liquid culture medium and the adding of the second volume of the second liquid culture medium is performed continuously. In some embodiments, the removing of the first volume of the first liquid culture medium and the adding of the second volume of the second liquid culture medium is performed periodically. In some embodiments, the first volume of the first liquid culture medium removed and the second volume of the second liquid culture medium added are increased over time.

In some embodiments, the container is incubated for a period of time greater than 7 days, and on days 1 through 3 of incubation, in each 24-hour period, the first volume of the first liquid culture medium removed and the second volume of the second liquid culture medium added is about 50% of the volume of the first liquid culturing medium; on days 4 through 6 of incubation, in each 24-hour period, the first volume of the first liquid culture medium removed and the second volume of the second liquid culture medium added is about 70% of the volume of the first liquid culture medium; and on day 7 and onwards of incubation, in each 24-hour period, the first volume of the first liquid culture medium removed and the second volume of the second liquid culture medium added is about 100% of the volume of the first liquid culture medium.

In some embodiments, the first liquid culture medium and/or second liquid culture medium is selected from the group of: a chemically-defined liquid culture medium, a serum-free liquid culture medium, a serum-containing liquid culture medium, an animal-derived component free liquid culture medium, and a protein-free medium.

Also disclosed are methods of producing a recombinant protein that include: culturing in a gradient perfusion process a mammalian cell containing a nucleic acid that encodes a recombinant protein, wherein the cell is suspended in a liquid culture medium under conditions that generate in the medium a fluid sheer force and dissolved oxygen ($O_2$) concentration that is essentially the same as that achieved in a volume of liquid culture medium occupying 4% to 40% of the volume of a gas-permeable conical container when the container is positioned at a reactor angle of about 5 degrees to about 85 degrees from horizontal, incubated at a temperature of about 31 °C to about 40 °C, and agitated at a frequency of about 20 revolutions per minute (RPM) to about 1000 RPM; and recovering the recombinant protein from the mammalian cell or the liquid culture medium.

In some disclosed embodiments, the mammalian cell is a Chinese hamster ovary (CHO) cell. In some embodiments, the recombinant protein is a secreted immunoglobulin, a secreted enzyme, a secreted growth factor, a secreted protein fragment, or a secreted engineered protein and wherein the recombinant protein is recovered from the liquid culture medium.

In some embodiments, the recombinant protein is recovered from the mammalian cell. In some embodiments, the recombinant protein that is recovered from the mammalian cell is an immunoglobulin, an enzyme, a growth factor, a protein fragment, or an engineered protein. In some embodiments, the liquid culture medium is selected from the group of: a chemically-defined liquid culture medium, a serum-free liquid culture medium, a serum-containing liquid culture medium, an animal-derived component free liquid culture medium, and a protein-free medium.

Also disclosed are methods for testing a manufacturing process for making a recombinant protein that include: providing a conical container containing a mammalian cell suspended in a first liquid culture medium occupying about 4% to about 80% of the volume of the container; incubating the container for a period of time at about 31 °C to about 40°C at a reactor angle of about 5 degrees to about 85 degrees from horizontal and with an agitation of about 20 revolutions per minute (RPM) to about 1000 RPM; continuously or periodically, during the period of time, removing a first volume of the first liquid culture medium and adding to the first liquid culture medium a second volume of a second liquid culture medium, wherein the first and second volumes are about equal; detecting the recombinant protein in the cell or in the first or second culture medium; and comparing the amount of recombinant protein present in the cell or in the first or second culture medium to a reference level of recombinant protein.

In some embodiments, the first volume of the first liquid culture medium is substantially free of mammalian cells. In some embodiments, the reference level of recombinant protein is a level of recombinant protein produced using a different culturing method. In some embodiments, the different culturing method utilizes a different first or second liquid culture medium, a different mammalian cell, a different conical container, a different temperature, a different level of agitation, or a different reactor angle of the conical container. In some embodiments, the different culturing method utilizes different raw materials, anti-clumping agents, or chemically-defined liquid culture media.

In some disclosed embodiments, the method is used to perform high throughput cell culture experiments to perform a design-of-experiment (DOE) or a quality-by-design (QBD) study. In some disclosed embodiments, the first liquid culture medium occupies about 4% to about 30% of the volume of the container. In some disclosed embodiments, the conical container is a gas-permeable 50-mL to 600-mL conical container. In some embodiments, the mammalian cell is suspended in about 2 mL to about 15 mL of the first liquid culture medium.

In some disclosed embodiments, the mammalian cell is a Chinese hamster ovary (CHO) cell. In some embodiments, the recombinant protein is a secreted immunoglobulin, a secreted enzyme, a secreted growth factor, a secreted protein fragment, or an engineered protein and wherein the recombinant protein is recovered from the first or second culture medium.

In some embodiments, the recombinant protein is recovered from the mammalian cell. In some embodiments the recombinant protein that is recovered from the mammalian cell is an immunoglobulin, an enzyme, a growth factor, a protein fragment, or an engineered protein. In some embodiments, the removing of the first volume of the first liquid culture medium and the adding of the second liquid culture medium is performed simultaneously. In some embodiments, the removing of the first volume of the first liquid culture medium and the adding of the second liquid culture medium is performed continuously. In some embodiments, the removing of the first volume of the first liquid culture medium and the adding of the second liquid culture medium is performed periodically. In some embodiments, the first volume of the first liquid culture medium removed and the second volume of the second liquid culture medium added are increased over time.

In some embodiments, the container is incubated for a period of time greater than 7 days, and on days 1 through 3 of incubation, in each 24-hour period, the first volume of the first liquid culture medium removed and the second volume of the second liquid culture medium added is about 50% of the volume of the first liquid culturing medium; on days 4 through 6 of incubation, in each 24-hour period, the first volume of the first liquid culture medium removed and the second volume of the second liquid culture medium added is about 70% of the volume of the first liquid culture medium; and on day 7 and onwards of incubation, in each 24-hour period, the first volume of the first liquid culture medium removed and the second volume of the second liquid culture medium added is about 100% of the volume of the first liquid culture medium.

In some embodiments, the first liquid culture medium and/or the second liquid culture medium are selected from the group consisting of: a chemically-defined liquid culture medium, a serum-free liquid culture medium, a serum-containing liquid culture medium, an animal-derived component free liquid culture medium, and a protein-free medium.

Also disclosed are methods of testing the efficacy of a first or second liquid culture medium, a raw ingredient or supplement present in a first or second liquid culture medium, or a source of a mammalian cell for use in a method of producing a recombinant protein. These methods include providing a conical container containing a mammalian cell suspended in a first liquid culture medium occupying about 4% to about 80% of the volume of the container; incubating the container for a period of time at about 31 °C to about 40 °C at a reactor angle of about 5 degrees to about 85 degrees from horizontal and with an agitation of about 20 revolutions per minute (RPM) to about 1000 RPM; continuously or periodically, during the period of time, removing a first volume of the first liquid culture medium and adding to the first liquid culture medium a second volume of a second liquid culture medium, where the first and second volumes are about equal; detecting the recombinant protein in the cell or in the first and/or second culture medium; comparing the amount of recombinant protein present in the cell or in the first and/or second culture medium to a reference level of recombinant protein produced by a different method that uses one or more of a different first or second liquid culture medium, a different raw ingredient or supplement present in the first or second liquid culture medium, or a different source of a mammalian cell; and identifying the first or second liquid culture medium, the raw ingredient or supplement present in the first or second liquid culture medium, or the source of the mammalian cell that is associated with an increased amount of recombinant protein as compared to the reference level as being efficacious for use in a method of producing a recombinant protein.

Also disclosed are methods of optimizing a manufacturing process of producing a recombinant protein. These methods include providing a conical container containing a mammalian cell suspended in a first liquid culture medium occupying about 4% to about 80% of the volume of the container; incubating the container for a period of time at about 31 °C to about 40 °C at a reactor angle of about 5 degrees to about 85 degrees from horizontal and with an agitation of about 20 revolutions per minute (RPM) to about 1000 RPM; continuously or periodically, during the period of time, removing a first volume of the first liquid culture medium and adding to the first liquid culture medium a second volume of a second liquid culture medium, where the first and second volumes are about equal; detecting the recombinant protein in the cell or in the first and/or second culture medium; comparing the amount of recombinant protein present in the cell or in the first and/or second culture medium to a reference level of recombinant protein produced by a different method; and identifying and removing or altering in a manufacturing process any culture components or parameters that are associated with a decrease in the amount of recombinant protein produced as compared to the reference level, or identifying and adding to a manufacturing process any culture components or parameters that are associated with an increase in the amount of recombinant protein produced as compared to the reference level.

Also disclosed are methods of testing for the presence of a contaminant in a first or second liquid culture medium, a raw material used to generate a first or second liquid culture medium, or a source of a mammalian cell. These methods include providing a conical container containing a mammalian cell suspended in a first liquid culture medium occupying about 4% to about 80% of the volume of the container; incubating the container for a period of time at about 31°C to about 40 °C at a reactor angle of about 5 degrees to about 85 degrees from horizontal and with an agitation of about 20 revolutions per minute (RPM) to about 1000 RPM; continuously or periodically, during the period of time, removing a first volume of the first liquid culture medium and adding to the first liquid culture medium a second volume of a second liquid culture medium, where the first and second volumes are about equal; detecting the recombinant protein in the cell or in the first and/or second culture medium; comparing the amount of recombinant protein present in the cell or in the first and/or second culture medium to a reference level of recombinant protein produced by a different method that uses one or more of a different first or second liquid culture medium, a different raw material to generate the first or second liquid culture medium, or a different source of the mammalian cell; and identifying the first or second liquid culture medium, the raw material used to generate the first or second liquid culture medium, or the source of a mammalian cell as containing a contaminant when the level of recombinant protein produced is less than the reference level. The contaminant can be a biological contaminant (e.g., a mycobacterium, a fungus, a bacterium, a virus (e.g., a vesivirus), or an undesired mammalian cell).

As used herein, the word "a" before a noun represents one or more of the particular noun. For example, the phrase "a mammalian cell" represents "one or more mammalian cells."

The term "mammalian cell" means any cell from or derived from any mammal (e.g., a human, a hamster, a mouse, a green monkey, a rat, a pig, a cow, or a rabbit). In some disclosed embodiments, a mammalian cell can be an immortalized cell. In some disclosed embodiments, the mammalian cell is a differentiated cell. In some disclosed embodiments, the mammalian cell is an undifferentiated cell.

The term "day 0" means the time point at which a mammalian cell is seeded into the first liquid culture medium.

The term "day 1" means a time period between day 0 and about 24 hours following the seeding of a mammalian cell into the first liquid culture medium.

The term "day 2" means a time period of about 24 hours to about 48 hours following the seeding of a mammalian cell into the first liquid culture medium.

The term "day 3" means a time period of about 48 hours to about 72 hours following the seeding of a mammalian cell into the first liquid culture medium.

The term "day 4" means a time period of about 72 hours to about 96 hours following the seeding of a mammalian cell into the first liquid culture medium. The term for each additional day ("day 5," "day 6," "day 7," and so on) is meant a time period that ranges over an additional about 24-hour period from the end of the immediately preceding day.

The term "substantially free" means a composition (e.g., a liquid culture medium) that is at least or about 90% free (e.g., at least or about 95%, 96%, 97%, 98%, or at least or about 99% free, or about 100% free) of a specific substance (e.g., a mammalian cell).

The term "0.5x volume" means about 50% of the volume. The term "0.6x volume" means about 60% of the volume. Likewise, 0.7x, 0.8x, 0.9x, and 1.0x means about 70%, 80%, 90%, or 100% of the volume, respectively.

The term "culturing" or "cell culturing" is meant the maintenance or growth of a mammalian cell under a controlled set of physical conditions.

The term "conical container" means an elongated vessel (e.g., a sterile vessel) that contains at least one end that is roughly cone-shaped or roughly hemispherical that has at least one gas permeable surface (e.g., an end that has at a gas-permeable membrane which may also act as a sterile barrier) and/or at least one vent cap. A non-limiting example of a conical container is a 50-mL sterile EPPENDORF™ tube with a vent cap which allows for gas permeation. Additional conical containers are known in the art and are commercially available.

The term "liquid culture medium" means a fluid that contains sufficient nutrients to allow a mammalian cell to grow *in vitro*. For example, a liquid culture medium can contain one or more of: amino acids (e.g., 20 amino acids), a purine (e.g., hypoxanthine), a pyrimidine (e.g., thymidine), choline, inositol, thiamine, folic acid, biotin, calcium, niacinamide, pyridoxine, riboflavin, thymidine, cyanocobalamin, pyruvate, lipoic acid, magnesium, glucose, sodium, potassium, iron sulfate, copper sulfate, zinc sulfate, and sodium bicarbonate. In some embodiments, a liquid culture medium can contain serum from a mammal. In some embodiments, a liquid culture medium does not contain serum or another extract from a mammal (a defined liquid culture medium). In some embodiments, a liquid culture medium can contain trace metals, a mammalian growth hormone, and/or a mammalian growth factor. Non-limiting examples of liquid culture medium are described herein. Additional examples of liquid culture medium are known in the art and are commercially available. A liquid culture medium can contain any density of mammalian cells. For example, as used herein, a first volume of the first culture medium removed from the container can be substantially free of mammalian cells.

The term "first liquid culture medium" means a volume of liquid culture medium that is suitable for the culture of a mammalian cell.

The term "second liquid culture medium" means a volume of liquid culture medium that is suitable for the culture of a mammalian cell that is separate from the volume of the first liquid culture medium prior to any mixing of the first and second liquid culture media.

The term "animal-derived component free liquid culture medium" means a liquid culture medium that does not contain any components (e.g., proteins or serum) derived from a mammal.

The term "serum-free liquid culture medium" means a liquid culture medium that does not contain the serum of a mammal.

The term "serum-containing liquid culture medium" means a liquid culture medium that contains a mammalian serum.

The term "chemically-defined liquid culture medium" means a liquid culture medium in which all of the chemical components are known. For example, a chemically-defined liquid culture medium does not contain fetal bovine serum, bovine serum albumin, or human serum albumin, as these preparations typically contain a complex mix of albumins and lipids.

The term "protein-free liquid culture medium" means a liquid culture medium that does not contain any protein (e.g., any detectable protein).

The term "agitation" means the movement of a container containing a liquid culture medium in order to increase the dissolved $O_2$ concentration in the liquid culture medium. Agitation can be performed using any art known method, e.g., an instrument that moves the container in a circular or ellipsoidal motion, such as a rotary shaker. Alternatively or in addition, agitation can be performed by tilting the container or rolling the container. Exemplary devices that can be used to agitate a container are described herein. Additional examples of such devices are also known in the art and are commercially available.

The term "immunoglobulin" means a polypeptide containing an amino acid sequence of at least 15 amino acids (e.g., at least 20, 30, 40, 50, 60, 70, 80, 90, or 100 amino acids) of an immunoglobulin protein (e.g., a variable domain sequence, a framework sequence, or a constant domain sequence). The immunoglobulin may, for example, include at least 15 amino acids of a light chain immunoglobulin, e.g., at least 15 amino acids of a heavy chain immunoglobulin. The immunoglobulin may be an isolated antibody (e.g., an IgG, IgE, IgD, IgA, or IgM). The immunoglobulin may be a subclass of IgG (e.g., IgG1, IgG2, IgG3, or IgG4). The immunoglobulin may be an antibody fragment, e.g., a Fab fragment, a F(ab')$_2$ fragment, or an a scFv fragment. The immunoglobulin may also be a bi-specific antibody or a tri-specific antibody, or a dimer, trimer, or multimer antibody, or a diabody, an Affibody®, or a Nanobody®. The immunoglobulin can also be an engineered protein containing at least one immunoglobulin domain (e.g., a fusion protein). Non-limiting examples of immunoglobulins are described herein and additional examples of immunoglobulins are known in the art.

The term "protein fragment" or "polypeptide fragment" means a portion of a polypeptide sequence that is at least or about 4 amino acids, at least or about 5 amino acids, at least or about 6 amino acids, at least or about 7 amino acids, at least or about 8 amino acids, at least or about 9 amino acids, at least or about 10 amino acids, at least or about 11 amino acids, at least or about 12 amino acids, at least or about 13 amino acids, at least or about 14 amino acids, at least or about 15 amino acids, at least or about 16 amino acids, at least or about 17 amino acids, at least or about 18 amino acids, at least or about 19 amino acids, or at least or about 20 amino acids in length, or more than 20 amino acids in length. A recombinant protein fragment can be produced using any of the methods described herein.

The term "engineered protein" means a polypeptide that is not naturally encoded by an endogenous nucleic acid present within an organism (e.g., a mammal). Examples of engineered proteins include enzymes (e.g., with one or more amino acid substitutions, deletions, insertions, or additions that result in an increase in stability and/or catalytic activity of the engineered enzyme), fusion proteins, antibodies (e.g., divalent antibodies, trivalent antibodies, or a diabody), and antigen-binding proteins that contain at least one recombinant scaffolding sequence.

The term "fluid sheer force" means a stress caused by a liquid flowing roughly parallel to a surface (e.g., a surface of a cell or a surface of a container). Fluid sheer force is generally defined as the force applied divided by the cross-sectional area of material with area parallel to the applied force vector. Exemplary methods of calculating fluid sheer force are described herein and are known in the art.

The term "dissolved $O_2$ concentration" or "dissolved oxygen concentration" means the amount of oxygen gas dissolved in a liquid culture medium (e.g., any of the liquid culture media described herein or known in the art). Non-limiting methods for measuring the dissolved $O_2$ concentration in a liquid culture medium are described herein and others are known in the art.

The term "recovering" means partially purifying or isolating (e.g., at least or about 5%, e.g., at least or about 10%, 15%, 20%, 25%, 30%, 40%, 45%, 50%, 55%, 60%, 65%, 70%, 75%, 80%, 85%, 90%, or at least or about 95% pure by weight) a recombinant protein from one or more other components present in the cell culture medium (e.g., mammalian cells or culture medium proteins) or one or more other components (e.g., DNA, RNA, or other proteins) present in a mammalian cell lysate. Non-limiting methods for recovering a protein from a liquid culture medium or from a mammalian cell lysate are described herein and others are known in the art.

The term "secreted protein" or "secreted recombinant protein" means a protein or a recombinant protein that originally contained at least one secretion signal sequence when it is translated within a mammalian cell, and through, at least in part, enzymatic cleavage of the secretion signal sequence in the mammalian cell, is released into the extracellular space (e.g., a liquid culture medium).

The phrase "gradient perfusion" refers to the incremental change (e.g., increase or decrease) in the volume of culture medium removed and added over incremental periods (e.g., an about 24-hour period, a period of between about 1 minute and about 24-hours, or a period of greater than 24 hours) during the culturing period (e.g., the culture medium refeed rate on a daily basis). For example, one embodiment of a gradient perfusion process may entail refeed protocols as follows: days 1-3 refeed of about 0.5x reactor volume of culture medium (RV)/day, days 4-6 refeed of about 0.7x RV/day, and day 7 and onwards refeed of about 1.0x RV/day. This particular example can vary with respect to the number of days having a certain refeed rate and/or with respect to the refeed rate over any particular 24-hour period. The fraction of media removed and replaced each day can vary depending on the particular cells being cultured, the initial seeding density, and the cell density at a particular time. "RV" or "reactor volume" means the volume of the culture medium present at the beginning of the culturing process (e.g., the total volume of the culture medium present after seeding).

The term "feed-batch culture" means the incremental or continuous addition of a second liquid culture medium to an initial cell culture without substantial or significant removal of the first liquid culture medium from the cell culture. In some disclosed embodiments of feed-batch culture, the second liquid culture medium is the same as the first liquid culture medium. In some disclosed embodiments of feed-batch culture, the second liquid culture medium is a concentrated form of the first liquid culture medium. In some disclosed embodiments of feed-batch culture, the second liquid culture medium is added as a dry powder.

The term "reactor angle" refers to the angle of deviation from the horizontal position that the container (e.g., a conical container) containing a mammalian cell is placed during the culturing methods described herein. For example, when the container containing a mammalian cell is a 50-mL conical tube and is standing vertical relative to the lab bench or ground, the reactor angle is 90°, and when the container containing a mammalian cell is a 50-mL conical tube and is placed horizontal relative to the lab bench or ground, the reactor angle is 0°. In another example, when a container containing a mammalian cell is a 50-mL conical tube and is placed equidistant between the vertical and horizontal positions (relative to the lab bench or ground), the reactor angle is 45°.

"Specific productivity rate" or "SPR" as used herein refers to the mass or enzymatic activity of a recombinant protein produced per mammalian cell per day. The SPR for a recombinant antibody is usually measured as mass/cell/day. The SPR for a recombinant enzyme is usually measured as units/cell/day or (units/mass)/cell/day.

"Volume productivity rate" or "VPR" as used herein refers to the mass or enzymatic activity of recombinant protein produced per volume of culture (e.g., per L of bioreactor, vessel, or tube volume) per day. The VPR for a recombinant antibody is usually measured as mass/L/day. The VPR for a recombinant enzyme is usually measured as units/L/day or mass/L/day.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. In case of conflict, the present specification, including definitions, will control.

Other features and advantages of the invention will be apparent from the following detailed description and figures, and from the claims.

## DESCRIPTION OF DRAWINGS

Figure 1 is a schematic showing non-limiting examples of different containers placed at different reactor angles with rotary agitation.

Figure 2A is a graph showing the viable cell density profiles of C02.31 cells cultured through 18 days in shaken tubes placed at about 45° from horizontal and at 90° from horizontal, which correspond to reactor angles of 45° and 90°, respectively.

Figure 2B is a graph showing the volumetric productivity rate profiles of C02.31 cells cultured through 18 days in shaken tubes placed at about 45° from horizontal and 90° from horizontal, which correspond to reactor angles of 45° and 90°, respectively.

Figure 2C is a graph showing the glutamine consumption rate of C02.31 cells cultured through 18 days in shaken tubes placed at about 45° from horizontal and 90° from horizontal, which correspond to reactor angles of 45° and 90°, respectively.

Figure 2D is a graph showing the lactate production of C02.31 cells cultured through 18 days in shaken tubes placed at about 45° from horizontal and 90° from horizontal, which correspond to reactor angles of 45° and 90°, respectively.

Figure 2E is a graph showing the specific productivity rate (SPR) profiles of C02.31 cells cultured through 18 days in shaken tubes placed at about 45° from horizontal and 90° from horizontal, which correspond to reactor angles of 45° and 90°, respectively.

Figure 2F is a graph showing the average percent increase in volumetric productivity rate and specific productivity rate when C02.31 cells are cultured at an angle of 45° from horizontal (reactor angle of 45°) or an angle of 90° from horizontal (reactor angle of 90°).

Figure 3A is a graph showing the volume productivity rate profiles of recombinant galactosidase clonal cell line C02.31 shaken suspension cultures maintained in CD CHO medium at different temperatures and placed at about 45° from horizontal (a reactor angle of 45°).

Figure 3B is a graph showing the specific productivity rate profiles of recombinant galactosidase clonal cell line C02.31 shaken suspension cultures maintained in CD CHO medium at different temperatures and placed at about 45° from horizontal (a reactor angle of 45°).

Figure 3C is a graph showing the percent cell viability of recombinant galactosidase clonal cell line C02.31 shaken suspension cultures maintained in CD CHO medium at different temperatures and placed at about 45° from horizontal (a reactor angle of 45°).

Figure 3D is a graph showing the viable cell density of recombinant galactosidase clonal cell line C02.31 shaken suspension cultures maintained in CD CHO medium at different temperatures and placed at about 45° from horizontal (a reactor angle of 45°).

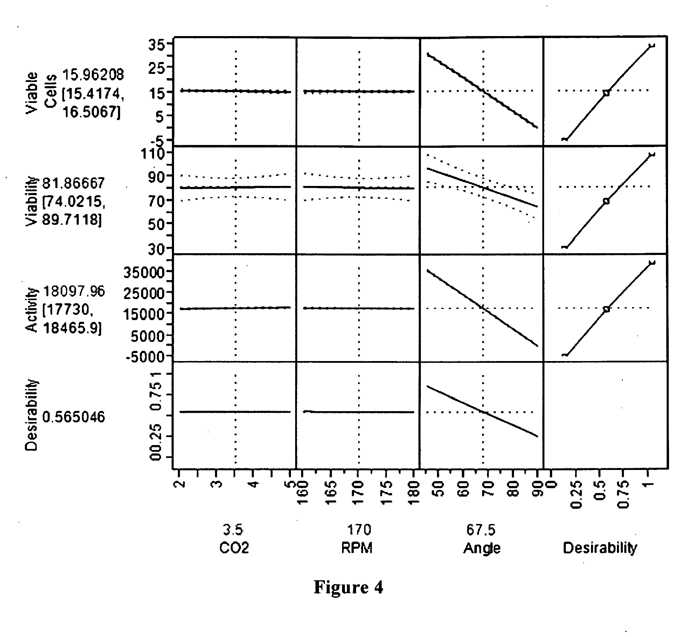

Figure 4 is a set of graphs showing the statistical effect of each of $CO_2$ exposure, frequency of agitation (RPM), and reactor angle on the number of viable cells, percentage viable cells, and recombinant protein activity or productivity. These analyses were performed using JMP software (see JMP website). The effect of each individual parameter on cell growth and recombinant protein productivity is shown in a range of values of between 0 and 1. A value of 0 represents a condition that has the least desirable effect on cell growth and recombinant protein productivity. A value of 1 represents a condition that has the most desirable effect on cell growth and recombinant protein productivity.

Figure 5A is a graph showing the viable cell density profiles of recombinant galactosidase clonal cell lines shaken in CD CHO media when placed at about 45° from horizontal (a reactor angle of 45°).

Figure 5B is a graph showing the viable cell density profiles of recombinant galactosidase clonal cell lines shaken in OptiCHO media when placed at about 45° from horizontal (a reactor angle of 45°).

Figure 5C is a graph showing the viable cell density profiles of recombinant galactosidase clonal cell lines shaken in FortiCHO media in tubes placed at about 45° from horizontal (a reactor angle of 45°).

Figure 6A is a graph showing the integral of viable cell density (IVCD) profiles of recombinant galactosidase clonal cell lines shaken in CD CHO media in tubes placed at about 45° from horizontal (a reactor angle of 45°).

Figure 6B is a graph showing the IVCD profiles of recombinant galactosidase clonal cell lines shaken in OptiCHO media in tubes placed at about 45° from horizontal (a reactor angle of 45°).

Figure 6C is a graph showing the IVCD profiles of recombinant galactosidase clonal cell lines shaken in FortiCHO media in tubes placed at about 45° from horizontal (a reactor angle of 45°).

Figure 7A is a graph of the volumetric productivity rate of four recombinant galactosidase clonal cell lines shaken in CD CHO media in tubes placed at about 45° from horizontal (a reactor angle of 45°).

Figure 7B is a graph of the volumetric productivity rate of four recombinant galactosidase clonal cell lines shaken in OptiCHO media in tubes placed at about 45° from horizontal (a reactor angle of 45°).

Figure 7C is a graph of the volumetric productivity rate of four recombinant galactosidase clonal cell lines shaken in FortiCHO media in tubes placed at about 45° from horizontal (a reactor angle of 45°).

Figure 8A is a graph of the specific productivity rate of four recombinant galactosidase clonal cell lines shaken in CD CHO media in tubes placed at about 45° from horizontal (a reactor angle of 45°).

Figure 8B is a graph of the specific productivity rate of four recombinant galactosidase clonal cell lines shaken in Opti CHO media in tubes placed at about 45° from horizontal (a reactor angle of 45°).

Figure 8C is a graph of the specific productivity rate of four recombinant galactosidase clonal cell lines shaken in Forti CHO media in tubes placed at about 45° from horizontal (a reactor angle of 45°).

Figure 9A is a graph of the percentage of viable recombinant galactosidase clonal C02.31 cells in CD CHO medium in shake tubes (ST) placed at about 45° from horizontal (a reactor angle of 45°) or in a 12-L bioreactor. Error bars represent the standard deviation of n.

Figure 9B is a graph of the viable cell density profiles of recombinant galactosidase clonal C02.31 cells cultured in CD CHO medium in shake tubes (ST) placed at about 45° from horizontal (a reactor angle of 45°) or in a 12-L bioreactor. Error bars represent the standard deviation of n.

Figure 9C is a graph of the product titer (units/L) of recombinant galactosidase present in the liquid culture medium of recombinant galactosidase clonal C02.31 cells cultured in CD CHO medium in shake tubes (ST) placed at about 45° from horizontal (a reactor angle of 45°) or in a 12-L bioreactor. Error bars represent the standard deviation of n.

Figure 9D is a graph showing the specific productivity rate (pg/cell/day) of recombinant galactosidase clonal C02.31 cells cultured in CD CHO medium in shake tubes (ST) placed at about 45° from horizontal (a reactor angle of 45°) or in a 12-L bioreactor. Error bars represent the standard deviation of n.

Figure 10 is a graph showing the viable cell density of recombinant galactosidase in satellite conical container process runs cultured at an angle of about 45° from horizontal (a reactor angle of about 45°) ("spin tube cultures") (n = 6) or in 12-L bioreactor cell culture process runs ("12L Bioreactor") (n = 2). The average data are shown.

Figure 11 is a graph of the titer (units/L) of recombinant human alpha-galactosidase over time in satellite conical container process runs cultured at an angle of about 45° from horizontal (a reactor angle of about 45°) ("spin tube cultures") (n = 6) or in 12-L bioreactor cell culture process runs ("12L Bioreactor") (n = 2). The average data are shown.

Figure 12A is a graph of the viable cell density over time of recombinant galactosidase clonal C02.31 cells cultured in CD CHO medium in 50-mL shake tubes and 600-mL maxi tubes. The error bars represent the standard deviation (n = 2).

Figure 12B is a graph of the percent cell viability over time in recombinant galactosidase clonal C02.31 cells cultured in CD CHO medium in 50-mL shake tubes and 600-mL maxi tubes. The error bars represent the standard deviation (n = 2).

Figure 13 is a graph of the viable cell density over time in recombinant galactosidase clonal C02.31 cells cultured in CD CHO medium in 50-mL shake tubes (n = 2) and 600-mL maxi tubes (n = 2), as compared to the historical average of the viable cell density of recombinant galactosidase clonal C02.31 cells cultured in CD CHO medium over time in 50-mL shake tubes ("historical shake tube," n = 9).

Figure 14A is a graph of the volumetric productivity rate (VPR) over time in recombinant galactosidase clonal C02.31 cells cultured in CD CHO medium in 50-mL shake tubes (n = 2) and 600-mL maxi tubes (n = 2), as compared to the historical average of the VPR of recombinant galactosidase clonal C02.31 cells cultured in CD CHO medium in 50-mL shake tubes ("historical shake tube," n = 9).

Figure 14B is a graph of the specific productivity rate (SPR) in recombinant galactosidase clonal C02.31 cells cultured in CD CHO medium in 50-mL shake tubes (n = 2) and 600-mL maxi tubes (n = 2).

Figure 15A is a graph of the viable cell density over time in recombinant galactosidase clonal C02.31 cells cultured in CD CHO medium using varied parameters. The error bars represent the standard deviation of n = 3.

Figure 15B is a graph of the viable cell density over time in recombinant galactosidase clonal C02.31 cells cultured in CD CHO medium using varied parameters. The error bars represent the standard deviation of n = 3.

Figure 15C is a graph of the percent cell viability over time in recombinant galactosidase clonal C02.31 cells cultured in CD CHO medium using varied parameters. The error bars represent the standard deviation of n = 3.

Figure 16 is a graph of the volumetric productivity rate over time in recombinant galactosidase clonal C02.31 cells cultured in CD CHO medium using varied parameters. The error bars represent the standard deviation of n = 3.

Figure 17A is a graph of the integrated viable cell density over time in recombinant galactosidase clonal C02.31 cells cultured in CD CHO medium using varied parameters. The error bars represent the standard deviation of n = 3.

Figure 17B is a graph of the integrated volumetric productivity rate over time in recombinant galactosidase clonal C02.31 cells cultured in CD CHO medium using varied parameters. The error bars represent the standard deviation of n = 3.

Figure 17C is a graph of the end-point comparison of titer profile versus integrated volumetric productivity rate profile for recombinant galactosidase clonal C02.31 cells cultured in CD CHO medium using varied parameters. The error bars represent the standard deviation of n = 3.

Figure 18A is a graph of the viable cell density over time in recombinant galactosidase clonal C02.31 cells cultured in CD CHO medium using varied parameters. The error bars represent the standard deviation of n = 3.

Figure 18B is a graph of the volumetric productivity rate over time in recombinant galactosidase clonal C02.31 cells cultured in CD CHO medium using varied parameters. The error bars represent the standard deviation of n = 3.

Figure 19A is a graph of the integrated viable cell density over time in recombinant galactosidase clonal C02.31 cells cultured in CD CHO medium using varied parameters. The error bars represent the standard deviation of n = 3.

Figure 19B is a graph of the integrated volumetric productivity rate over time in recombinant galactosidase clonal C02.31 cells cultured in CD CHO medium using varied parameters. The error bars represent the standard deviation of n = 3.

Figure 20A is a graph of the viable cell density over time in recombinant galactosidase clonal C02.31 cells cultured in CD CHO medium using varied parameters. The error bars represent the standard deviation of n = 2.

Figure 20B is a graph of the viable cell density over time in recombinant galactosidase clonal C02.31 cells cultured in CD CHO medium using varied parameters. The error bars represent the standard deviation of n = 2.

Figure 20C is a graph of the percent cell viability over time in recombinant galactosidase clonal C02.31 cells cultured in CD CHO medium using varied parameters. The error bars represent the standard deviation of n = 2.

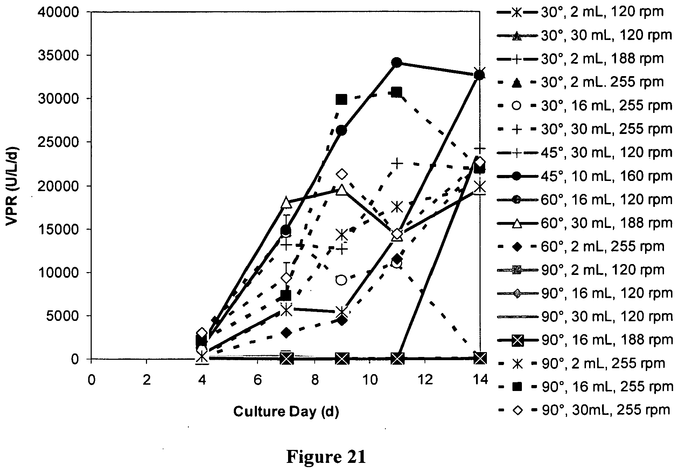

Figure 21 is a graph of the volumetric productivity rate over time in recombinant galactosidase clonal C02.31 cells cultured in CD CHO medium using varied parameters. The error bars represent the standard deviation of n = 2.

Figure 22A is a graph of the integrated viable cell density over time in recombinant galactosidase clonal C02.31 cells cultured in CD CHO medium using varied parameters. The error bars represent the standard deviation of n = 2.

Figure 22B is a graph of the integrated volumetric productivity rate over time in recombinant galactosidase clonal C02.31 cells cultured in CD CHO medium using varied parameters. The error bars represent the standard deviation of n = 2.

Figure 22C is a graph of the end-point comparison of titer profile versus integrated volumetric productivity rate in recombinant galactosidase clonal C02.31 cells cultured in CD CHO medium using varied parameters. The error bars represent the standard deviation of n = 2.

Figure 23A is a fit of real-time viable cell density data to model predictions of viable cell density.

Figure 23B is a fit of real-time volumetric productivity rate data to model predictions of volumetric productivity data.

Figure 24A is a set of graphs showing the interaction profiles of the effect of each parameter on viable cell density.

Figure 24B is a set of graphs showing the interaction profiles of the effect of each parameter on volumetric productivity rate.

Figure 25 is a set of graphs showing the best operating conditions based on the empirical data input into the chosen statistical model taking into account standard deviations (shown with dotted lines following the response trends).

Figure 26A is a graph showing the average peak viable cell density of grouped recombinant galactosidase clonal C02.31 cells maintained in CD CHO medium for 12-14 days with varied parameters. The error bars represent the standard deviation of n = number of conditions in each group.

Figure 26B is a graph showing the average peak volumetric productivity rate of grouped recombinant galactosidase clonal C02.31 cells maintained in CD CHO medium for 12-14 days with varied parameters.

## DETAILED DESCRIPTION

Disclosed herein are improved methods of culturing a mammalian cell. The culturing methods can achieve a viable mammalian cell concentration (e.g., in the liquid culture medium, e.g., the first liquid culture medium, or a combination of the first and second liquid culture medium) of greater than $10 \times 10^6$ cells per mL, greater than $15 \times 10^6$ cells/mL, greater than $20 \times 10^6$ cells/mL, greater than $25 \times 10^6$ cells/mL, greater than $30 \times 10^6$ cells/mL, greater than $35 \times 10^6$ cells/mL, greater than $40 \times 10^6$ cells/mL, greater than $45 \times 10^6$ cells/mL, greater than $50 \times 10^6$ cells/mL, greater than $55 \times 10^6$ cells/mL, or greater than $60 \times 10^6$ cells/mL. For example, the culturing method can result in a viable mammalian cell concentration of between $10 \times 10^6$ cells/mL and $30 \times 10^6$ cells/mL, between $10 \times 10^6$ cells/mL and $25 \times 10^6$ cells/mL, between $12 \times 10^6$ cells/mL and $20 \times 10^6$ cells/mL, between $20 \times 10^6$ cells/mL and $65 \times 10^6$ cells/mL, between $25 \times 10 \times 10^6$ cells/mL and $60 \times 10^6$ cells/mL, between $25 \times 10^6$ cells/mL and $55 \times 10^6$ cells/mL, between $25 \times 10^6$ cells/mL and $50 \times 10^6$ cells/mL, or between $30 \times 10^6$ cells/mL and $50 \times 10^6$ cells/mL. A variety of different methods can be used to determining the cell density or viable cell density. For example, the sample of the cell culture can be diluted in physiological buffer, the diluted cell suspension placed in a hemocytometer, and the cells counted using light microscopy. In another method, the viable cell density can be determined using a similar method, but including in the physiological buffer a dye that is selectively taken up by non-viable cells (e.g., trypan blue, such as Vi-CELL method from Beckman Coulter (see Beckman Coulter website)). In yet another example, the cell density or viable cell density can be determined using fluorescence-assisted flow cytometry (e.g., GUAVA from Merck Millipore (see Millipore website), and other cell counting methods.

In some embodiments, the culturing method results in a significantly improved specific productivity rate. For example, the specific productivity rate achieved by the methods provided herein is at least 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, 110-fold, 120-fold, 130-fold, 140-fold, 150-fold, 160-fold, 170-fold, 180-fold, 190-fold, or 200-fold greater than the specific productivity rate achieved under substantially the same culturing conditions, but with the reactor angle set at 0°. The productivity achieved by the present methods can be at least 10,000 units/L, at least 15,000 units/L, at least about 20,000 units/L, at least about 25,000 units/L, at least about 30,000 units/L, at least about 35,000 units/L, or at least about 40,000 units/L (in the first and/or second liquid culture medium). In some embodiments, the productivity achieved by the present methods can be at least 1 g/L, at least 1.5 g/L, at least 2.0 g/L, at least 2.5 g/L, at least 3.0 g/L, at least 4.0 g/L, at least 4.5 g/L, or at least 5.0 g/L.

The biological activity of a recombinant protein can be assessed using a variety of methods known in the art, and will depend on the activity of the specific recombinant protein. For example, the biological activity of a recombinant protein that is an immunoglobulin (e.g., an antibody or an antibody fragment) can be determined by measuring the affinity of the antibody to bind to its specific epitope (e.g., using Biocore or competitive enzyme-linked immunosorbent assays). The recombinant protein may be an enzyme (e.g., a recombinant galactosidase, e.g., a recombinant alpha-galactosidase) and the biological activity may be determined by measuring the enzyme's activity (e.g., determining the catalytic rate constant of the enzyme by measuring a decrease in the concentration of a detectable substrate or an increase in the concentration of a detectable product (e.g., using spectrophotometry or light emission). For example, the biological activity of a recombinant galactosidase can be detected by measuring a decrease in the level of globotriasylceramide (GL-3) or galabiosylceramide, or an increase in the level of ceramide dihexoside or galactose.

**Methods of Culturing a Mammalian Cell**

In a method that is exemplary of those described herein, a conical container is first provided. A first liquid culture medium is added to the conical container such that the medium occupies about 4% to about 80% (e.g., about 4% to about 70%, about 4% to about 60%, or about 4% to about 30%) of the volume of the container. At least one mammalian cell is added to the first liquid culture medium, i.e., either before the medium is added to the conical container or afterward. The container is incubated for a period of time at about 31° C to about 40 °C while disposed at a reactor angle of about 5 degrees to about 85 degrees (e.g., about 10 degrees to about 85 degrees, about 15 degrees to about 85 degrees, about 5 degrees to about 65 degrees, or about 35 to about 50 degrees) from horizontal and agitated, e.g., on a rotary shaking device, at about 20 RPM to about 1000 RPM (e.g., about 20 RPM to about 400 RPM, about 120 RPM to about 240 RPM, about 140 RPM to about 220 RPM, about 160 RPM to about 180 RPM, about 400 RPM to about 600 RPM, about 600 RPM to about 800 RPM, or about 800 RPM to about 1000 RPM). The cells can be incubated, for example, in an incubator, such as a shake incubator with throw (orbit) diameter from about 3 mm to about 50 mm. During incubation, continuously or periodically over the period of time, a first volume of the first liquid culture medium (e.g., containing any mammalian cell concentration, e.g., a first volume of first liquid culture medium which is or is made substantially free of mammalian cells) is removed, and a second volume of a second liquid culture medium is added to the first liquid culture medium. Typically, the first and the second volumes are roughly equal, but can vary by a small amount, e.g., by up to about 10% when the first and second volumes are compared. In some disclosed embodiments, the second volume of the second liquid culture medium added is less (e.g., at most about 1%, 2%, 3%, 4%, 5%, 6%, 7%, 8%, 9%, or 10% less) than the first volume of the first liquid culture medium removed. As is known in the art, the term incubating can include short periods of time (e.g., at most 10 minutes, at most 20 minutes, at most 30 minutes, at most 40 minutes, at most 50 minutes, or at most 1 hour) in which a container containing the mammalian cell and liquid culture medium is removed from an incubator in order to remove the first volume of the first liquid culture medium and add the second volume of the second liquid culture medium.

Various non-limiting examples of each aspect of these culturing methods are described below. The exemplary aspects of the methods provided herein can be used in any combination without limitation.

*Mammalian Cells*

The methods disclosed herein can be used to culture a variety of different mammalian cells. The mammalian cell can be a cell that grows in suspension or an adherent cell. Non-limiting examples of mammalian cells that can be cultured using any of the methods described herein include: Chinese hamster ovary (CHO) cells (e.g., CHO DG44 cells, CHO-K1s cells, C02.31 clonal cells, A14.13 clonal cells, C02.57 clonal cells, and F05.43 clonal cells), Sp2.0, myeloma cells (e.g., NS/0), B-cells, hybridoma cells, T-cells, human embryonic kidney (HEK) cells (e.g, HEK 293E and HEK 293F), African green monkey kidney epithelial cells (Vero) cells, and Madin-Darby Canine (Cocker Spaniel) kidney epithelial cells (MDCK) cells. Additional mammalian cells that can be cultured using the methods described herein are known in the art.

The mammalian cell can contain a recombinant nucleic acid (e.g., a nucleic acid stably integrated in the mammalian cell's genome) that encodes a recombinant protein. Non-limiting examples of recombinant nucleic acids that encode exemplary recombinant proteins are described below, as are recombinant proteins that are producible using the methods described herein. In some instances, the mammalian cell disposed in the container for culturing is derived from a larger culture. For example, the mammalian cell in the container can be derived from a large-scale bioreactor culture, i.e., a satellite culture can be prepared using the methods.

*Culture Media*

Liquid culture media are known in the art. The first and/or second tissue culture medium can be supplemented with a mammalian serum (e.g., fetal calf serum and bovine serum), and/or a growth hormone or growth factor (e.g., insulin, transferrin, and epidermal growth factor). Alternatively or in addition, the first and/or second liquid culture medium can be a chemically-defined liquid culture medium, an animal-derived component free liquid culture medium, a serum-free liquid culture medium, or a serum-containing liquid culture medium. Non-limiting examples of chemically-defined liquid culture media, animal-derived component free liquid culture media, serum-free liquid culture media, and serum-containing liquid culture media are commercially available.

A liquid culture medium typically contains an energy source (e.g., a carbohydrate, such as glucose), essential amino acids (e.g., the basic set of twenty amino acids plus cysteine), vitamins and/or other organic compounds required at low concentrations, free fatty acids, and/or trace elements. The first and/or second liquid culture medium can, if desired, be supplemented with, e.g., a mammalian hormone or growth factor (e.g., insulin, transferrin, or epidermal growth factor), salts and buffers (e.g., calcium, magnesium, and phosphate salts), nucleosides and bases (e.g., adenosine, thymidine, and hypoxanthine), protein and tissue hydrolysates, and/or any combination of these additives.

Non-limiting examples of liquid culture media that are particularly useful in the presently described methods include, e.g., CD CHO, Opti CHO, and Forti CHO (all available from Life Technologies; Grand Island, NY), Hycell CHO medium (Thermo Fisher Scientific, Inc.; Waltham, MA), Ex-cell CD CHO Fusion medium (Sigma-Aldrich Co.; St. Louis, MO), and PowerCHO medium (Lonza Group, Ltd.; Basel, Switzerland). Medium components that also may be useful in the present methods include, but are not limited to, chemically-defined (CD) hydrolysates, e.g., CD peptone, CD polypeptides (two or more amino acids), and CD growth factors. Additional examples of liquid tissue culture medium and medium components are known in the art.

Skilled practitioners will appreciate that the first liquid culture medium and the second liquid culture medium described herein can be the same type of media or different media.

*Containers*

The container disclosed herein can be a sterile conical container (e.g., a 600-mL, a 50-mL, 40-mL, 30-mL, 25-mL, 20-mL, 15-mL, 10-mL, or 5-mL container). The container can have a volume of at least 2 mL (e.g., a volume of between or about 2 mL to about 600 mL, between about 2 mL to about 2 L, or between about 2 mL to about 3 L (e.g., between or about 5 mL and 500 mL, between about 2 mL and about 10 mL, between about 2 mL and 5 mL, between about 5 mL and about 15 mL, between about 15 mL and about 50 mL, between about 40 mL and about 100 mL, between about 100 mL and about 600 mL, between about 200 mL and about 600 mL, between about 250 mL and about 500 mL, between about 50 mL and about 3 L, between about 50 mL and about 2.5 L, between about 50 mL and about 2 L, between about 50 mL and about 1.5 L, between about 50 mL and about 1 L, or between about 50 mL and about 800 mL, inclusive)). The container can include at least one gas permeable surface (e.g., at least one surface having a gas permeable membrane which may also act as a sterile barrier) and/or at least one vented cap. The container can be a sterile tissue culture flask. Alternatively, the container can be a sterile tube with approximately flat or flush ends having at least one gas-permeable surface (e.g., at least one gas permeable membrane) and/or at least one vented cap. Alternatively, the container can be a sterile tissue culture tube having an approximately hemispherical end and at least one gas-permeable surface (e.g., at least one gas permeable membrane) and/or at least one vented gap. A container may have on its outer surface a structure that allows the tube to be stably placed in an tissue culture incubator at a reactor angle of about 5 degrees to about 85 degrees (e.g., about 10 degrees to about 85 degrees, about 15 degrees to about 85 degrees, about 5 degrees to about 65 degrees, or about 35 degrees to about 50 degrees) from horizontal.

The interior surface of the container may have at least one coating (e.g., at least one coating of gelatin, collagen, poly-L-ornithine, polystyrene, and laminin). The container can be, e.g., a TubeSpin® bioreactor available from TubeSpin® Bioreactors 50 from Techno Plastic Products AG, Trasadingen, Switzerland, or a 50 mL CultiFlask from Sartorius AG, Goettingen, Germany. Additional examples of containers (e.g., different shapes and dimensions of containers) and interior surface coatings of containers are known in the art and can be used in the present methods.

*Reactor Angle*

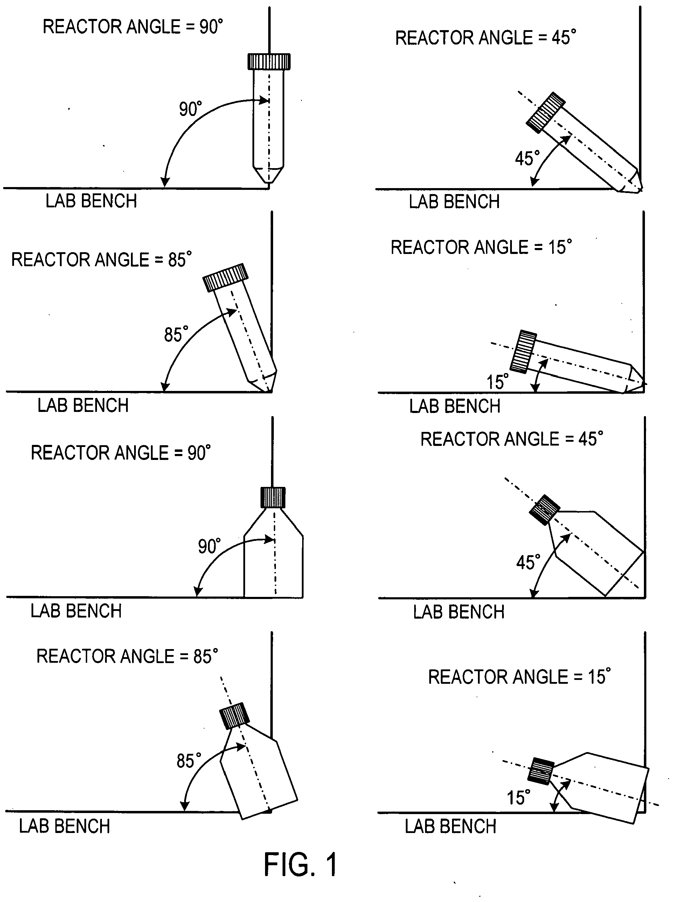

As disclosed herein, the container can be incubated at a reactor angle of about 5 degrees to about 85 degrees (e.g., about 10 degrees to about 85 degrees, about 15 degrees to about 85 degrees, about 5 degrees to about 65 degrees, or about 35 to about 50 degrees) from horizontal (see, the non-limiting examples shown in Figure 1). For example, the container can be placed at a reactor angle of about 60 degrees to about 85 degrees from horizontal, about 70 degrees to about 85 degrees from horizontal, about 15 degrees to about 60 degrees, about 30 degrees to about 55 degrees from horizontal, about 40 degrees to about 55 degrees horizontal, or about 40 degrees to about 50 degrees from horizontal. The container may be placed at a reactor angle of about 45 degrees from horizontal to about 50 degrees from horizontal, or from about 40 degrees from horizontal to about 45 degrees from horizontal. The container may be placed in a device that specifically and securely positions the container at a reactor angle of about 5 degrees to about 85 degrees from horizontal (e.g., specifically positions the container at a reactor angle of about 10 degrees to about 85 degrees, about 15 degrees to about 85 degrees, about 25 degrees to about 85 degrees, about 25 degrees to about 55 degrees, or about 40 degrees to about 55 degrees from horizontal). The positioning of the container can be performed using any means known in the art, e.g., through the use of a brace or a locking element.

*Agitation*

The methods described herein require the agitation of the culture containing the mammalian cell and the first and/or second liquid culture medium. The agitation can occur at a frequency of about 20 revolutions per minute (RPM) to about 1000 RPM (e.g., at about 20 RPM to about 400 RPM, at about 120 RPM to about 220 RPM, at about 140 RPM to about 220 RPM, at about at about 160 RPM to about 180 RPM, at about 140 RPM to about 150 RPM, at about 150 RPM to about 160 RPM, at about 160 RPM to about 170 RPM, at about 170 RPM to about 180 RPM, at about 180 RPM to about 190 RPM, at about 190 RPM to about 200 RPM, at about 200 RPM to about 210 RPM, at about 210 RPM to about 220 RPM, at about 140 RPM to about 180 RPM, at about 140 RPM to about 160 RPM, at about 160 RPM to about 200 RPM, at about 180 RPM to about 220 RPM, at about 220 RPM to about 300 RPM, at about 300 RPM to about 350 RPM, at about 350 RPM to about 400 RPM, at about 400 RPM to about 600 RPM, at about 600 RPM to about 800 RPM, or at about 800 RPM to about 1000 RPM) (e.g., in an incubator, such as a shake incubator with throw (orbit) diameter from about 3 mm to about 50 mm).

As can be appreciated in the art, the level of agitation (e.g., RPM speed) can be varied depending upon the size and shape of the container (e.g., the diameter of the container) and the throw (orbit) diameter of the incubator that is used to perform the incubating. For example, a smaller throw (orbit) diameter can require a higher level of agitation (e.g., a higher RPM speed), while a larger throw (orbit) diameter can require a lower level of agitation (e.g., a lower RPM speed) to achieve a similar level of fluid sheer force and dissolved $O_2$ concentration. In another example, a container having a larger diameter can require a lower RPM speed, while a container having a smaller diameter can require a higher RPM speed to achieve a similar level of fluid sheer force and dissolved $O_2$ concentration. In some disclosed embodiments, the incubating is performed using a shake tube incubator with a throw (orbit) diameter of between about 25 mm to about 50 mm and an agitation of between about 20 RPM to about 400 RPM (e.g., about 120 RPM to about 240 RPM, about 140 RPM to about 220 RPM, about 160 RPM to about 180 RPM). In some disclosed embodiments, the incubating is performed using a shake tube incubator with a throw (orbit) diameter of about 1 mm to about 25 mm and an agitation of about 20 RPM to about 1000 RPM (e.g., about 100 RPM to about 1000 RPM, about 200 RPM to about 1000 RPM, about 100 RPM to about 200 RPM, about 200 RPM to about 300 RPM, about 300 RPM to about 400 RPM, about 400 RPM to about 500 RPM, about 500 RPM to about 600 RPM, about 600 RPM to about 700 RPM, about 700 RPM to about 800 RPM, about 800 RPM to about 900 RPM, about 900 RPM to about 1000 RPM).

As can be appreciated in the art, the type of agitation that is employed will depend on the structure of the container. For example, the container can be a conical container (e.g., a conical container having a volume greater than 2 mL, e.g., an about 2-mL to about 600-mL conical container, or an about 2-mL to about 50-mL conical container) and the agitation can be performed using rotary circular shaking at a frequency of about 120 RPM to about 400 RPM (e.g., about 120 RPM to about 220 RPM, about 140 RPM to about 220 RPM, or about 160 RPM to about 180 RPM). Alternatively or in addition, the container can be agitated using a rotary ellipsoidal shaking, or horizontal and/or vertical tilting of the container. The agitation can be performed continuously or periodically.

The agitation of the container can result in essentially the same fluid sheer force and dissolved oxygen ($O_2$) concentration as that achieved in a gas-permeable conical container containing a liquid culture medium that occupies 4% to 80% (e.g., 4% to 40% or 4% to 30%) volume of the container when the container is positioned at a reactor angle of 5 degrees to about 85 degrees (e.g., at a reactor angle of about 10 degrees to about 85 degrees, about 15 degrees to about 85 degrees, about 25 degrees to about 85 degrees, or about 40 degrees to about 55 degrees) from horizontal, incubated at a temperature of about 31 °C to about 40 °C, and agitated at a frequency of about 20 RPM to about 1000 RPM (e.g., about 20 RPM to about 400 RPM, about 120 RPM to about 240 RPM, about 140 RPM to about 220 RPM, about 160 RPM to about 180 RPM, about 400 RPM to about 600 RPM, about 600 RPM to about 800 RPM, or about 800 RPM to about 1000 RPM). The use of a reaction angle of about 5 degrees to about 85 degrees (e.g., at a reactor angle of about 10 degrees to about 85 degrees, about 15 degrees to about 85 degrees, about 25 degrees to about 85 degrees, or about 40 degrees to about 55 degrees) from horizontal results in an increase concentration of dissolved $O_2$ and/or better gas mixing in the first and/or second liquid culture medium than use of a reaction angle of about 90 degrees from horizontal.

The agitation can be performed using a humidified atmosphere controlled incubator (e.g., at a humidity of greater than 20%, 30%, 40%, 50%, 60%, 70%, 75%, 80%, 85%, 90%, or 95%, or a humidity of 100%) with a mechanical device that provides the agitation of one or more of the containers containing the mammalian cell and a liquid culture medium (e.g., the first and/or second liquid culture medium).

*Temperature*

The culturing methods described herein can be performed at a temperature of about 31 °C to about 40 °C. Skilled practitioners will appreciate that the temperature can be changed at specific time point(s) in the culturing method, e.g., on an hourly or daily basis. For example, the temperature can be changed or shifted (e.g., increased or decreased) at about one day, two days, three days, four days, five days, six days, seven days, eight days, nine days, ten days, eleven days, twelve days, fourteen days, fifteen days, sixteen days, seventeen days, eighteen days, nineteen days, or about twenty days or more after the initial seeding of the container with the mammalian cell). For example, the temperature can be shifted upwards (e.g., a change of up to or about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, or up to or about 20 degrees C). For example, the temperature can be shifted downwards (e.g., a change of up to or about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, or up to or about 20 °C).

### *Exemplary Combinations of Conditions*

Described in this subsection are some exemplary combinations of conditions that can be used in any of the methods described herein having a container (e.g., a gas-permeable conical container) with a volume, e.g., of about 30 mL to about 100 mL, e.g., about 50 mL. In some examples, the methods use a gas-permeable conical container having a volume of about 30 mL to about 60 mL (e.g., about 50 mL) cultured at a reactor angle of between about 5° to about 15° (e.g., between about 3° to about 7°) and, when the volume of liquid culture medium is between about 2% and about 10% (e.g., between about 2% and about 8% or between about 2% and about 6%) of the volume of the container the agitation is at least 150 RPM (e.g., between about 220 RPM to 300 RPM, e.g., between about 240 RPM to about 280 RPM), or when the volume of liquid culture medium is at least 10% of the volume of the container (e.g., between about 10% to about 40%, e.g., between about 10% to about 30% or between about 15% to about 25% of the volume of the container) the agitation is between about 50 RPM to about 150 RPM (e.g., between about 65 RPM to about 105 RPM).
In some examples, the methods use a gas-permeable conical container having a volume of about 30 mL to about 60 mL (e.g., about 50 mL) cultured at a reactor angle of between about 15° to about 25° (e.g., between about 18° to about 22°) and, when the volume of liquid culture medium is at least 25% (e.g., at least 30% or at least 35%, or between about 35% and about 45% or between about 37% and about 43%) of the volume of the container, the agitation is at least 250 RPM (e.g., at least 300 RPM or at least 320 RPM, or between about 300 RPM and about 360 RPM).
In some examples, the methods use a gas-permeable conical container having a volume of about 30 mL to about 60 mL (e.g., about 50 mL) cultured at a reactor angle of between about 25° to about 35° (e.g., between about 27° to about 33°) and, when the volume of liquid culture medium is between about 2% and about 15% (e.g., between about 2% and about 12%, between about 2% and about 10%, or between about 2% and about 8%) of the volume of the container, the agitation is between about 20 RPM and about 160 RPM (e.g., between about 60 RPM and about 160 RPM, between about 80 RPM and about 160 RPM, or between about 100 RPM and about 140 RPM), or when the volume of liquid culture medium is between about 20% to about 40% (e.g., between about 25% to about 40%, between about 25% to about 35%, or between about 30% to about 34%) of the volume of the container, the agitation is at least 200 RPM (e.g., at least about 220 RPM or at least about 240 RPM, or between about 200 RPM and about 300 RPM, between about 220 RPM and about 280 RPM, or between about 235 RPM and about 275 RPM).
In some examples, the methods use a gas-permeable conical container having a volume of about 30 mL to about 60 mL (e.g., about 50 mL) cultured at a reactor angle of between about 35° to about 55° (e.g., between about 40° to about 50° or between about 42° to about 48°) and, when the volume of liquid culture medium is between about 2% to about 30% (e.g., between about 4% and about 28%, between about 6% and about 28%, between about 10% and about 25%, between about 15% and about 25%, or between about 18% and about 22%) of the volume of the container, the agitation is between about 100 RPM and about 220 RPM (e.g., between about 120 RPM and about 200 RPM, between about 120 RPM and about 180 RPM, or between about 140 RPM and about 180 RPM).
In some examples, the methods use a gas-permeable conical container having a volume of about 30 mL to about 60 mL (e.g., about 50 mL) cultured at a reactor angle of between about 55° to about 62° (e.g., between about 58° to about 62°) and, when the volume of liquid culture media is between about 2% and about 20% (e.g., between about 2% and about 15%, between about 2% and about 10%, or between about 2% and about 6%) of the volume of the container, the agitation is at least 230 RPM (e.g., at least 240 RPM, at least 250 RPM, or at least 260 RPM, or between about 230 RPM and about 280 RPM or between about 240 RPM and about 270 RPM), or when the volume of liquid culture media is at least 40% (e.g., at least 45%, at least 50%, at least 55%, or at least 60%, or between about 40% and about 80%, between about 45% and about 75%, between about 50% and about 70%, between about 55% and about 65%, or between about 58% and 62%) of the container volume the agitation is between about 100 RPM and about 230 RPM (e.g., between about 120 RPM and about 210 RPM, between about 140 RPM and about 190 RPM, between about 150 RPM and about 210 RPM, between about 160 RPM and about 210 RPM, or between about 178 RPM and about 198 RPM).
In some examples, the methods use a gas-permeable conical container having a volume of about 30 mL to about 60 mL (e.g., about 50 mL) cultured at a reactor angle of between about 60° to about 70° (e.g., between about 62° and about 68°), a volume of liquid culture medium that is at least 70% (e.g., at least 75% or at least 80%, or between about 60% and about 90%, between about 70% and about 90%, or between about 75% and about 85%) of the volume of the container and an agitation of at least 200 RPM (e.g., at least 220 RPM, at least 240 RPM, or at least 250 RPM, or between about 210 RPM and about 290 RPM, between about 230 RPM and about 270 RPM, or between about 240 RPM and about 260 RPM).

In some examples, the methods use a gas-permeable conical container having a volume of about 30 mL to about 60 mL (e.g., about 50 mL) cultured at a reactor angle of between about 70° and about 85° (e.g., between about 75° and about 85° or between about 80° and about 85°) and, when the volume of liquid culture media is between about 30% and about 55% (e.g., between about 35% and about 50% or between about 40% and about 45%) the agitation is between about 280 RPM and about 380 RPM (e.g., between about 290 RPM and about 370 RPM, between about 280 RPM and about 360 RPM, between about 290 RPM and about 350 RPM, between about 300 RPM and about 340 RPM, between about 320 RPM and about 340 RPM), or when the volume of liquid culture media is between about 60% and about 90% (e.g., between about 60% and about 80%, between about 65% and about 85%, between about 65% and about 80%, or between about 65% and about 75%), the agitation is between about 280 RPM and about 380 RPM (e.g., between about 290 RPM and about 370 RPM, between about 280 RPM and about 360 RPM, between about 290 RPM and about 350 RPM, between about 300 RPM and about 340 RPM, or between about 320 RPM and about 340 RPM).

In some examples, the container (e.g., a gas-permeable conical container having a volume of about 30 mL to about 60 mL (e.g., about 50 mL)) contains a volume of liquid culture medium that is between about 40% and about 60% (e.g., between about 45% and about 55%) of the container volume, is placed at a reactor angle of between about 45° and about 70° (e.g., between about 45% and about 60%), and agitated at least 200 RPM (e.g., at least 220 RPM, at least 240 RPM, at least 260 RPM, or at least 280 RPM).

In general, a lower volume of liquid culture medium in a container typically grows best with a low reactor angle, and a higher volume typically grows best with a higher rate of agitation.

### *Culture Medium Removal and Replacement*

The methods described herein include removing from the container a first volume of a first liquid culture medium (e.g., containing any concentration of mammalian cells, e.g., a first volume of a first liquid culture medium that is substantially free of cells), and adding to the first liquid culture medium a second volume of a second liquid culture medium. Removal and adding can be performed simultaneously or sequentially, or a combination of the two. Further, removal and adding can be performed continuously (e.g., at a rate that removes and replaces a volume of between 0.1% to 800% (e.g., between 1% and 700%, between 1% and 600%, between 1% and 500%, between 1% and 400%, between 1% and 350%, between 1% and 300%, between 1% and 250%, between 1% and 100%, between 100% and 200%, between 5% and 150%, between 10% and 50%, between 15% and 40%, between 8% and 80%, and between 4% and 30%) of the volume of the container or the first liquid culture medium volume over any given time period (e.g., over a 24-hour period, over an incremental time period of about 1 hour to about 24 hours, or over an incremental time period of greater than 24 hours)) or periodically (e.g., once every third day, once every other day, once a day, twice a day, three times a day, four times a day, or five times a day), or any combination thereof. Where performed periodically, the volume that is removed or replaced (e.g., within about a 24-hour period, within an incremental time period of about 1 hour to about 24 hours, or within an incremental time period of greater than 24 hours) can be, e.g., between 0.1% to 800% (e.g., between 1% and 700%, between 1% and 600%, between 1% and 500%, between 1% and 400%, between 1% and 300%, between 1% and 200%, between 1% and 100%, between 100% and 200%, between 5% and 150%, between 10% and 50%, between 15% and 40%, between 8% and 80%, and between 4% and 30%) of the volume of the container or the first liquid culture medium volume. The first volume of the first liquid culture medium removed and the second volume of the second liquid culture medium added can in some instances be held approximately the same over each 24-hour period (or, alternatively, an incremental time period of about 1 hour to about 24 hours or an incremental time period of greater than 24 hours) over the entire or part of the culturing period. As is known in the art, the rate at which the first volume of the first liquid culture medium is removed (volume/unit of time) and the rate at which the second volume of the second liquid culture medium is added (volume/unit of time) can be varied. The rate at which the first volume of the first liquid culture medium is removed (volume/unit of time) and the rate at which the second volume of the second liquid culture medium is added (volume/unit of time) can be about the same or can be different.

Alternatively, the volume removed and added can change (e.g., gradually increase) over each 24-hour period (or alternatively, an incremental time period of between 1 hour and about 24 hours or an incremental time period of greater than 24 hours) during the culturing period. Non-limiting examples of methods that include a gradual increase in volumes are described herein. For example the volume of the first liquid culture medium removed and the volume of the second liquid culture medium added within each 24-hour period (or alternatively, an incremental time period of between about 1 hour and above 24 hours or an incremental time period of greater than 24 hours) over the culturing period can be increased (e.g., gradually or through staggered increments) over the culturing period from a volume that is between 0.5% to about 20% of the container volume or the first liquid culture medium volume to about 25% to about 150% of the container volume or the first liquid culture medium volume.

Skilled practitioners will appreciate that the first liquid culture medium and the second liquid culture medium can be the same type of media. In other instances, the first liquid culture medium and the second liquid culture medium can be different.

The first volume of the first liquid culture medium can be removed, e.g., by centrifuging (e.g., slow-speed swinging bucket centrifugation) the container, and removing the first volume of the first liquid culture that is substantially free of cells from the supernatant. Alternatively or in addition, the first volume of the first liquid culture medium can be removed by seeping or gravity flow of the first volume of the first liquid culture medium through a sterile membrane with a molecular weight cut-off that excludes the mammalian cell.

The second volume of the second liquid culture medium can be added to the first liquid culture medium, e.g., by perfusion pump. The second liquid culture medium can be added to the first liquid culture medium manually (e.g., by pipetting the second volume of the second liquid culture medium directly onto the first liquid culture medium) or in an automated fashion.

In some instances, removing the first volume of the first liquid culture medium (e.g., a first volume of the first liquid culture medium that is substantially free of mammalian cells) and adding to the first liquid culture medium a second volume of the second liquid culture medium does not occur within at least 1 hour (e.g., within 2 hours, within 3 hours, within 4 hours, within 5 hours, within 6 hours, within 7 hours, within 8 hours, within 9 hours, within 10 hours, within 12 hours, within 14 hours, within 16 hours, within 18 hours, within 24 hours, within 36 hours, within 48 hours, within 72 hours, within 96 hours, or after 96 hours) of the seeding of the container with a mammalian cell.

### $CO_2$

Methods described herein can further include incubating the container in an atmosphere containing at most or about 15% $CO_2$ (e.g., at most or about 14% $CO_2$, 12% $CO_2$, 10% $CO_2$, 8% $CO_2$, 6% $CO_2$, 5% $CO_2$, 4% $CO_2$, 3% $CO_2$, 2% $CO_2$, or at most or about 1% $CO_2$). Moreover, any of the methods described herein can include incubating the container in a humidified atmosphere (e.g., at least or about 20%, 30%, 40%, 50%, 60%, 70%, 85%, 80%, 85%, 90%, or at least or about 95% humidity, or about 100% humidity).

### Exemplary Devices

Non-limiting examples of devices that can be used to perform the culturing methods described herein include: Appropriate Technical Resources (Maryland, USA) distributes INFORS Multiron shake incubator (INFORS; Basel, Switzerland), and Kuhner shake incubator (Kuhner AG; Basel, Switzerland). Non-limiting examples of devices that can be used to perform the culturing methods include a rotary incubator with a throw (orbit) diameter of between about 3 mm to about 50 mm (e.g., between about 1 mm and about 25 mm, or between about 25 mm and about 50 mm). Additional examples of shake incubators and rolling culture incubators are known in the art.

### Dissolved $O_2$ and Liquid Sheer Force

Also disclosed are culturing methods that include culturing in a gradient perfusion process a mammalian cell suspended in a liquid culture medium under conditions that generate in the medium a fluid sheer force and dissolved oxygen ($O_2$) concentration that are the same as (or essentially the same as) that achieved in a gas-permeable conical container containing 4% to 80% (e.g., 4% to 40% or 4% to 30%) volume of the container of liquid culture medium when positioned at a reactor angle of about 5 degrees to about 85 degrees (e.g., about 10 degrees to about 85 degrees, about 15 degrees to about 85 degrees, about 25 degrees to about 85 degrees, or about 40 degrees to about 55 degrees) from horizontal, incubated at a temperature of about 31 °C to about 40 °C, and agitated at a frequency of about 20 revolutions per minute (RPM) to about 1000 RPM (e.g., about 20 RPM to about 400 RPM, about 120 RPM to about 240 RPM, about 140 RPM to about 220 RPM, about 160 RPM to about 180 RPM, about 400 RPM to about 600 RPM, about 600 RPM to about 800 RPM, or about 800 RPM to about 1000 RPM).

As is known in the art, a variety of cell culture parameters can be adjusted to achieve a specific dissolved $O_2$ concentration and a specific fluid sheer force. Non-limiting examples of such parameters that can be adjusted include: the container volume, the volume of the liquid culture medium, the shape of the container, the type of agitation (e.g., rotating, tilting, and/or rolling), the frequency of agitation, the type of liquid culture medium, the interior coating of the container, and the temperature of the liquid culture medium. Additional examples of such culture parameters are known in the art. Any combination of culture parameters described herein or known in the art can be combined in any fashion to achieve in the culture medium a fluid sheer force and dissolved oxygen ($O_2$) concentration that is the same as (or essentially the same as) that achieved in a gas-permeable conical container containing about 4% to about 80% (e.g., about 4% to about 70%, about 4% to about 60%, or about 4% to about 30%) volume of the container of liquid culture medium when positioned at a reactor angle of about 5 degrees to about 85 degrees (e.g., about 10 degrees to about 85 degrees, about 15 degrees to about 85 degrees, about 25 degrees to about 85 degrees, or about 40 degrees to about 55 degrees) from horizontal, incubated at a temperature of about 31 °C to about 40 °C, and agitated at a frequency of about 20 revolutions per minute (RPM) to about 1000 RPM (e.g., about 20 RPM to about 400 RPM, about 120 RPM to about 240 RPM, about 140 RPM to about 220 RPM, about 160 RPM to about 180 RPM, about 400 RPM to about 600 RPM, about 600 RPM to about 800 RPM, or about 800 RPM to about 1000 RPM).

Dissolved $O_2$ levels in a liquid culture medium can be detected using a variety of different methods. For example, dissolved $O_2$ can be measured using a dissolved $O_2$ electrode or probe (for example, the $O_2$ probes and electrodes available from Eutech Instruments WD-35201-80 Dissolved Oxygen Probe, Rosemount Analytical 499 Series Dissolved Oxygen/Ozone Sensor, and Extech DO705 Dissolved Oxygen Electrode). Methods of calibrating and using the $O_2$ probes and electrodes can be performed using the manufacturer's instructions.

The sheer fluid force in a liquid culture medium can be calculated using methods known in the art. A non-limiting example of a suitable textbook that describes the calculation of liquid sheer force in liquid culture medium is described in Fluid Mechanics, Robert A. Granger, 1995, Dover Publications, Inc., Mineola, NY, and Fundamentals of Fluid Mechanics, Bruce R. Munson et al., John Wiley & Sons, Inc., 2009.

## Methods of Producing a Recombinant Protein

Also disclosed herein are methods of producing a recombinant protein, which include culturing a cell that is capable of producing the recombinant protein using a method described herein. Following performance of the method, the recombinant protein can be recovered from the mammalian cell and/or from the first or second culture medium. In some embodiments, the recombinant protein is recovered from the first and/or second liquid culture medium at any given time point during the culturing method (e.g., recovered from the first and/or second liquid culture medium on one or more of days 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 of culture, or after more than 100 days of culture). Some embodiments of these methods further include adding a volume of a third culture medium or a volume of a fourth liquid culture medium, but in each instance the total volume of liquid culture medium in the container should be about equal or less than the first liquid culture medium volume.

Skilled practitioners will appreciate that any of the various culture parameters (e.g., containers, volumes, rates or frequencies of replacing culture volumes, agitation frequencies, temperatures, media, and $CO_2$ concentrations) can be used in any combination in to perform these methods. Further, any of the mammalian cells described herein or known in the art can be used to produce a recombinant protein.

A nucleic acid encoding a recombinant protein can be introduced into a mammalian cell using a wide variety of methods known in molecular biology and molecular genetics. Non-limiting examples include transfection (e.g., lipofection), transduction (e.g., lentivirus, adenovirus, or retrovirus infection), and electroporation. In some instances, the nucleic acid that encodes a recombinant protein is not stably integrated into a chromosome of the mammalian cell (transient transfection), while in others the nucleic acid is integrated. Alternatively or in addition, the nucleic acid encoding a recombinant protein can be present in a plasmid and/or in a mammalian artificial chromosome (e.g., a human artificial chromosome). Alternatively or in addition, the nucleic acid can be introduced into the cell using a viral vector (e.g., a lentivirus, retrovirus, or adenovirus vector). The nucleic acid can be operably linked to a promoter sequence (e.g., a strong promoter, such as a β-actin promoter and CMV promoter, or an inducible promoter). A vector containing the nucleic acid can, if desired, also contain a selectable marker (e.g., a gene that confers hygromycin, puromycin, or neomycin resistance to the mammalian cell).

In some instances, the recombinant protein is a secreted protein and is released by the mammalian cell into the extracellular medium (e.g., the first and/or second liquid culture medium). For example, a nucleic acid sequence encoding a soluble recombinant protein can contain a sequence that encodes a secretion signal peptide at the N- or C-terminus of the recombinant protein, which is cleaved by an enzyme present in the mammalian cell, and subsequently released into the extracellular medium (e.g., the first and/or second liquid culture medium). In other instances, the recombinant protein is a soluble protein that is not secreted, and the recombinant protein is recovered from within the mammalian cell.

Non-limiting examples of recombinant proteins that can be produced by the methods provided herein include immunoglobulins (including light and heavy chain immunoglobulins, antibodies, or antibody fragments (e.g., any of the antibody fragments described herein), enzymes (e.g., a galactosidase (e.g., an alpha-galactosidase), Myozyme®, or Cerezyme®), proteins (e.g., human erythropoietin, tumor necrosis factor (TNF), or an interferon alpha or beta), or immunogenic or antigenic proteins or protein fragments (e.g., proteins for use in a vaccine). Additional examples of recombinant proteins that can be produced by the methods provided herein are listed in Table 1, as well as the different diseases that each exemplary recombinant protein can be used to treat. In some embodiments, the recombinant protein is an engineered antigen-binding polypeptide that contains at least one multifunctional recombinant protein scaffold (see, e.g., the recombinant antigen-binding proteins described in Gebauer et al., Current Opin. Chem. Biol. 13:245-255, 2009; and U.S. Patent Application Publication No. 2012/0164066). Non-limiting examples of recombinant proteins that are antibodies include: panitumumab, omalizumab, abagovomab, abciximab, actoxumab, adalimumab, adecatumumab, afelimomab, afutuzumab, alacizumab, alacizumab, alemtuzumab, alirocumab, altumomab, amatuximab, anatumomab, apolizumab, atinumab, tocilizumab, basilizimab, bectumomab, belimumab, bevacizumab, biciromab, canakinumab, cetuximab, daclizumab, densumab, eculizumab, edrecolomab, efalizumab, efungumab, ertumaxomab, etaracizumab, golimumab, infliximab, natalizumab, palivizumab, panitumumab, pertuzumab, ranibizumab, rituximab, tocilizumab, and trastuzumab. Additional examples of therapeutic antibodies that can be produced by the methods described herein are known in the art. Additional non-limiting examples of recombinant proteins that can be produced by the present methods include: alglucosidase alfa, laronidase, abatacept, galsulfase, lutropin alfa, antihemophilic factor, agalsidase beta, interferon beta-la, darbepoetin alfa, tenecteplase, etanercept, coagulation factor IX, follicle stimulating hormone, interferon beta-la, imiglucerase, dornase alfa, epoetin alfa, and alteplase.

**Table 1. List of Exemplary Recombinant Proteins and Diseases Treated with Each Exemplary Protein**

| Lysosomal storage diseases and associated enzymatic defects | |
|---|---|
| Disease | Enzymatic Defect |
| Pompe disease | acid $\alpha$-glucosidase (e.g., Myozyme®, Lumizyme®) |
| MPSI* (Hurler disease) | $\alpha$-L-iduronidase (e.g., Aldurazyme®) |
| MPSII (Hunter disease) | iduronate sulfatase |
| MPSIII (Sanfilippo) | heparan N-sulfatase |
| MPS IV (Morquio A) | galactose-6-sulfatase |
| MPS IV (Morquio B) | acid $\beta$-galactosidase |
| MPS VII (Sly disease) | $\beta$-glucoronidase |
| I-cell disease | N-acetylglucosamine-1-phosphotransferase |
| Schindler disease | $\alpha$-N-acetylgalactosaminidase ($\alpha$-galactosidase B) |
| Wolman disease | acid lipase |
| Cholestrol ester storage disease | acid lipase |
| Farber disease | lysosomal acid ceramidase |
| Niemann-Pick disease | acid sphingomyelinase |
| Gaucher disease | $\beta$-glucosidase (e.g. Cerezyme®, Ceredase®) |
| Krabbe disease | galactosylceramidase |
| Fabry disease | $\alpha$-galactosidase A |
| GM1 gangliosidosis | acid $\beta$-galactosidase |
| Galactosialidosis | $\beta$-galactosidase and neuraminidase |
| Tay-Sach's disease | hexosaminidase A |
| Sandhoff disease | hexosaminidase A and B |
| *MPS = mucopolysaccaridosis | |

A secreted, soluble recombinant protein can be recovered from the liquid culture medium (e.g., the first and/or second liquid culture medium) by removing or otherwise physically separating the liquid culture medium from the mammalian cells. A variety of different methods for removing liquid culture medium from mammalian cells are known in the art, including, for example, centrifugation, filtration, pipetting, and/or aspiration. The secreted recombinant protein can then be recovered and further purified from the liquid culture medium using a variety of biochemical techniques including various types of chromatography (e.g., affinity chromatography, molecular sieve chromatography, cation exchange chromatography, or anion exchange chromatography) and/or filtration (e.g., molecular weight cut-off filtration).

To recover an intracellular recombinant protein, the mammalian cell can be lysed. A wide variety of methods for lysing mammalian cells are known in the art, including, for example, sonication and/or detergent, enzymatic, and/or chemical lysis. A recombinant protein can be purified from a mammalian cell lysate using a variety of biochemical methods known in the art, typically starting with a step of centrifugation to remove the cellular debris, and then one or more additional steps (e.g., one or more types of chromatography (e.g., affinity chromatography, molecular sieve chromatography, cation exchange chromatography, or anion exchange chromatography) and/or filtration (e.g., molecular weight cut-off filtration)).

In some embodiments, the recovered recombinant protein is at least or about 50% pure by weight, e.g., at least or about 55% pure by weight, at least 60% pure by weight, at least 65% pure by weight, at least 70% pure by weight, at least 75% pure by weight, at least 80% pure by weight, at least 85% pure by weight, at least 90% pure by weight, at least 95% pure by weight, at least 96% pure by weight, at least 97% pure by weight, at least 98% pure by weight, or at least or about 99% pure by weight, or greater than 99% pure by weight.

**Methods for Testing a Manufacturing Process**

Also disclosed herein are methods for testing a manufacturing process for making a recombinant protein. These methods include performing a method of producing a recombinant protein described above and, during the method and/or afterward, detecting or measuring at least one (e.g., two, three four, five, six, seven, eight, nine, ten, eleven, twelve, thirteen, or fourteen) culture readout (e.g., the recombinant protein in the cell or in the first and/or second culture medium, glucose consumption, viable cell concentration, lactate production, volumetric productivity, specific productivity, lactate yield from glucose, glutamine concentration, glutamate concentration, pH of culture medium, partial pressure or concentration of dissolved $CO_2$, concentration or partial pressure of dissolved $O_2$, metabolite mass transfer, and metabolite mass balance); and comparing the at least one culture readout to a reference level of the at least one (e.g., two, three, four, five, six, seven, eight, nine, ten, eleven, twelve, or thirteen) culture readout (e.g., a reference level of the amount of recombinant protein present in the cell or in the first and/or second culture medium, glucose consumption, viable cell concentration, lactate production, volumetric productivity, specific productivity, lactate yield from glucose, glutamine concentration, glutamate concentration, pH of culture medium, concentration or partial pressure of dissolved $CO_2$, concentration or partial pressure of dissolved $O_2$, metabolite mass transfer, and metabolite mass balance).

Skilled practitioners will appreciate that any of the various culture parameters (e.g., containers, volumes, rates or frequencies of replacing culture volumes, agitation frequencies, temperatures, media, $CO_2$ concentrations, and reactor angle) described herein can be used in any combination in to perform these methods. Further, any of the mammalian cells described herein or known in the art can be used in the methods.

The reference level of the at least one culture readout (e.g., a level of recombinant protein in the cell or in the first and/or second culture medium, glucose consumption, viable cell concentration, lactate production, volumetric productivity, specific productivity, lactate yield from glucose, glutamine concentration, glutamate concentration, pH of culture medium, concentration or partial pressure of dissolved $CO_2$, concentration or partial pressure of dissolved $O_2$, metabolite mass transfer, and metabolite mass balance) can be a level produced using a different culturing method, e.g., a culturing method that utilizes at least one different culture parameter (e.g., a different first and/or second liquid culture medium, a different mammalian cell, a different frequency and/or type of agitation, a different reactor angle of placement of the conical container, a different batch refeed or perfusion rate (e.g., 10% to 200% of the container volume or the first liquid culture medium volume over a 24-hour time period or other incremental time period), and any of the other culture parameters described herein). The reference level of recombinant protein can be, e.g., a level of recombinant protein produced using a set of culturing parameters that result in a different level of dissolved $O_2$ and/or a different level of liquid sheer stress.

The methods described herein can be used to test the effect of any component or feature of a manufacturing process. For example, the method described herein can be used to test the effect of different raw materials, agitation levels, containers, anti-clumping agents, culture media (e.g., chemically-defined culture media), or nutrient elements or compounds on the at least one culture readout (e.g., any of the culture readouts described herein, e.g., the effect on recombinant protein production and/or mammalian cell growth). For example, disclosed herein are methods of testing the efficacy of a first or second liquid culture medium, a raw ingredient or supplement present in a first or second liquid culture medium, or a source of a mammalian cell for use in a method of producing a recombinant protein that include providing a conical container containing a mammalian cell suspended in a first liquid culture medium occupying about 4% to about 80% of the volume of the container; incubating the container for a period of time at about 31 °C to about 40 °C at a reactor angle of about 5 degrees to about 85 degrees from horizontal and with an agitation of about 20 revolutions per minute (RPM) to about 1000 RPM; continuously or periodically, during the period of time, removing a first volume of the first liquid culture medium and adding to the first liquid culture medium a second volume of a second liquid culture medium, where the first and second volumes are about equal; detecting or determining at least one culture readout (e.g., any of the culture readouts described herein, e.g., the recombinant protein in the cell or in the first and/or second culture medium); comparing the at least one culture readout to a reference level of the at least one culture readout (e.g., any of the culture readouts described herein, e.g., a recombinant protein in the cell or in the first and/or second liquid culture medium) produced by a different culturing method that uses one or more of a different first or second liquid culture medium, or a different source of a mammalian cell; and identifying the first or second liquid culture medium, the raw ingredient or supplement present in the first or second liquid culture medium, or the source of the mammalian cell that is associated with beneficial change (e.g., increase or decrease) in the at least one culture readout (e.g., an increased amount of recombinant protein) as compared to the reference level as being efficacious for use in a method of producing a recombinant protein. For example, an increase in recombinant protein level, an increase in viable cell concentration, an increase in volumetric productivity, an increase in specific productivity, and an increase in glucose consumption compared to the reference level indicates that the first or second liquid culture medium, the raw ingredient or supplement present in a first or second liquid culture medium, or the source of the mammalian cell are efficacious for use in a method of producing a recombinant protein.

The methods described herein can also be used to test the effect of changing any of the various cell culturing parameters described herein or known in the art (e.g., the volume or shape of a container, the frequency of agitation, the sheer force, the culture seeding density, the pH of the first or second liquid culture medium, dissolved $O_2$ concentration or partial pressure, the inner surface coating of the container, the various contents within a liquid culture media (e.g., the first and/or second liquid culture media), the amount and/or type of agitation, the mammalian cell type or line, the $CO_2$ exposure or dissolved $CO_2$ concentration or partial pressure, the temperature, the volume of liquid culture medium (e.g., the volume of the first and/or second liquid culture media), and/or the rate or frequency of removing the first volume of the first culture medium and adding the second volume of the second culture medium to the first culture medium). The methods can also be used to test the quality of water used to prepare the liquid culture medium (e.g., the first and/or second liquid culture medium) and/or the effect of different trace metals in the liquid culture medium on at least one culture readout (e.g., any of the culture readouts described herein, e.g., the effect on recombinant protein production and/or mammalian cell growth). The methods can also be used to test the effect of a growth factor or growth hormone on at least one culture readout (e.g., any of the culture readouts described herein, e.g., the effect on recombinant protein production and/or mammalian cell growth). The method can also be used to test filtration processes and filters used to prepare the first and/or second liquid culture medium. The method can also be used to test liquid culture medium stability and the effect of a liquid culture medium on biological functions (e.g., at least one of any of the culture readouts described herein, e.g., the effect on recombinant protein production and/or mammalian cell growth). The method can also be used to screen various recombinant cell lines and cell banks for their ability to produce a desired recombinant protein (e.g., a desired secreted therapeutic protein). As noted herein, the method can also be used to screen any cell culture process parameter, including but limited to, the type and frequency of agitation, sheer force, perfusion rate and volume, culture seeding density, and others.

The method described herein can also be used to test for the presence of a contaminant in a first or second liquid culture medium, a raw material used to generate a first or second liquid culture medium, or a source of a mammalian cell. For example, disclosed herein are methods of testing for the presence of a contaminant in a first or second liquid culture medium, raw materials used to generate a first or second liquid culture medium, or a source of a mammalian cell that include providing a conical container containing a mammalian cell suspended in a first liquid culture medium occupying about 4% to about 80% of the volume of the container; incubating the container for a period of time at about 31 °C to about 40 °C at a reactor angle of about 5 degrees to about 85 degrees from horizontal and with an agitation of about 20 revolutions per minute (RPM) to about 1000 RPM; continuously or periodically, during the period of time, removing a first volume of the first liquid culture medium and adding to the first liquid culture medium a second volume of a second liquid culture medium, where the first and second volumes are about equal; detecting or determining at least one culture readout (e.g., any of the culture readouts described herein, e.g., the recombinant protein in the cell or in the first and/or second liquid culture medium); comparing the at least one culture readout to a reference level of the at least one culture readout (e.g., any of the culture readouts described herein, e.g., amount of recombinant protein present in the cell or in the first and/or second culture medium) produced by a different culturing method that uses one or more of a different first or second liquid culture medium, different raw materials to generate the first or second liquid culture medium, or a different source of the mammalian cell; and identifying the first or second liquid culture medium, the raw materials used to generate the first or second liquid culture medium, or the source of a mammalian cell as containing a contaminant when the level of the at least one culture parameter is detrimentally changed (e.g., increased or decreased) compared to the reference level. For example, a decrease in recombinant protein production (e.g., a decrease in recombinant protein in the cell or in the first and/or second culture medium), volumetric productivity, or viable cell concentration as compared to the reference level is a detrimental change that indicates the presence of a contaminant in the first or second liquid culture medium, a raw material used to generate the first or second liquid culture medium, or the source of the mammalian cell. Some methods further include one or more assays to determine the identity of the contaminant present in the first or second liquid culture medium, the raw material used to generate the first or second liquid culture medium, or the source of the mammalian cell. The contaminant can be a biological contaminant (e.g., a mycobacterium, a fungus, a bacterium, a virus, or an undesired mammalian cell). The contaminant can also be a physically uncharacterized substance.

The methods can used to conduct high throughput cell culture experiments to perform a design-of-experiment (DOE) or a quality-by-design (QBD) optimization of cell culturing methods. For example, disclosed herein are methods of optimizing a manufacturing process of producing a recombinant protein that include providing a conical container containing a mammalian cell suspended in a first liquid culture medium occupying about 4% to about 80% of the volume of the container; incubating the container for a period of time at about 31 °C to about 40 °C at a reactor angle of about 5 degrees to about 85 degrees from horizontal and with an agitation of about 20 revolutions per minute (RPM) to about 1000 RPM; continuously or periodically, during the period of time, removing a first volume of the first liquid culture medium and adding to the first liquid culture medium a second volume of a second liquid culture medium, where the first and second volumes are about equal; detecting at least one culture readout (e.g., any of the culture readouts described herein, e.g., amount of recombinant protein in the cell or in the first and/or second liquid culture medium); comparing the at least one culture readout to a reference level of the at least one culture readout (e.g., any of the culture readouts described herein, e.g., amount of recombinant protein present in the cell or in the first and/or second liquid culture medium) produced by a different culture method; and identifying and removing or altering in the manufacturing process any culture components or parameters that are associated with a detrimental change (e.g., increase or decrease) in the at least one culture readout (e.g., any of the culture readouts described herein, e.g., amount of recombinant protein produced) as compared to the reference level of the at least one culture readout (e.g., any of the culture readouts described herein, e.g., recombinant protein produced), or identifying and adding to a manufacturing process any culture components or parameters that are associated with a beneficial change (e.g., increase or decrease) in the at least one culture readout (e.g., any of the culture readouts described herein, e.g., amount of recombinant protein produced) as compared to the reference level of the at least one culture readout (e.g., any of the culture readouts described herein, e.g., recombinant protein produced). For example, an increase in the amount of recombinant protein produced, volumetric productivity, specific productivity, or viable cell concentration is a beneficial change in a culture readout, and a decrease in the amount of recombinant protein produced, volumetric productivity, specific productivity, or viable cell concentration is a detrimental change in a culture readout. In some instances, the method is used to identify in a high throughput fashion, optimized cell culture conditions that can be used for up-scaled (e.g., bioreactor) production of a recombinant protein.

In any of the methods described in this section, the reference level of the at least one culture readout can be from a larger-scale culture (e.g., a perfusion bioreactor, e.g., a 2000-L perfusion bioreactor, 40-L perfusion bioreactor, or a 12-L perfusion bioreactor). In some embodiments of any of the methods described in this section, the mammalian cell is cultured in a conical container using any of the methods described herein over the same time period that a larger-scale culture is performed (cultured in paralleled). For example, the inoculum used to inoculate the conical container in any of the methods described herein is also used to inoculate a larger-scale perfusion bioreactor at approximately the same time.

In one embodiment, the inoculum that is used to seed the conical container is obtained from a larger-scale culture (e.g., a larger-scale perfusion bioreactor). For example, an aliquot from a larger-scale culture at any time point (e.g., removed during the growth phase, the transition phase (e.g., an optional period when the culture is being transitioned to a different set of growth conditions, e.g., a different liquid culture medium and/or temperature), or the harvest phase) and used to inoculate the conical container (e.g., used to start a satellite conical container culture). An aliquot can be removed from the larger-scale culture during the growth phase and used to inoculate or seed a conical container containing a liquid culture medium, and the conical container is then incubated under conditions that replicate or are similar to the growth phase conditions employed in the larger-scale culture. An aliquot can alternatively, or additionally, be removed from the larger-scale culture during a transition phase and used to inoculate or seed a conical container containing a liquid culture medium, and the conical container is then incubated under conditions that replicate or are similar to the transition phase conditions employed in the larger-scale culture. An aliquot can alternatively, or additionally, be removed from the larger-scale culture during the harvest phase and used to inoculate or seed a conical container containing a liquid culture medium, and the conical container is then incubated under conditions that replicate or are similar to the harvest phase conditions employed in the larger-scale culture. In any of these methods, one or more culture parameters can be altered in the methods used to culture the mammalian cell in the conical containers (as compared to the culture parameters or components used to culture the mammalian cell in the larger-scale culture), at least one culture readout is measured, and the at least one culture readout is compared to the at least one culture readout determined for the larger-scale culture. As can be appreciated by those in the art, these methods can be used to test the effect of a specific culture parameter or component on at least one culture readout during one or more specific phases in the culturing process (e.g., the effect of one or more culture parameters and/or culture component(s) on at least one culture readout during the growth phase, optional transition phase, and/or harvest phase).

In certain disclosed embodiment, these method can also be performed to determine whether a contaminant is present in the larger-scale bioreactor, by determining or detecting at least one culture readout in the conical container culture, comparing the at least one culture readout to a reference level of the at least one culture readout (e.g., a level of the at least one culture readout from a culture that is substantially free of contamination), and identifying the larger-scale bioreactor as containing a contaminant when the at least one culture readout in the shake flask culture as compared to the reference level of the at least one culture readout indicates that a contaminant is present in the shake flask. The contaminant can be, for example, a biological contaminant, such as a virus, a fungus, an undesired mammalian cell, or a bacterium, such as a mycobacterium. The contaminant can be, for example, a vesivirus.

**EXAMPLES**

The invention is further described in the following examples, which do not limit the scope of the invention described in the claims.

**Example 1. Beneficial Properties of an Exemplary Culturing Method**

Provided herein are methods of culturing mammalian cells in a culture medium that achieve higher densities of viable mammalian cells and provide an improved volume productivity rate. In the cell culture process runs described below, recombinant galactosidase cell culture was used to determine the reactor angles and perfusion rates that provide significantly improved high-cell-density mammalian cell culture methods. The data provided herein show that perfusion culture performed by placing the culture tubes at a reactor angle of around 45° from horizontal and agitating the cultures at a frequency around 160 RPM provides an unexpected advantage (e.g., improved volumetric productivity, higher viable cell density, and more efficient cell metabolism) over a similar culturing method (e.g., the same culture medium) performed by placing the culture tubes at an reactor angle of 90° from horizontal (described hereafter in the cell culture process runs as 90°) and agitating the cultures at a frequency around 220 RPM.

The data described below also demonstrate that culturing methods that include placing the culture tubes at a reactor angle around 45° from horizontal and agitating the cultures at around 160 RPM with a gradient perfusion rate can be used to effectively screen four recombinant galactosidase candidate clonal cell lines (A14.13, C02.31, C02.57, and F05.43) in three production media (CD CHO, CD OptiCHO, and CD FortiCHO) to determine the best cell line and medium based on cell growth, recombinant protein productivity, and recombinant protein (product) quality.

Finally the data described below show that the exemplary culturing methods provided herein are closely comparable to the performance of recombinant galactosidase clonal cell lines cultured in 12-L perfusion bioreactors in terms of cell growth, productivity, and product quality. In sum, the data show that the presently provided culturing conditions can used to screen clones and cell lines, develop and screen media formulations, test the effects of process parameters on cell culture performance, and to maintain satellite cultures for process optimization and manufacturing support. The general methods used to perform these cell culture process runs are described below.

***Materials and Equipment***

*Recombinant Galactosidase Suspension Cell Culture*

The cell used in each cell culture process run is listed in Table 1. The research cell banks (RCBs) were created to ensure that all RCB vials, regardless of the origin, have the approximate same population doubling level (PDL), as calculated according to Equation 1 (below).

$$PDL = \frac{\ln(\frac{Xv_n}{Xv_{n-1}})}{\ln(2)} \qquad \text{Equation 1}$$

$Xv_1$: Viable cell density ($10^6$ cells/mL) measured at time $T_{n-1}$
$Xv_2$: Viable cell density ($10^6$ cells/mL) measured at time $T_n$

*Equipment*

The following equipment was used to perform the cell culture process runs described herein:

a Multitron Shaker incubator (Appropriate Technical Resources, Inc.), model number AJ125;
a Beckman Coulter Vi-Cell Cell Viability Analyzer (Beckman Coulter, Inc.), model XR;
a Beckman Coulter Allegra centrifuge, model number X-22R;
a YSI Biochemistry Analyzer (Yellow Springs Instruments, Inc.), model number 2700 Select;
an Osmometer (Advanced Instruments, Inc.); model number 2020; and
a Blood gas analyzer (Bayer AG); model number 248.

*Culture Maintenance*

Unless otherwise specified, the start-up cultures for the shake tubes were generated from seed cultures expanded in shake flasks following a vial thaw of a specific RCB (Table 2). One vial from each RCB was used per seed train. The shake tubes were inoculated at either $5 \times 10^5$ or $1.5 \times 10^6$ viable cells/mL, with a constant working volume of 10 mL per tube. The cells were maintained in a shaker incubator in a controlled environment of 5% $CO_2$, 37 °C, and 80% relative humidity, at about a reactor angle of 45° from horizontal or at about a reactor angle of 90° from horizontal, and shaking speeds of 160 and 220 RPM, respectively, or as determined in the given cell culture process run.

**Table 2. Recombinant galactosidase research cell banks used in the studies**

| Cell Culture Process Run | Recombinant Galactosidase Cell Bank | Clone ID | Medium (Lot No.) | Origin | Population Doubling Time | No. of Passages |
|---|---|---|---|---|---|---|
| I | RCBp7v10 10/14/11 AV | C02.31 | CD CHO (N/A) | RCB17307-4 08/04/11 | 9 | N/A |
| III, IV | RCBp7v4 10/14/11 AV | C02.31 | CD CHO (092311 M) | RCB17307-4 08/04/11 (CD CHO) | 13 | 12 |
| III, IV | RCBp7v5 10/14/11 AV | C02.31 | CD OptiCHO (09261 1M2) | | 13 | 12 |
| III, IV | RCBp7v8 10/14/11 AV | C02.31 | CD FortiCHO (082511M) | | 11 | 12 |
| III, IV | RCBp9v5 10/18/11 AV | A14.13 | CD CHO (09231 1M) | RCB17307-4 08/04/11 (CD CHO; PDL = 13) | 13 | 9 |
| III, IV | RCBp9v3 10/18/11 AV | A14.13 | CD OptiCHO (09261 1M2) | | 13 | 9 |
| III, IV | RCBp9v10 10/18/11 AV | A14.13 | CD FortiCHO (082511M) | | 13 | 16 |
| III, IV | RCBv8 11/04/11 AV | C02.57 | CDCHO (092311M) | RCB18401-150 06/29/11 (CD CHO; PDL = 8) | 8 | 8 |
| III, IV | RCBv1 11/04/11 AV | C02.57 | OptiCHO (092611 M2) | | 9 | 7 |
| III, IV | RCBv11 11/04/11 AV | C02.57 | FortiCHO (082511M) | | 8 | 7 |
| III, IV | RCBp7v2 11/10/11 AV | F05.43 | CDCHO (092311M) | RCB18401:9 2 04/28/11 (CD CHO; PDL = 4) | 8 | 7 |
| III, IV | RCBp7v2 11/10/11 AV | F05.43 | OptiCHO (092611M2) | | 5 | 7 |
| III, IV | RCBp7v2 11/10/11 AV | F05.43 | FortiCHO (082511M) | | 5 | 7 |

$$\omega_2 = \omega_1 \sqrt{\frac{r_1}{r_2}}$$   Equation 2

$\omega_1$:   Shaking speed of 180 RPM
$r_1$:   Shaking diameter (throw or orbit diameter) of 50 mm
$\omega_2$:   Shaking speed (RPM) at 25mm shaking diameter

$r_2$:    Shaking diameter of 25 mm

$$Glc_{cons} = ([Glc]_m - [Glc]_c) * PR \qquad \text{Equation 3}$$

$$Gln_{cons} = ([Gln]_m - [Gln]_c) * PR \qquad \text{Equation 4}$$

$$VPR = Titer * PR \qquad \text{Equation 5}$$

$$SPR = \frac{VPR}{Xv * 10^3} * 10^3 \qquad \text{Equation 6}$$

$[Glc]_m$:    Glucose concentration in media (g/L)
$[G/c]_c$:    Glucose concentration in culture (g/L)
$[Gln]_m$:    Glutamine concentration in media (mmol/L)
$[Gln]_c$:    Glutamine concentration in culture (mmol/L)
PR:    Perfusion rate
VPR:    Volumetric productivity (U/L/d)
Titer:    rhα-Gal activity (U/L)
SPR:    Specific productivity rate (U/E9 cells/d)
Xv:    Viable cell count (1 x $10^6$ cells/mL)

The manufacturer recommended settings (see, e.g., the Sartorius website) for agitating the tubes at around 90° from horizontal were adapted to the used shaker incubator according to Equation 2 (shown above). Starting on day one after the inoculation, the cultures were sampled daily (0.5 mL) to determine the viable cell density by ViCELL method. Specifically, the cells were diluted with phosphate buffered saline (PBS) to a density of 1-2 x $10^6$ cells/mL. The cultures were sampled while mixing on a vortexer set to 1.5 vortexing speed for increased homogeneity. Following the cell count, the tubes were centrifuged at approximately 233 x g for 5 minutes, and the removed spent media were used immediately to assay glucose and glutamine consumption rates as well as cell metabolites (lactate and glutamate) using the YSI Biochemistry Analyzer, or stored at -80 °C until a rhα-Gal activity assay was performed to determine product titer. The culture medium was exchanged at ratios described in Table 3, and the glucose and glutamine consumption rates, as well as the rhα-Gal volumetric and specific productivities were calculated using Equations 3-6 (shown above).

Table 3 below shows the gradient perfusion batch refeed schedule followed for Cell Culture Process Runs I, III, and IV. The seeding density at Day 0 (cell culture seeding day called Day 0) was 5 x $10^5$ cells/mL. RV stands for reactor volume of culture medium.

**Table 3.** Gradient Perfusion Batch Refeed Schedule.

| Cell Culture Process Run | Day of culture after seeding | Refeed rate |
| --- | --- | --- |
| I, III, and IV | Day 1-3 | 0.5 RV/day |
|  | Day 4-6 | 0.7 RV/day |
|  | Day 7 onwards | 1.0 RV/day[a] |
| [a]Past day 7 (Cell Culture Process Run I and II), a refeed rate of 0.8 RV/d was performed by the weekend coverage shift (for simple operation and consistence by different operators during weekend). | | |

*Clonal Cell Line and Media Ranking*

Galactosidase is a glycoside hydrolase enzyme that hydrolyzes the terminal alpha-galactosyl moieties from glycolipids and glycoproteins. For example, alpha-galactoside can hydrolyze the ceramide trihexoside and can convert melibiose into galactose and glucose. Secreted alpha-galactosidase is normally post-translationally modified with mannose-6-phosphate moieties, and has the ability to bind to mannose-6-phosphate receptor (e.g., soluble cation-independent mannose-6-phosphate receptor).

During Cell Culture Process Run II, the cultures were sampled three times per week for cell density, recombinant human alpha-galactosidase ($rh\alpha$-Gal) activity, and mass determination, and for metabolite measurements. In addition, samples from each culture were collected four times during the study for the high performance liquid chromatography-based Fast ABC assay, and five times for the Biacore assay, to estimate the amount of $rh\alpha$-Gal clipping occurring in the cultures, and the percentage of $rh\alpha$-Gal binding to the soluble cation-independent mannose-6-phosphate receptor (sCIM6PR), respectively. For each culture-sampling time point, the specific lactate production rate was calculated using Equation 7 (provided below), in addition to the glucose and glutamine consumption rates, and the $rh\alpha$-Gal volumetric and specific productivities (Equations 3-6). The cumulative viable cell density was calculated for every culture at the end of its run according to Equations 8A and 8B (provided below), and the culture doubling time ($T_d$) during the seed training stage was calculated by Equation 9 (provided below), respectively. For media ranking, culture pH and osmolarity were measured at the end of the study from spent culture removed on day 4 of each culture, at its respective peak density, and at the end of its run, and kept at -80 °C.

$$Lac = \frac{(\frac{[lac]_n}{Fw_{lac}} * PR)}{Xv_n * 10^3}$$
\hfill Equation 7

$$IVC_0 = vol * Xv_0$$
\hfill Equation 8A

$$IVC_n = INC_{n-1} + vol * (\frac{(Xv_n + Xv_{n-1})}{2} * (T_n - T_{n-1})) * 10^6$$
\hfill Equation 8B

$$T_d = (T_n - T_{n-1}) * \frac{\ln(2)}{\ln(\frac{Xv_n}{Xv_{n-1}})}$$
\hfill Equation 9

| | |
|---|---|
| *Lac:* | Specific lactate productivity rate (mM/x$10^9$ cells/d) |
| $[lac]_n$: | Lactate concentration measure at time $T_n$ (g/L) |
| $Fw_{lac}$: | Lactate molecular weight (g/mol) |
| *PR:* | Perfusion rate |
| $T_d$: | Doubling time (h) |
| $(T_n - T_{n-1})$: | Time difference (h) between two cell counts on different days |
| $Xv_n$: | Viable cell density ($10^6$ cells/mL) measured at time $T_n$ |
| $Xv_{n-1}$: | Viable cell density ($10^6$ cells/mL) measured at time $T_{n-1}$ |

The four recombinant galactosidase clonal cell lines were ranked based on their growth and metabolite profiles, viability, productivity, and product quality obtained in each of the three production media. A separate ranking was conducted for the media alone, where all clonal cell lines cultured in one medium were regarded as one entity for which the medium performance was evaluated. The list of ranking factors is presented in Table 4.

Each of the ranking factors was assigned a weight which decreased proportionally to the importance of the factor in supporting favorable cell culture performance. Depending on their performance against a given factor ($\pm$ standard deviation), each clonal cell line was allocated from 1 to 4 points (1 to 3 for media), with 4 (or 3) indicating the best, and 1 the worst performance. The weight of the factors was then used to calculate the score of each clonal cell line or media (Equation 10).

For the end result, the arithmetic mean of the individual rankings of each clonal cell line in each medium, or each medium alone, was calculated. The clonal cell lines and media with the highest mean were ranked as 1, and those with the lowest were ranked as 4 or 3, respectively.

$$Score = weight * points \qquad \text{Equation 10}$$

Score: The score of a clonal cell line or a medium
weight: The weight of a given factor
points: The number of gained points

**Table 4.** List of ranking factors.

| FACTOR | Description | WEIGHT |
|---|---|---|
| Biacore binding activity | Mean % binding measured on D9, 12, 15, 18, and 24 (where available), and compared to the specifications[a] | 10 |
| rh$\alpha$-Gal clipping | Mean % of Peak B (recombinant galactosidase intact form) measured by Fast ABC on D7, 12, 18, and 28 (where available), and compared to the specifications[b] | 9 |
| Specific activity | Mean slope of the rh$\alpha$-Gal activity plotted as a function of rh$\alpha$-Gal mass, and compared to the specifications[c] | 8 |
| VPR | End number accumulated productivity as measured by rh$\alpha$-Gal activity | 7 |
| Xv | End number IVC | 6 |
| Peak Xv | Peak viable cell density attained | 5 |
| Longevity of the culture | Survived or not until the end of the run (*) Number of cultures supported until the end of the run (+) | 4 |
| Viability | Mean % viability throughout the run | 3 |
| Seed train performance (*) | Mean doubling time (Td) | 2 |
| SPR | Mean specific productivity throughout the run | 1 (2-media) |
| Lac production | Mean specific lactate productivity throughout the run | 0.5 (1-media) |
| Cell clumping (+) | The extent of cell aggregation as determined from ViCell camera images | 0.75 |
| pH (+) | At the end of the run: how much it diverged from the specifications[d] | 0.5 |
| Osmolarity (+) | At the end of the run: how much it diverged from the specifications[e] | 0.25 |

(*) Applies only to the clonal cell line ranking; (+) applies only to the media ranking.
"Acceptable Biocore binding activity range for recombinant galactosidase: 68-125%; 2000 L manufacturing mean and one standard deviation range: 96% $\pm$ 7.8.
[b]2000 L manufacturing mean and two standard deviation range for Peak B: 69.4% (64.1-77.4).
[c]Acceptable range for recombinant galactosidase specific activity: 58-83 U/mg; 2000 L manufacturing mean and two standard deviation range: 73 U/mg (65-81).
[d]pH specifications for Invitrogen media used in the study: 6.9-7.4 (CD CHO), 6.9-7.3 (CD OptiCHO), 6.6-7.3 (CD FortiCHO).
[e]Osmolarity specifications for Invitrogen media used in the study: 305-345 (CD CHO), 260-290 (CD OptiCHO), 275-295 (CD FortiCHO) mOsm/kg.

### Cell Culture Process Run I

The purpose of this study was to determine which shaking speed and reactor angle allows for improved cell culture performance at peak density, and beyond. In the current cell culture process run the media refeed regime was confined to one reactor volume per day (1 RV/day). The goal of these cell culture process runs was to develop a cell culturing method that would result in a cell concenration of 40 x $10^6$ cells/mL over a long period. The start-up culture used for this study was generated from the shake flask seed culture expanded for three days after vial thaw (RCBp7v10, 10/14/11 AV).

*Growth Profiles*

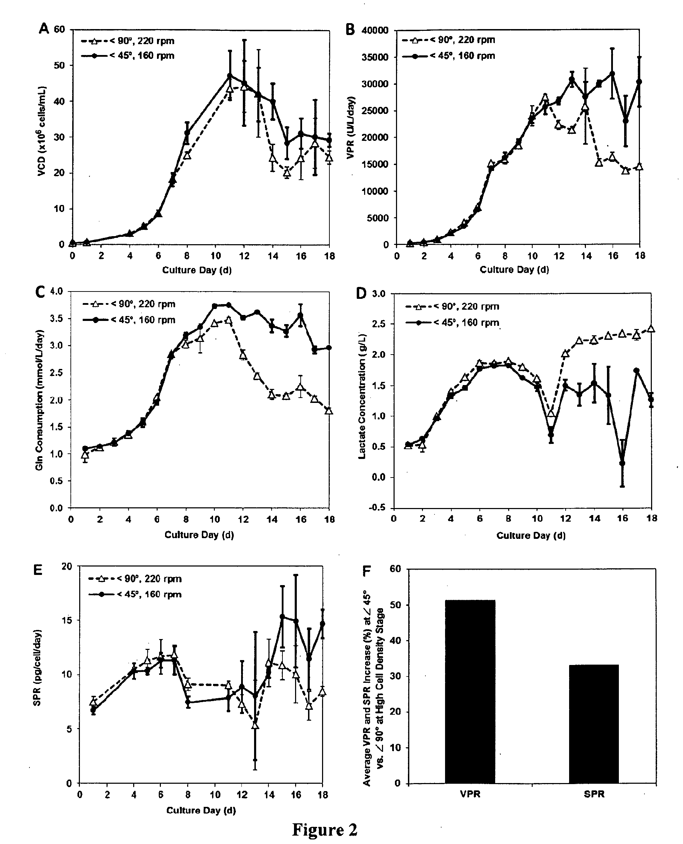

The viable cell density profiles of 18-day long cultures of C02.31 maintained in CD CHO under different shaking parameters were observed. A similar viable cell density was measured in cultures agitated at a reactor angle of about 90° from horizontal or a reactor angle of about 45° from horizontal, although a slightly higher viable cell count was obtained for the cultures agitated at a reactor angle of about 45° from horizontal during the last 5 days of the post-peak density stage as compared to the cultures agitated at a reactor angle of about 0° from horizontal. Cultures agitated at both reactor angles maintained a similar viability of 85-90% throughout the study (Figure 2A).

*Metabolite Profiles*

Glucose consumption profiles were similar between the two culture conditions, but the cultures agitated at a reactor angle of about 90° from horizontal (90°) consumed less glucose (about 5 g/L/day) during the last 5 days of the study as compared to the cultures agitated at a reactor angle of about 45° from horizontal (6 g/L/day). The lactate production profiles of both culture conditions were similar until day 11, when lower lactate concentration was detected in the cultures agitated at a reactor angle of about 45° from horizontal (Figure 2D). No significant difference was observed in glutamine consumption rate between the two culture conditions until day 11 of the study, when glutamine consumption in the cultures agitated at a reactor angle of about 90° from horizontal (90°) started gradually decreasing to about 2 mmol/L/day at the end of the run, whereas it was maintained at about 3-3.5 mmol/L/day until day 18 of the study in the cultures agitated at a reactor angle of about 45° from horizontal (Figure 2C). No significant difference throughout the 18 days of culture was observed in glutamate production between the two culture conditions.

*Productivity Profiles*

The volumetric productivity (VPR) profiles of the two culture conditions were identical until day 11, where the productivity measured in both conditions was about 30,000 U/L/d. Starting from day 12, which is peak production stage, the VPR of the cultures agitated at a reactor angle of about 90° from horizontal (90°) was gradually decreasing to about 50% at the end of the run, whereas it was maintained at the 30,000 U/L level in the cultures agitated at a reactor angle of about 45° from horizontal from day 12 until day 18 (Figure 2B). Similarly, the specific productivity of cultures agitated at a reactor angle of about 45° from horizontal started increasing from day 11, whereas it remained constant in the cultures agitated at a reactor angle of about 90° from horizontal (90°) (Figure 2E). The data show that the volume productivity rate of the cultures was increased over 50% and the specific productivity rate of the cultures was increased over 30% by agitating the cultures at a reactor angle of 45° compared to the volume productivity rate and specific productivity rate achieved by agitating the cultures at a reactor angle of 0° (Figure 2F).

**Cell Culture Process Run II**

The cell culture process runs described below were performed to determine the effect of cell culture temperature on recombinant galactosidase clonal cell line C02.31 growth, productivity, and product quality in CD CHO. An increase in specific productivity has been observed in fed-batch and perfusion cultures of recombinant CHO cells subjected to mild hypothermia (28-33°C). However, the magnitude of the temperature effect was found to be both cell line- and recombinant protein-dependent. Therefore, a series of three independent cell culture process runs was performed to evaluate the performance of recombinant galactosidase clonal cell line C03.21 in CD CHO (MTX bank DWCB NB17594) perfusion cultures over a range of temperature set points maintained for two weeks, following a vial thaw and a 7-8-day growth phase at 37 °C. When the cultures reached the cell density of about $20 \times 10^6$/mL, a temperature shift was performed. In each experiment, the culture temperature of 37°C was used as a control. The details of the biphasic process are summarized in Table 5. The effect of cell culture temperature on cell growth, viability, metabolite turnover, productivity, and product quality was analyzed from cell culture harvested material. The study was performed using the shake tube high throughput model, where the cultures incubated at 5% $CO_2$ and 80% relative humidity, and were agitated at 160 RPM and at a reactor angle of about 45° from horizontal.

**Table 5.** Summary of the cell culture process run conditions used for the biphasic cell culture process in shake tubes.

| Inoculation Density (1 x 10⁶ cells/mL) | Growth Temperature (°C) | Temperature after Shift (°C) | Target Density (1 x 10⁶ cells/ml) | Cell Bleeding Density (from --> to) (1 x 10⁶ cells/ml) | n |
|---|---|---|---|---|---|
| 0.5 | 37 | 31 | 20 | > 17 --> 17 | 3 |
| 0.5 | 37 | 33 | 20 | > 17 --> 17 | 3 |
| 0.5 | 37 | 35 | 20 | > 17 --> 17 | 6 |
| 0.5 | 37 | 36 | 20 | > 17 --> 17 | 3 |
| 0.5 | 37 | 37 | 20 | > 17 --> 17 | 9 |
| 0.5 | 37 | 38 | 20 | > 17 --> 17 | 6 |
| 0.5 | 37 | 40 | 20 | > 17 --> 17 | 3 |

A media refeed rate of 0.5 reactor volume per day (RV/d) (1.0x RV represents the amount of liquid culture medium present in each container at the start of the culturing period) was employed for the first three days of the process after inoculation, followed by a refeed (perfusion) rate of 0.7x RV/d for the next three days, and 1.0x RV/d starting from day 7 (temperature shift at approximately $20 \times 10^6$ cells/ml) until the end of the run. Starting from day 7-8, cell bleeding was performed daily on all cultures exceeding the density of $17 \times 10^6$ cell/mL at the time of sampling to extend the high-density culture stage to 14 days while maintaining viability > 90%.

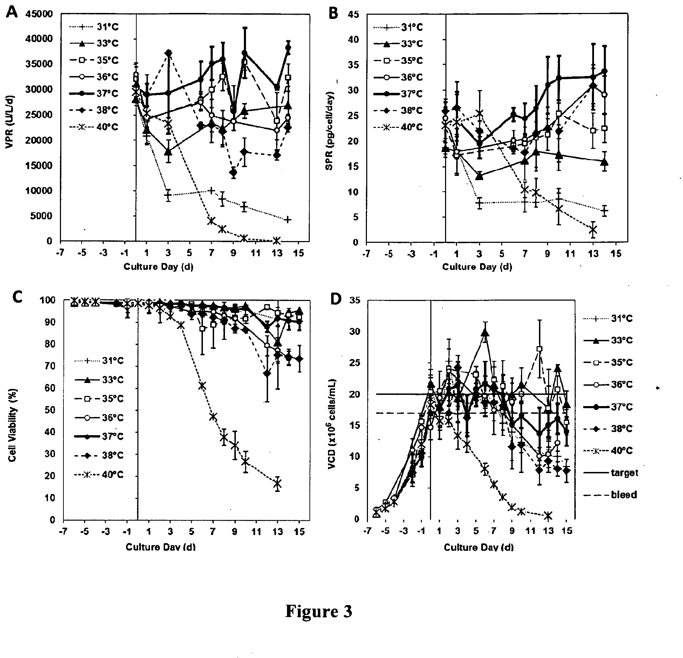

The data from these cell culture process runs show that in general, regular cell bleeding allowed the maintenance of viability at > 90% throughout the run for cultures grown at temperatures $\leq$ 37 °C (Figure 3C). The viability of the cultures maintained at temperatures > 37 °C dropped below 90% on day 4 (40 °C) and day 9 (38 °C, 39 °C), respectively, post temperature shift, then continued decreasing until the end of the run (Figure 3C). At 33 °C and 35 °C, the cells were growing better than in control cultures (37 °C), but at 31°C and at temperatures > 37 °C, the cells were growing more slowly than in control cultures (Figure 3D). Growth was severely impaired at 40 °C leading to a premature termination of the cultures on day 13 (Figure 3D). During the second week post-temperature shift, the cells maintained at temperatures > 37 °C were significantly smaller than the cells in the control cultures, whereas the diameter of cells grown at temperatures < 37 °C was generally reduced by only 2%. The cells cultured at 35 °C had the same size as the cells grown at 37 °C.

In addition, the data indicate that cell metabolism was significantly altered in cultures maintained at 31, 33, and 40 °C as compared to the control (37 °C). The reduced metabolic activity observed at 31 and 40 °C corresponded with a significant reduction in volumetric and specific productivities as compared to the control cultures and cultures maintained at 35 °C. In general, productivity was the highest at both 37 and 35 °C (Figures 3A and 3B). In general, the highest, steady-state, specific activity and M6P binding on a Biacore assay was measured for the product collected from cell cultures maintained at 37 and 35 °C, and a significant drop in product quality was observed for cultures maintained at 31, 39, and 40 °C.

Overall, cell growth and viability were reduced at culture temperature of >37 °C, whereas culture temperature < 37 °C resulted in increased cell viability and growth. However, at 31°C cell metabolism seemed to be too low to support good growth. Culture temperatures of < 36 and > 37 °C also resulted in a reduced cell diameter. Surprisingly, cell metabolism seemed to be optimal at a 37-39 °C range, resulting in the highest productivity, and the highest sCIMPR binding in a Biacore assay, which peaked at 37 °C. recombinant galactosidase specific activity was comparable at the 33-39 °C range. Culture temperature < 37 °C did not have a negative impact on the integrity of the produced recombinant galactosidase molecule as determined by the HPLC-based Fast ABC assay, whereas an increased amount of the fragmented molecule was measured in cell culture harvests obtained from cultures maintained at temperatures > 37 °C.

The effect of various individual culture parameters ($CO_2$, frequency of agitation, and reactor angle) were statistically compared to determine their individual effect on viable cell concentration, the percent cell viability, and the activity of the cells using partial factorial design of experiment (DOE). The JMP statistical analyses (see JMP website) show that the reactor angle alone has a significant effect on the viable cell concentration, the percent cell viability, and cell productivity (Figure 4).

### Cell Culture Process Run III

The shake tube model established in *Cell Culture Process Run II* was applied to screen four recombinant galactosidase clonal cell lines and three production media for cell growth, productivity, and product quality to select the best clonal cell line and medium for commercial cell culture process development. The capacity of each medium was also evaluated by extending the culture time to one month but without exceeding the media exchange rate of 1 RV/day.

RV stands for the first culture medium volume in the container. In this cell culture process run, 1 RV equals 10 mL. The clonal cell lines subjected to the study are described in Table 6. Clones: C02.31 and C02.57 (C clones) were derived from the same parental cell line as the primary candidates for the recombinant galactosidase process development; and clonal cell lines A14.13 (A clonal cell line) and F05.43 (F clonal cell line) were selected as backup clones. The initial small scale cell growth process runs showed that the C clonal cell lines tended to achieve higher overall productivity and higher M6P binding in the Biacore assay, however, their productivity tended to decrease in prolonged culture. The F clonal cell line performed similarly to the C clonal cell lines, whereas the A clonal cell line tended to have lower, but more stable productivity over time.

**Table 6.** Recombinant galactosidase clonal cell lines screened in shake tubes.

| Specification | Recombinant Galactosidase Clonal Cell Line | | | |
|---|---|---|---|---|
| | A14.13 | C02.31 | C02.57 | F05.43 |
| **Primary Candidate** | | + | | |
| **Productivity** | | + | + | + |
| **Stability** | + | | | |

To allow for a meaningful comparison, all clonal cell lines were first adapted to growth in the three media, following by cell banking at similar population doubling levels (PDL) (Table 2). The research cell banks used in this study were then thawed and expanded in shake flasks for three days before being seeded into 35 shake tubes at $0.5 \times 10^6$ cells/mL. The three media were tested side by side with the same clonal cell lines and under the same conditions in triplicate. According to previous findings, all cultures were agitated at a reactor angle of around 45° from horizontal and 160 RPM, and followed the batch-refeed (Gradient Perfusion) regime described in Table 3.

*Cell Growth Profiles*

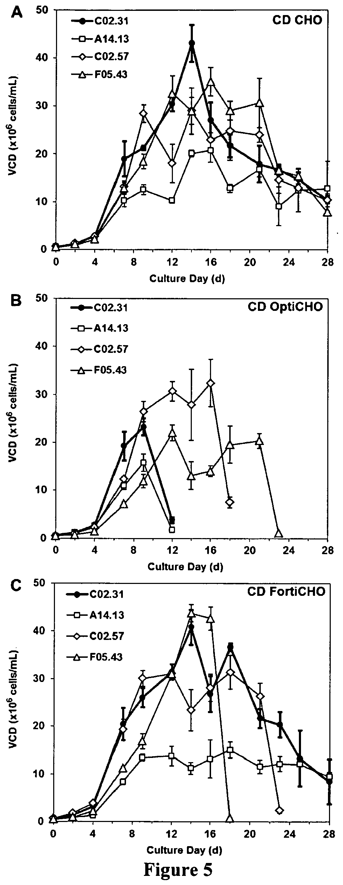

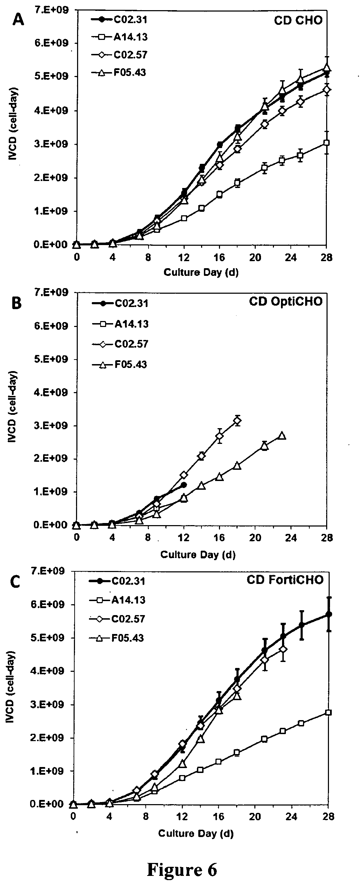

The growth and viability profiles of recombinant galactosidase suspension cultures of four clonal cell lines in three production media are shown in Figure 5. For the other two media and all remaining clones, triplicate shake tubes were inoculated. Cell viability and growth (Figures 5A-5C) were measured over a one-month period. The CD CHO medium supported cell viability > 90% for all four clonal cell lines during 18 days of culture, with the end-of-the-run viability of about 60%. See Figure 5A. Cell viability in CD OptiCHO dropped drastically from about 100% on day 9 to approximately 10% on day 12 for clones: C02.31 and A14.13, and for clonal cell lines C02.57 and F05.43 to approximately 20% and 10% on day 18 and 23, respectively. See Figure 5B. The CD FortiCHO medium could support viability > 90% for only 16 days, and only for clonal cell line F05.43; for all other clonal cell lines the cell viability dropped to about 70% on day 14 of the study, and was close to 50% and 70% for clones: C02.31 and A14.13, respectively, at the end of the run. See Figure 5C. The cultures of C02.57 in the CD FortiCHO culture medium were terminated on day 23 due to cell viability close to 20%. The highest viable cell density (approximately $45 \times 10^6$ cells/mL) was reached on day 14 in both CD CHO (clonal cell line C02.31) and CD FortiCHO (clonal cell line F05.43), respectively. However, clonal cell line F05.43 did not survive until the end of the run in the CD FortiCHO medium (Figure 5C). The final cell density for all surviving clonal cell lines was about $10 \times 10^6$ cells/mL on day 28 across all media (Figures 5A-5C). In all media, clonal cell line A14.13 reached a similar maximal cell density of about $10 \times 10^6$ cells/mL (Figures 5A-5C). The maximal cell density recorded in CD OptiCHO was approximately $30 \times 10^6$ cells/mL (clonal cell line C02.57, day 9), but all cultures were terminated prematurely due to low viable cell count (Figure 5B). The total viable cell mass was also assessed (see Figure 6). The data in Figure 6 show that CD OptiCHO medium resulted in the lowest total viable cell mass for all four tested clonal cell lines, CD CHO medium resulted in a good total viable cell mass for all four tested clonal cell lines, while CD Forti CHO medium resulted in the highest total viable cell mass for the C02.31 clonal cell line.

*Metabolite Profiles*

Before the cultures started deteriorating, the glucose consumption profiles in all three media were very similar between the four clones, however, the lactate accumulation profiles were only similar for the two C clones, whereas the A and the F clonal cell lines followed a different, but also a matching pattern. Interestingly, in the absence of glucose at the high cell density stage, the cells started consuming lactate if the next media refeed was performed later than 22-24 h following the previous one. The measured lactate production in CD FortiCHO was always below 0.5 g/L/day, whereas in CD CHO and CD OptiCHO the highest registered lactate production was 1.5 and 2.5 g/L/day, respectively. About the same maximal amount of glutamine (about 3.5 mM/day) was consumed in all three media. As for glucose consumption, the glutamine consumption trends were similar for the four clonal cell lines in all media, but in CD CHO, both C clonal cell lines had a distinctive glutamine consumption rates starting from day 14 of the culture. The glutamate production profiles were again very similar across the clonal cell lines and media, however, on average 2.5 mM/day of glutamate was produced in CD CHO medium, 1.7 mM/day in CD OptiCHO, and 3.5 mM/day in CD FortiCHO, respectively.

*Productivity Profiles*

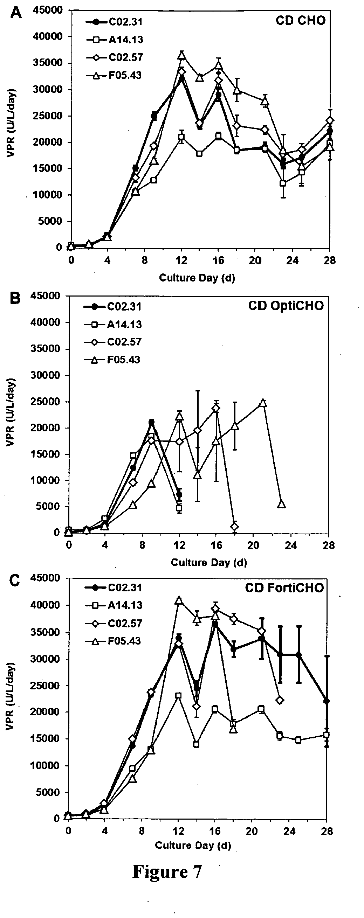

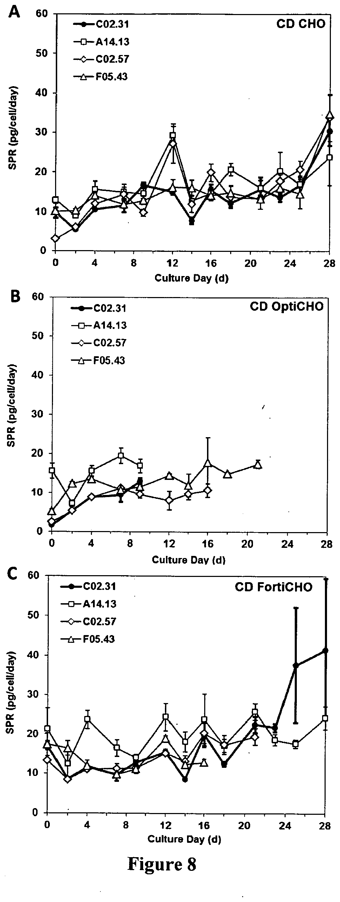

In CD CHO, the A14.13 clonal cell line had most stable productivity of approximately $20 \times 10^3$ U/L/day (day 12-28), while the two C clonal cell lines and the F05.43 clonal cell line reached about $30-35 \times 10^3$ U/L/day on day 12, but productivity then decreased to about $20-25 \times 10^3$ U/L/day on day 28 (Figure 7A). The overall productivity in CD OptiCHO was the lowest ($< 25 \times 10^3$ U/L/day for all four clones), and could not be maintained until the end of the study (Figure 7B). The highest overall productivity close to $40 \times 10^3$ U/L/day was measured in CD FortiCHO (clones: C02.57 and F05.43), but it could not be maintained for more than 4 days (Figure 7C). In CD FortiCHO, clonal cell line A14.13 had stable productivity of about $20 \times 10^3$ U/L/day, and clonal cell line C02.31 showed a relatively stable productivity at a level of about $30 \times 10^3$ U/L/day (day 12-25) (Figure 7C). All four clonal cell lines had a similar specific productivity of about 1000-1500 U/$10^9$ cells/day in all three media (Figures 8A-C).

*Quality of the Recombinant Product*

The quality of the recombinant protein produced by each clonal cell line in each medium was evaluated by assessing the enzyme's specific activity, binding to the sCIMPR relative to the control (purified recombinant galactosidase), and integrity measured by Fast ABC assay. Overall, the specific activity and sCIMPR binding were the best in CD CHO culture medium, however, the percentage of M6P binding relative to the control was in the same specification range for both the CD CHO culture medium and CD FortiCHO culture medium. The specific activity was closely comparable between clonal cell lines C02.31 and F05.43 in both the CD CHO culture medium and the CD FortiCHO culture medium, whereas the sCIMPR binding was found better for the C02.31 clonal cell line than the F05.43 clonal cell line in all three media. The sCIMPR binding in the CD CHO culture medium was also the most consistent for all of the clonal cell lines throughout the culture, whereas it was the poorest in the CD OptiCHO culture medium, similar to the specific activity and Fast ABC results. The lowest amount of clipping was observed in the CD FortiCHO medium for all four clones. In general, a decrease in specific activity and sCIMPR binding, as well as an increase in the amount of the clipped form of the recombinant protein has been observed with decreasing cell viability.

*pH, Osmolarity, and Cell Aggregation in the Media*

The changes in the pH and osmolarity of the culture media were assessed on the media removed at three time points during each culture. In the CD CHO culture medium, the pH stayed within the specified range for the first two weeks of the cultures of all clones. Once the cultures reached their peak densities, the pH started decreasing to as low as 6.6 at the end of the run for clonal cell F05.43. It remained relatively stable for clonal cell A14.13 cultures that did not grow to densities higher than $20 \times 10^6$ cells/mL. In all CD OptiCHO cultures, the pH was relatively unstable, dropping as low as about 6.4 for the C02.31 and A14.13 cultures terminated on day 12, and as high as approximately 7.6 for the other two cultures, that were terminated on day 18 (C02.57) and 23 (F05.43), respectively, pointing to a poor pH control in this medium. The pH of all cultures in CD FortiCHO remained within the specified range, however, two of the four cultures had to be terminated prematurely. Past day 14, osmolarity of the CD CHO cultures started decreasing to about 250 mOsm/kg at the end of the run, whereas it dropped below 200 mOsm/kg in the A14.13 cultures on day 12. It was approximately 250 mOsm/kg on day 23 of the F05.43 clonal cell cultures that were sustained in this medium the longest. Osmolarity in CD FotiCHO remained close to 250mOsm/kg throughout the culture for all four clones.

Cell culture images of each clonal cell line in each of the three media were taken by the Vi-Cell camera for the assessment of cell aggregation. A comparable amount of cell aggregation was observed in CD CHO and CD FortiCHO media, whereas no cell clumping was recorded in CD OptiCHO throughout the culture. Cell aggregation increased with the age of the cultures.

*Ranking of Recombinant Galactosidase Clonal Cell Lines and Production Media*

The performance of the four recombinant galactosidase clonal cell lines in three commercially available media was evaluated against a list of ranking factors for an unbiased end result, allowing to choose the best recombinant galactosidase clone/media combination for the most robust process development. The clonal cell lines and media were ranked separately. Clonal cell line ranking is presented in Table 7, and media ranking in Table 8. According to the ranking, clonal cell line C02.31 was found the best performing, and clonal cell line C02.57-the second best. Clonal cell line C02.57 performed on average 10% less well than C02.31 (Table 7). CD CHO was held to be the most robust medium, and CD OptiCHO held to be the least robust medium (Table 8).

**Table 7.** Recombinant galactosidase clonal cell line ranking in three production media.

|  | C02.31 | A14.13 | C02.57 | F05.43 |
|---|---|---|---|---|
| CD CHO | 178<br>**1** | 139<br>**4** | 165<br>**2** | 154<br>**3** |
| CD OptiCHO | 138<br>**2** | 126<br>**4** | 144<br>**1** | 130<br>**3** |
| CD FortiCHO | 192<br>**1** | 138<br>**3** | 153<br>**2** | 108<br>**4** |
| RANK (Mean) | 1.33 | 3.67 | 1.67 | 3.33 |
| **Final RANK** | **1** | **4** | **2** | **3** |

**Table 8.** Recombinant galactosidase production media ranking.

|  | **CD CHO** | **CD OptiCHO** | **CD FortiCHO** |
|---|---|---|---|
| Total Score | 150 | 76 | 136 |
| **Final RANK** | **1** | **3** | **2** |

***Cell Growth Process Run IV: Comparison of Highthroughput (HT) Model to 12 L Bioreactor***

Cell growth, productivity, and product quality obtained for clonal cell line C02.31 suspension cultures maintained in shake tubes at a reactor angle of about 45° from horizontal and at an agitation of 160 RPM were compared to the performance of the same clonal cell line cultured in a 12-L perfusion bioreactor. The main differences between the two culturing systems are summarized in Table 9. Because of nutrient limitations observed in the shake tube cultures due to the lower media exchange rate than in the 12 L reactors, the shake tube cultures were taken under consideration only until day 18, when cell viability was still > 90%, to compare cultures of the same state for the model validation.

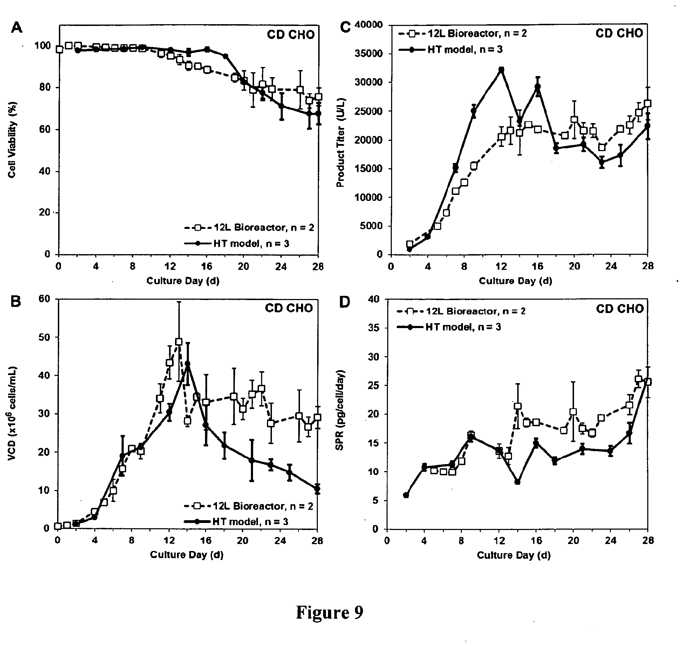

The viability profiles of the clonal cell line C02.31 cultures in CD CHO in both systems were closely comparable, as were the growth profiles (Figures 9A and 9B). No major difference was observed between the two systems in glucose consumption and lactate concentration profiles in the medium, however, due to the twice higher media

**Table 9.** Culturing systems compared in the study.

|  | **HT Model** | **12 L Bioreactors** |
|---|---|---|
| Environmental Control | Without online controllers | Controlled environment |
| Mixing Method | Orbital shaking | Stirred tank |
| Media Volume | 0.01 L | 12 L |
| Media Refeed Method | Batch refeed | Perfusion |
| Media Refeed Volume | 1 RV/d | 2 RV/d |

exchange rate (perfusion rate) per day, cells grown in the 12-L reactors consumed twice as much glucose and almost as much more glutamine as the cells maintained in the shake tubes. In contrast, a slightly higher concentration of glutamate was measured in the spent media obtained from the shake tube cultures as compared to the ones in the bioreactors. Yet the specific production rate was found comparable between the two systems (Figure 9D). In both the shake tube cultures and the reactor cultures, the same specific activity of the product was obtained, and a similar M6P binding profile for the product was found. Overall, the clonal cell line's performance in the shake tubes in all media was closely comparable to that observed in the 12-L bioreactors, with the best product quality measured in the CD CHO culture medium, and the worst in the CD OptiCHO culture medium, higher lactate production in the CD CHO culture medium as compared to the CD FortiCHO culture medium, highest VPR in the CD CHO culture medium, lowest in the CD OptiCHO culture medium, SPR similar in all three media, and highest degree of cell aggregation in the CD CHO culture medium.

In sum, the data provided herein show that culturing methods provided herein are closely comparable to and are able to model the performance of 12-L perfusion bioreactor culturing methods in terms of cell growth, productivity, and product quality. Based on these findings, the currently provided perfusion culturing methods can be used to develop and screen media formulations, test the effects of various culturing parameters on cell culture performance, and to maintain satellite cultures for process optimization and manufacturing support.

**Example 2. Satellite Shake Tube Cultures from a 12-L Bioreactor Culture**

Experiments were performed to test whether satellite shake tube cultures from a 12-L recombinant human alpha-galactosidase bioreactor culture would replicate the growth and productivity of a 12-L recombinant human alpha-galactosidase bioreactor culture.

*Materials and Methods*

Satellite shake tube cultures were inoculated using a sample from a 12-L recombinant human alpha-galactosidase bioreactor culture obtained on day 3 of the culture (e.g., during the growth phase of the bioreactor cell culture process run). The final concentration in each shake tube culture after seeding was 11-15 x $10^6$ cells/mL. The volume of liquid culture medium in each satellite shake tube culture (after seeding) was 10 mL. The volume of the shake tube used to contain each satellite culture was 50 mL. Two triplicate sets of satellite shake tube cultures were obtained from two 12-L bioreactor cultures. The satellite shake tube cultures were maintained through the end of the bioreactor cell culture process runs. The satellite shake tube cultures were grown at 37 °C, 80% relative humidity, and 5% $CO_2$, and were agitated at a frequency of 160 RPM at a reactor angle of 45 degrees from horizontal. Cell-bleeding methods were implemented in the satellite shake tube cell culture process runs in order to maintain constant cell density. The cell-bleeding methods used in the satellite shake tube cell culture process runs were similar to the cell-bleeding methods used in the 12-L bioreactor cell culture process runs. Specifically, cell-bleeding methods were implemented once the satellite shake tube cultures reached a density of 40-45 x $10^6$ cells/mL, with a volume of about 10%-15% of the liquid culture medium (containing cells) periodically removed from each satellite shake tube and replaced with approximately an equal volume of fresh liquid culture medium.

A medium exchange of two-tube volumes of medium was performed daily for each shake tube (as described in Example 1) (1X volume of medium (substantially free of cells) was removed and replaced with fresh culture medium twice during each 24-hour period, typically once in the early morning and once in the later afternoon). Sampling for cell growth, culture metabolism, productivity, and product quality in the satellite shake tube cell culture process runs was performed on the same schedule as the 12-L bioreactor cell culture process runs. Each cell culture process run was performed using a C02.31 clone cells maintained in CD CHO medium.

*Results*

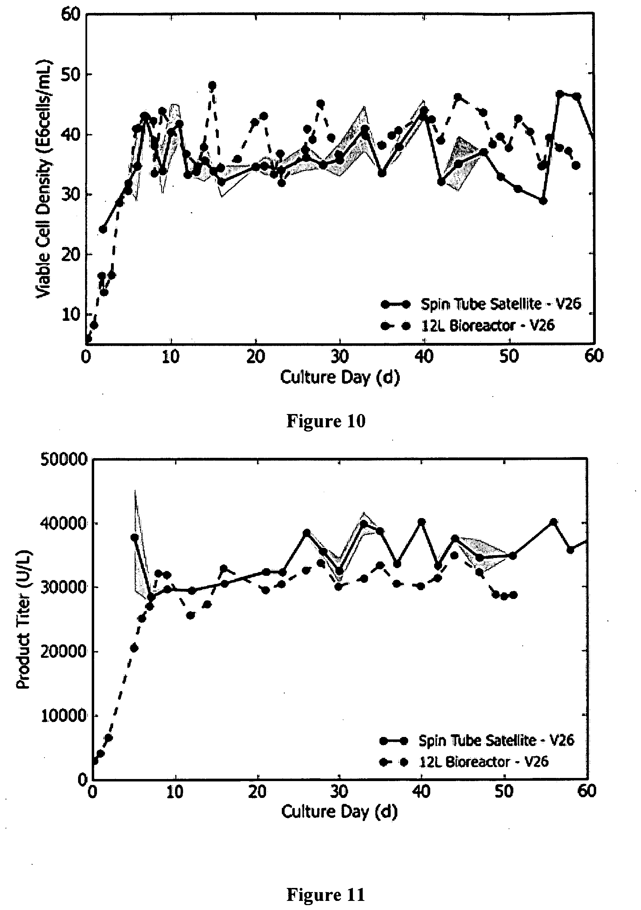

The data show that the satellite shake tube cell culture process runs and the 12-L bioreactor cell culture process runs have similar viable cell density profiles (Figure 10). Over the majority of the culture period, the satellite spin tube cell culture process runs maintained a viable cell density between 35 x $10^6$ cells/mL and 45 x $10^6$ cells/mL using the employed cell-bleeding methods (Figure 10). The productivity (titer) profile for the satellite shake tube cell culture process runs and the 12-L bioreactor cell culture process runs showed a similar trend throughout the entire culture period (Figure 11). These data show that a recombinant human alpha-galactosidase satellite shake tube cell culture process run can replicate the growth and productivity of a 12-L recombinant human alpha-galactosidase bioreactor cell culture process run.

**Example 3. 600-mL Shake Tube Cultures**

Experiments were performed to test the growth properties and volumetric productivity of cell cultures grown in 600-mL shake tubes (as referred to as "maxi tubes") using the methods described herein. The growth properties and volumetric productivity of these 600-mL cell cultures were compared to the 50-mL shake tube cultures grown using the methods described herein.

*Materials and Methods*

In each experiment, the 50-mL shake tubes with a vented cap or the 600-mL maxi tubes (Techno Plastic Products (TPP) AG, Trasadingen, Switzerland) were inoculated with a starting cell concentration of 5 x $10^5$ cells/mL

using cells from seed cultures expanded in shake flasks for 14 and 3 passages following the thaw of a vial of C02.31 cells in Cell Culture Process Runs I and II (described below), respectively. Different cell banks of the same cell line (recombinant cell line C02.31 expressing and secreting human recombinant alpha-galactosidase) were thawed for Cell Culture Process Runs I and II. The cell cultures were maintained in a controlled environment of 5% $CO_2$, 37 °C, and 80% relative humidity in a shaking incubator. A control 50-mL shake tube condition established earlier was run with each experiment at a constant working volume of 10 mL per shake tube. Both these control 50-mL shake tubes and 300 mL per maxi tube (600 mL size) were cultured at a reactor angle of 45° (from horizontal) and a shaking speed of 160 RPM.

Cell Culture Process Run I was performed to compare the cell growth in terminal batch cultures in both shake tubes and maxi tubes. Starting on day one after the initial inoculation, the cultures were sampled daily (0.5 mL) to determine the viable cell density (VCD) using a Beckman Coulter Vi-Cell Cell Viability Analyzer (Beckman Coulter, Inc.) for 11 days.

Cell Culture Process Run II was performed using the batch refeed process. Beginning on day one after inoculation, the cultures were sampled every Monday, Wednesday, and Friday (0.5 mL) to determine the VCD by ViCell and productivity was determined for 15 days. Following the cell count, the shake tubes were centrifuged at approximately 223 x g for 5 minutes, and the maxi tubes were centrifuged at approximately 300 x g for 8 minutes. The centrifugation time was determined for the maxi tubes through setting a proportion to the shake tubes using Equation 11. The spent media was removed after centrifugation, and stored at -80 °C until a rh$\alpha$-Gal activity assay was performed to determine the product titer. The culture medium was exchanged at ratios described in Tables 9 and 10, and the rh$\alpha$-Gal volumetric and specific productivities were calculated using Equations 12 and 13.

**Table 10. Batch Refeed Schedule for Shake Tubes.** Seeding Density at Day 0: 5 x $10^5$ cells/mL. RV/d: Reactor Volume per day.

| Day of Culture after Seeding | Refeed Rate |
|---|---|
| Days 1 - 3 | 0.5 RV/d |
| Days 4 - 6 | 0.7 RV/d |
| Day 7 onwards | 1.0 RV/d |

**Table 11. Batch Refeed Schedule for Maxi Tubes.** Seeding Density at Day 0: 5 x $10^5$ cells/mL. RV/d: Reactor Volume per day.

| Day of Culture after Seeding | Refeed Rate |
|---|---|
| Days 1 - 3 | 0.5 RV/d |
| Days 4 - 6 | 0.7 RV/d |
| Day 7 onwards | 0.8 RV/d |

$$ln\ (Ro/R) = \omega^2 t \qquad \textbf{Equation 11}$$

*Ro*:    Distance from center of the centrifuge to bottom of vessel
*R*:    Distance from center of the centrifuge to the top of the liquid
$\omega$:    Angular velocity
*t*:    Time

$$VPR = Titer * PR \qquad \textbf{Equation 12}$$

$$SPR = \frac{VPR}{Xv} \qquad \textbf{Equation 13}$$

*PR*:    Perfusion rate

*VPR*:    Volumetric productivity (U/L/d)
*Titer*:    rh $\alpha$ -Gal activity (U/L)
*SPR*:    Specific productivity rate (U/$10^9$ cells/d)
*Xv*:    Viable cell count ($10^6$ cells/mL)

### Results: Cell Culture Process Run I

On day 34 after vial thaw, a cell line producing recombinant human alpha-galactosidase was used to inoculate shake tubes under two different conditions (Table 12). Batch culture performance of these cultures was evaluated over 11 days.

**Table 12. Cell Culture Process Run I Conditions Tested**

| Vessel type | Total Volume | Working Volume |
|---|---|---|
| Shake Tube | 50 mL | 10 mL |
| Maxi Tube | 600 mL | 300 mL |

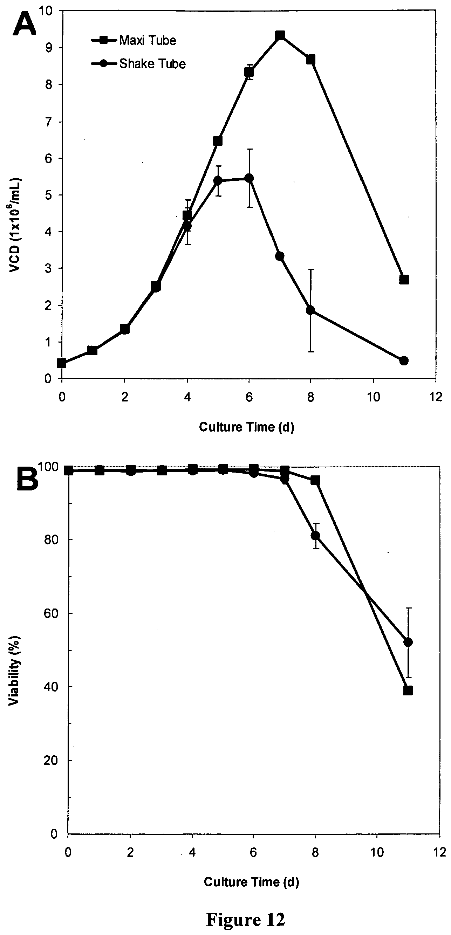

The viable cell density profiles of the cultures maintained in CD CHO for 11days are shown in Figure 12A. Similar growth profiles were exhibited until day four. After day four, the shake tubes began to enter the stationary growth phase, while the maxi tube cultures continued exponential growth. The cultures grown in the shake tubes reached a peak viable cell density of 4.5 x $10^6$ cells/mL on days five and six. The cultures grown in maxi tubes reached a peak viable cell density of 9.0 x $10^6$ at day seven. Both cultures maintained similar viability profiles (Figure 12B), with viabilities greater than 90% observed in both cultures until entering the decline phase. These data show that the maxi tubes are capable of supporting better cell growth at high cell density stages.

### Results: Cell Culture Process Run II

Based on the data in Cell Culture Process Run I above, a perfusion-like process was applied to the maxi tube cultures to see if they would have similar growth properties to a perfusion-like process performed using the 50-mL shake tube cultures described in the earlier Examples. On day 7 after vial thaw, a cell line expressing human recombinant $\alpha$-galactosidase was used to inoculate duplicate maxi tubes and shake tubes in this cell culture process run. The cell culture performance (both cell growth and productivity) was evaluated in a 15-day batch refeed process.

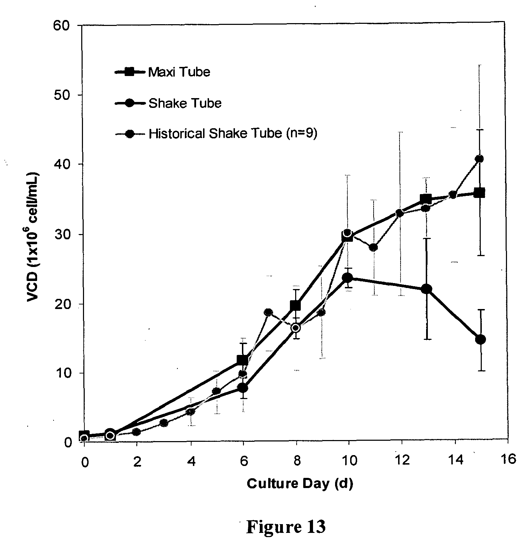

The viable cell density profiles of the 15-day cultures are shown in Figure 13. Similar growth profiles were observed for the two culture vessels up to day 10, when the cell growth in the experimental control (shake tube) growth plateaued at a peak viable cell density of 25 x $10^6$ cells/mL. The culture in the maxi tube began to plateau at day 13, reaching a peak viable cell density of 35 x $10^6$ cells/mL. However, when using the average viable cell density of historical data (n = 9) of cultures in the 50-mL shake tube model, the growth pattern aligns identically with the maxi tube growth pattern.

Due to the limitations of the centrifuge, the maxi tubes were not able to achieve an angle of 0° (horizontal) during centrifugation. As a result, centrifugation of the maxi tubes resulted in the formation of a loose pellet. The loose pellet caused a change in the refeed parameters for the maxi tube cultures. Namely, the refeed schedule was modified for removal of 0.8 RV per day instead of 1.0 RV per day for day 7 onwards. In addition, the spent media may not be aseptically tipped out of the maxi tube cultures, and had to be removed by pipette to prevent cell loss.

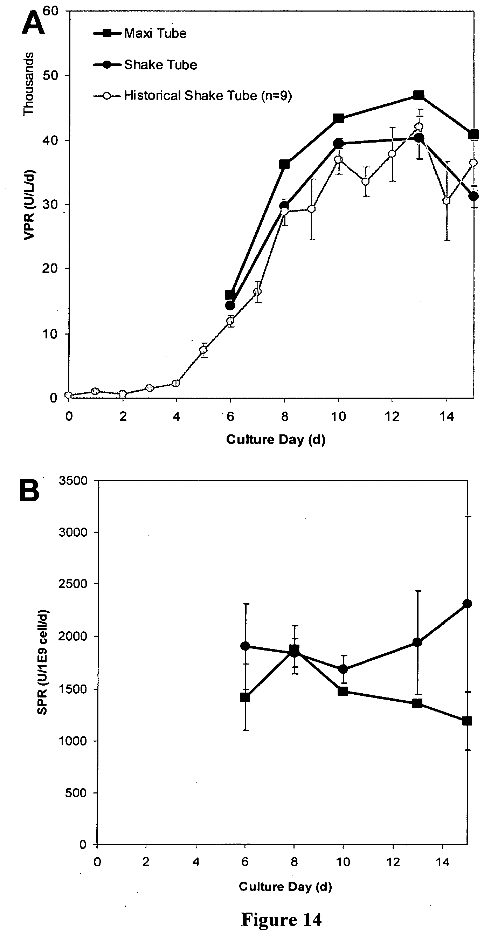

The volumetric productivity rate (VPR) was measured beginning at day 6 (Figure 14A). The cultures had similar productivity for both the maxi tubes and the shake tubes, while the maxi tubes had slightly higher productivity. The differences in the productivity were insignificant between the two vessels, with the greatest difference of approximately 25% at day 15. Both cultures reached peak productivity at day 13 with -15% difference in VPR. The maxi tube cultures had a peak VPR of 47,000 U/L/day and the shake tube cultures had a peak VPR of 40,000 U/L/day. The experimental control (shake tube culture) closely corresponded with the historical data collected (n = 9). In contrast, the cultures grown in the maxi tubes had a lower specific productivity rate (SPR) than the cultures grown in shake tubes (Figure 4B). The maxi tube cultures maintained a SPR of ~1400 U/$10^9$ cells/day as compared to the SPR of cultures grown in shake tubes of ~1900 U/$10^9$ cells/day, through day 13. After day 13, the SPR of the shake tube cultures and the maxi tube cultures exhibited a significant difference in SPR. The shake tube cultures had approximately double the SPR of the maxi tubes (2300 U/$10^9$ cells/day vs. 1200 U/$10^9$ cells/day, respectively) due to poor growth related to lower cell density in the shake tube cultures.

In sum, these data show that high cell densities can be achieved in a batch culture using a maxi tube (600-mL volume), a reactor angle of 45° (from horizontal), and an agitation frequency of 160 RPM. Using similar set of gradient media refeed rates and culturing conditions, a culture in a maxi tube had comparable growth and productivity as compared to control cultures grown in 50-mL shake tubes. In the batch refeed cultures, both the maxi tube and shake tube cultures had similar productivity (VPR greater than 40,000 U/L/day), though the maxi tube cultures had significantly higher cell density than the shake tube cultures. After day 10, the shake tube cultures had a higher specific productivity rate than the maxi tube cultures due to poor cell growth in the shake tube cultures. The application of the increased shake tube size to the batch refeed model of perfusion-based cell culture processes makes it feasible to obtain purified product samples for drug substance analysis and provides for a direct comparison with the product obtained from a larger bioreactor.

## Example 4. Multi-Parameter Study of Shake Tube Model

A set of experiments was performed to determine the cell growth and productivity achieved in the 50-mL shake tube model under a wide variety of culture conditions and parameters. In these multi-parameter experiments, the parameter ranges tested were: reactor angles of between 5° and 85°, rotational speeds between 20 RPM and 330 RPM, and working volumes of 2 mL to 40 mL.

### *Materials and Methods*

As discussed in detail below, Cell Culture Process Runs I and II were designed to test the extremes of each variable (in triplicate), and Cell Culture Process Run III was designed with a narrower range from the previously determined control 50-mL shake tube cell culture process runs (tested in triplicate).

Cell Culture Process Run III was designed using JMP software's custom design tool. The model was designed using two responses (peak viable cell density and peak volumetric productivity rate), and three continuous variables (shaking speed, reactor angle, and working volume) taking into account second order interactions. The lower limit and upper limit were set according to Table 13. The design was customized to have a power of 2 to account for any non-linear relations between variables. Lastly, the study was designed to have a total of 18 conditions run in duplicate.

**Table 13. Continuous Variable Settings used in JMP**

| Variable | Lower Limit | Upper Limit |
|---|---|---|
| Shaking Speed (RPM) | 120 | 255 |
| Reactor Angle (°) | 30 | 90 |
| Working Volume (mL) | 2 | 30 |

All shake tubes were inoculated at a concentration of $5 \times 10^5$ cells/mL using clonal C02.31 cells from seed cultures expanded in shake flasks for three passages for Cell Culture Process Run I and eight passages for Cell Culture Process Runs II and III following vial thaw. The cell cultures were maintained in a controlled environment of 5% $CO_2$, 37 °C, and 80% relative humidity in a shaking incubator. A control condition established earlier was run with each experiment with a constant working volume of 10 mL per tube, a reactor angle of 45°, and a shaking speed of 160 RPM.

For Cell Culture Process Runs I and III, starting on day one after the inoculation, the cultures were sampled (0.5 mL) to determine the viable cell density using a Beckman Coulter Vi-Cell Cell Viability Analyzer (Beckman Coulter, Inc.). For Cell Culture Process Run II, the cultures were sampled daily (20 $\mu$L) to determine the viable cell density by manual count using the Trypan Blue Exclusion method. Manual count was performed for Cell Culture Process Run II to minimize the cell bleed of low culture volumes. Following the cell count, the tubes were centrifuged as approximately 233 x g for 5 minutes, and the removed spent media were stored immediately at -80 °C until a rh$\alpha$-Gal activity assay was performed to determine product titer. The culture medium was exchanged at ratios described in Table 14, and the rh$\alpha$-Gal volumetric and specific productivities were calculated using Equations 12 and 13 (above). Additionally, the integrated viable cell densities (IVCD) and integrated volumetric productivity rates (IVPR) were calculated using Equations 14 and 15.

**Table 14. Batch Refeed Schedule for Shake Tubes.** Seeding Density at Day 0: $5 \times 10^5$ cells/mL. RV/d: Reactor Volume per day.

| Day of Culture after Seeding | Refeed Rate |
|---|---|
| Days 1 - 3 | 0.5 RV/d |
| Days 4 - 6 | 0.7 RV/d |

(continued)

| Day of Culture after Seeding | Refeed Rate |
|---|---|
| Day 7 onwards | 1.0 RV/d |

$$IVCD_n = IVCD_{n-1} + \left(\left(\frac{Xv_n + Xv_{n-1}}{2}\right) * (t_n - t_{n-1})\right) \qquad \text{Equation 14}$$

$$IVPR_n = IVPR_{n-1} + \left(\left(\frac{VPR_n + VPR_{n-1}}{2}\right) * (t_n - t_{n-1})\right) \qquad \text{Equation 15}$$

*PR*: Perfusion rate

*VPR:* Volumetric productivity rate (U/L/d)

*Titer:* rh $\alpha$ -Gal activity (U/L)

*SPR:* Specific productivity rate (U/$10^9$ cells/d)

*Xv*: Viable cell density ($10^6$ cells/mL)

*IVCD:* Integrated viable cell density (cells-d/mL)

t: Time (d)

*IVPR:* Integrated volumetric productivity rate (U/L)

Manual cell counting was performed using a hemocytometer chamber and cover-slips cleaned with isopropyl alcohol (IPA). The corner of the cover-slips were wetted with IPA and affixed to the hemocytometer. The cell samples were homogenously mixed with 1:1 trypan blue. A 10-$\mu$L aliquot of the mixture was transferred to a hemocytometer chamber. The cells were counted in the four large outer squares; each large outer square contained a grid of 16 smaller squares. Cells lying on the boundaries of the larger square were counted only on two of the four sides. Uncolored cells were counted as viable; those stained with blue were considered dead. The percent viability and viable cell density were calculated using Equations 16 and 17, respectively.

$$\text{Viability} = \left(\frac{\text{Viable Cells}}{\text{Total Cells}}\right) \cdot 100\% \qquad \text{Equation 5}$$

$$\text{Viable Cell Density} = \left(\frac{\text{Viable Cells}}{\text{Squares Counted}}\right) \cdot \text{Dilution Factor} \cdot 10^4 \qquad \text{Equation 6}$$

Total Cells: Sum of Viable Cells and Dead Cells

The statistical analyses in these experiments were performed using JMP software. The responses used were peak viable cell density (VCD or Xv) and peak volumetric productivity rate (VPR), with maximized desirability. The statistical model was assessed through the "Fit Model" function with an effect screening report. The "Sorted Parameter Estimates" reported the factors that significantly effected response variables, which is statistically determined through a t-test. The "Interaction Profiler" reported the trend of effects caused by interaction (or lack thereof) on the response variables. Lastly, the "Prediction Profiler" plots the independent trends of the effects of the parameters on response variables and uses the model to predict the best conditions through maximizing the desirability function.

### Results: Cell Culture Process Run I

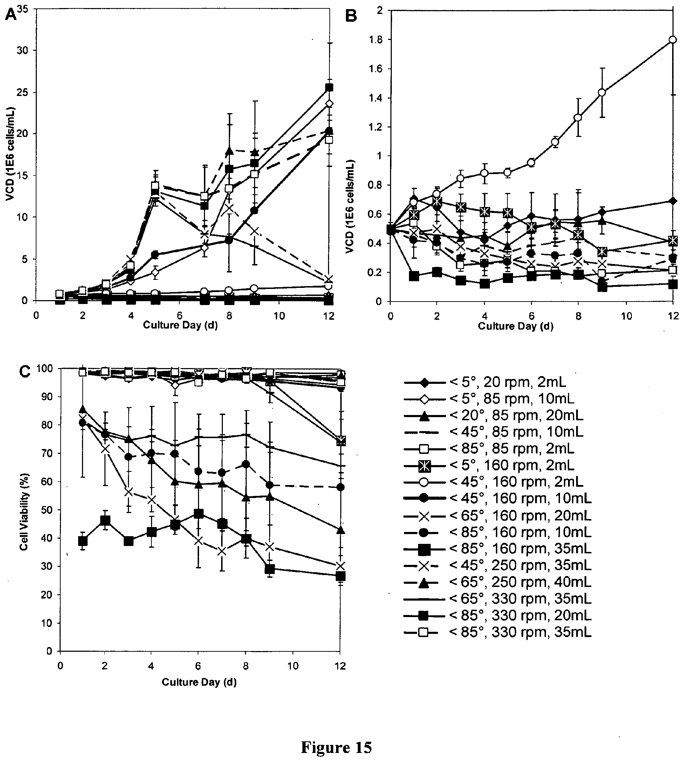

On day 9 after vial thaw, a cell line expressing and secreting recombinant human $\alpha$-galactosidase (clonal cell line C02.31) was used to inoculate shake tubes under 16 different conditions (Table 15). The cell culture performance was evaluated by determining cell growth and productivity over a 12-day batch refeed process. Of the 16 conditions tested, conditions #3, #4, #5, #9, #10, and #11 failed to support growth as measured by a 25% decrease in viable cell density (Xv) (Figure 15A). Of the six failed conditions, five experienced a drop in viability of 30% or more (Figure 15C).

**Table 15. Cell Culture Process Run I conditions tested.**

| Condition | Shaking Speed | Reactor Angle | Working Volume |
|---|---|---|---|
| 1 | 20 RPM | 5° | 2 mL |
| 2 | 85 RPM | 5° | 10 mL |
| 3 | 85 RPM | 20° | 20 mL |
| 4 | 85 RPM | 45° | 10 mL |
| 5 | 85 RPM | 85° | 2 mL |
| 6 | 160 RPM | 5° | 2 mL |
| 7 | 160 RPM | 45° | 2 mL |
| 8 | 160 RPM | 45° | 10 mL |
| 9 | 160 RPM | 65° | 20 mL |
| 10 | 160 RPM | 85° | 10 mL |
| 11 | 160 RPM | 85° | 35 mL |
| 12 | 250 RPM | 45° | 35 mL |
| 13 | 250 RPM | 65° | 40 mL |
| 14 | 330 RPM | 65° | 35 mL |
| 15 | 330 RPM | 85° | 20 mL |
| 16 | 330 RPM | 85° | 35 mL |

The viable cell density profiles of the 12-day cultures of cells expressing recombinant human $\alpha$-galactosidase maintained in CD CHO under different parameters are shown in Figure 15A. Similar growth profiles were exhibited for seven conditions (conditions #2, #8, #12, #13, #14, #15, and #16) up to day 4. After day four, the growth patterns began to diverge with two conditions (conditions #12 and #14) reaching peak density at day 5. The remaining five of the seven conditions identified as well-performing reached similar viable cell densities of 20 x 10$^6$ cells/mL at day 12, with conditions #2 and #15 reaching as high as 25 x 10$^6$ cells/mL. Condition #7 exhibited a slower growth rate reaching approximately 2 x 10$^6$ cells/mL at day 12 (Figure 15B). Conditions #1 and #6 were also growing more slowly and resulted in only a 1.4-fold increase of the starting viable cell density. All of the conditions that supported cell growth maintained a percentage of viable cells between 90% to 100% throughout the study (Figure 15C).

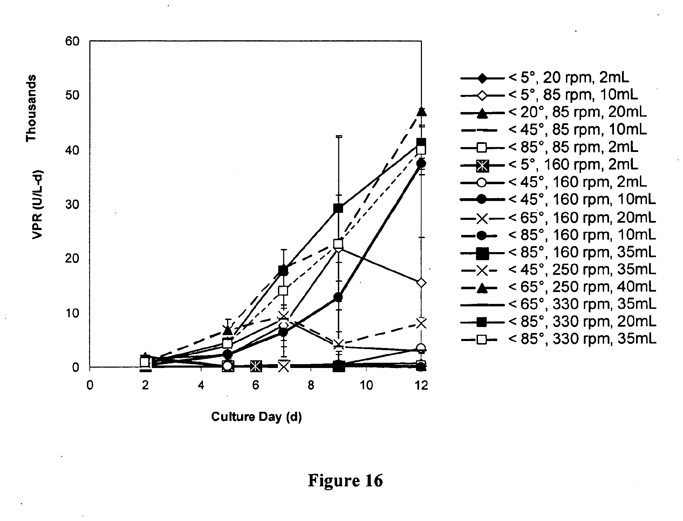

The volumetric productivity rate observed for culture conditions that failed to support cell growth was often too low for detection. The VPR profiles of the growing cultures were similar until day 5, where the productivity measured was about 3000 U/L/day (Figure 16). Starting from day 8, the VPR of the cultures varied significantly. Cultures that experienced similar growth patterns did not have similar productivity. Of the five best conditions (conditions #2, #8, #13, #15, and #16), condition #16 had the lowest peak density but the highest activity of 40,000 U/L/day alone with condition #15.

The growth and productivity profiles were integrated for better comparison of conditions. The profiles of the various conditions differed in growth/productivity rates and reached peak values at different days. The cumulative profiles thus allowed analysis of the total growth and productivity over the entire 12-day process.

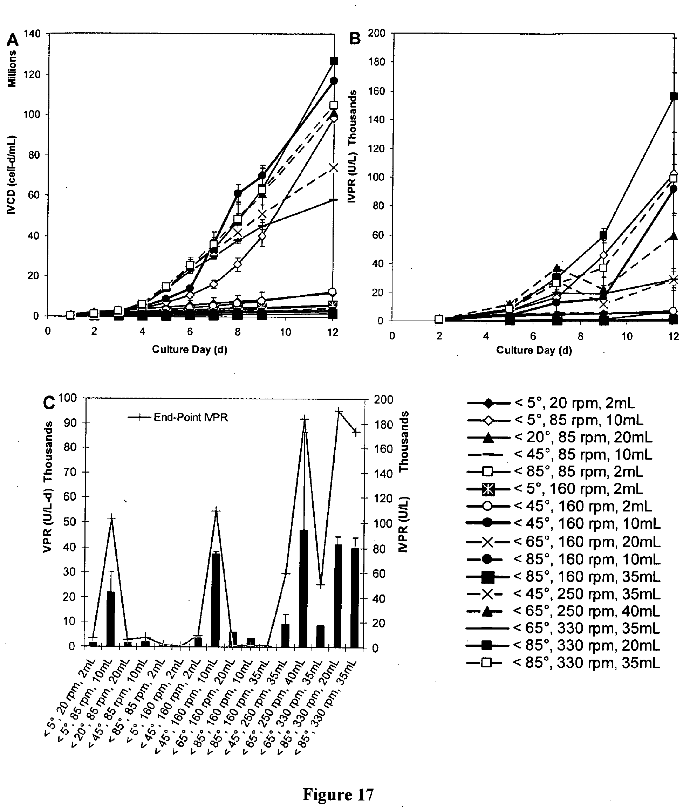

The integrated viable cell density (IVCD) profiles of the 12-days cultures of the cells expressing recombinant

human $\alpha$-galactosidase maintained in CD CHO under different parameters are shown in Figure 17A. The end-point analysis of IVCD resulted in the same seven conditions being identified as having significant growth compared to the remaining conditions. However, the conditions experiencing the most growth over the 12-day process changed. Conditions #12 and #14 with the highest peak cell densities would not be ideal operating conditions because the cell cultures peak early and then slowly die off. In contrast, conditions #8 and #15 had the highest ICVD, meaning the viable cell density over the 12-day process was cumulatively higher suggesting better operating conditions.

The integrated volumetric productivity rate (IVPR) profiles of the 12-day cultures are shown in Figure 17B. The IVPR profiles followed a similar trend to the VPR results, however, condition #15 exhibits almost double the total productivity for the 12 days compared to those conditions with a similar VPR at day 12 (Figure 17C).

### Results: Cell Culture Process Run II

On day 32 after vial thaw, C02.31 cells expressing and secreting recombinant human $\alpha$-galactosidase were used to inoculate shake tubes under 6 different conditions (Table 16). Cell culture performance, as measured by cell growth and productivity, was evaluated in a 14-day batch refeed process. Of the 6 conditions tested, condition #5 failed to support growth due to mechanical reasons, as opposed to the poor conditions. The combination of low reactor angle (5°) and high rotational speed (330 RPM) in condition #5 failed to keep the corresponding tubes in the rack.

**Table 16. Cell Culture Process Run II Conditions tested.**

| Condition | Shaking Speed | Reactor Angle | Working Volume |
|---|---|---|---|
| 1 | 20 RPM | 65° | 2 mL |
| 2 | 125 RPM | 85° | 2 mL |
| 3 | 160 RPM | 45° | 10 mL |
| 4 | 260 RPM | 5° | 2 mL |
| 5 | 330 RPM | 5° | 2 mL |
| 6 | 330 RPM | 20° | 20 mL |

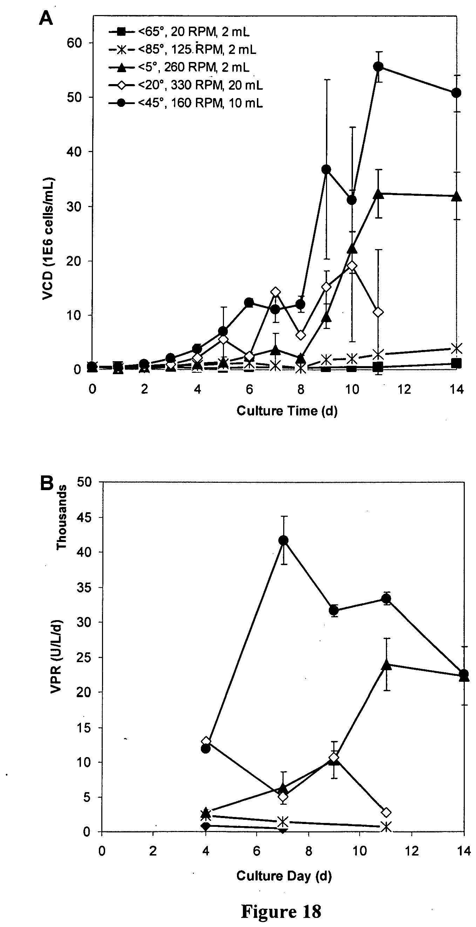

The viable cell density profiles of the 14-day cultures of cells expressing and secreting recombinant human $\alpha$-galactosidase under different parameters are shown in Figure 18A. Five of the tested conditions (#1, #2, #3, #4, and #6) were considered well-performing. Conditions #1 and #2 exhibited slow growth, reaching peak viable cell densities by day 14 of $1 \times 10^6$ cells/mL and $4 \times 10^6$ cells/mL, respectively. The other three conditions (conditions #3, #4, and #6) reached peak viable cell densities of approximately $20 \times 10^6$ cells/mL or above, with the control (condition #3) reaching a viable cell density of $55 \times 10^6$ cells/mL. Condition #6 experienced contamination resulting in a large drop in viable cell density at day 11 (followed by termination of this culture).

The volumetric productivity rate of culture conditions that maintained a viable cell density below $3 \times 10^6$ cells/mL were not analyzed due to concentrations being below the detection threshold (condition #1). The VPR profiles of the well-performing culture conditions followed their growth profiles (Figure 18B). Just as condition #2 exhibited slow growth, it also exhibited a minimal VPR, reaching only 700 U/L/day. Conditions #3, #4, and #6 had a peak VPR of 42,000 U/L/day, 24,000 U/L/day, and 10,000 U/L/day, respectively. These peak VPR were reached at day 7 for conditions #3 and #6, and at day 11 for condition #4.

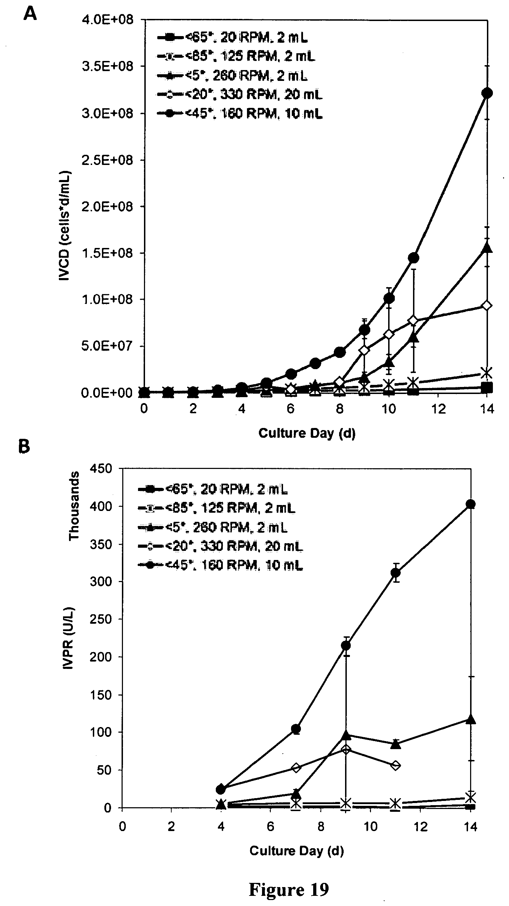

The integrated viable cell density (IVCD) profiles of the 14-day cultures of cells expressing and secreting recombinant human $\alpha$-galactosidase under different parameters are shown in Figure 19A. End-point analysis of IVCD resulted in the same observations as the growth profile analysis, with the control condition (condition #3) yielding the best results, followed by conditions #4 and #6.

The IVPR profiles of the 14-days cultures are shown in Figure 19B. The integrated volumetric productivity rate profiles exhibited a similar trend as compared to the VPR profiles and the growth and IVCD profiles. Condition #3 had the best overall productivity reaching just over 300,000 U/L for the 14-day process and was followed by conditions #4 and #6 with approximately half the productivity (150,000 U/L and 100,000 U/L, respectively).

### Results: Cell Culture Process Run III

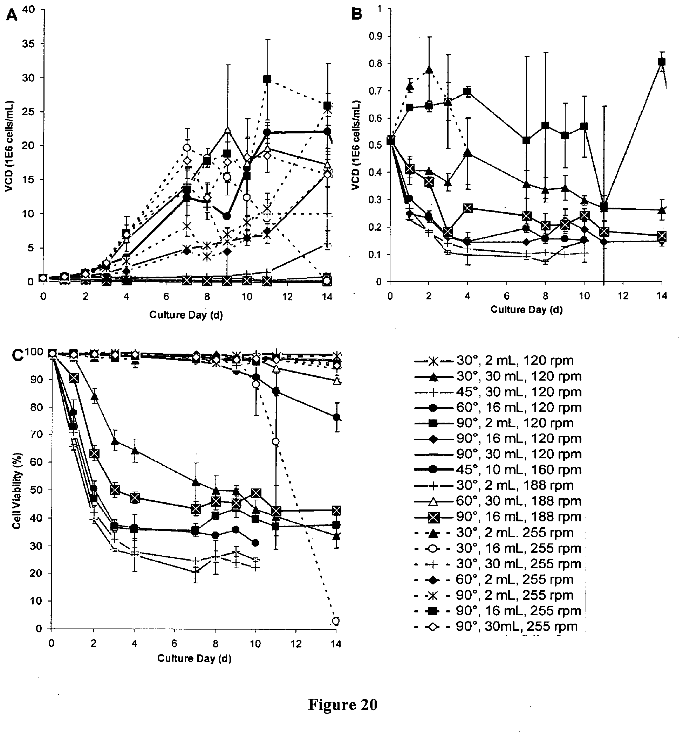

On day 21 after thawing of cells that express and secrete recombinant human $\alpha$-galactosidase, the cells in CD CHO medium were used to inoculate shake tubes grown under 18 different conditions (Table 17). The conditions tested were generated using JMP software as described in this Example. Cell culture performance was determined by measuring growth and productivity of the cultures over the 14-day batch refeed process. Of the 18 conditions tested, conditions #2, #3, #4, #6, #7, and #11 failed to support growth as measured by a 50% (or greater) decrease in viable cell density (Xv) (Figures 20A and 20B). All six of these conditions also experienced a drop in the percent cell viability of 50% or more (Figure 20C).

**Table 17. Cell Culture Process Run III Conditions Tested.**

| Condition | Shaking Speed | Reactor Angle | Working Volume |
|---|---|---|---|
| 1 | 120 RPM | 30° | 2 mL |
| 2 | 120 RPM | 30° | 30 mL |
| 3 | 120 RPM | 45° | 30 mL |
| 4 | 120 RPM | 60° | 16 mL |
| 5 | 120 RPM | 90° | 2 mL |
| 6 | 120 RPM | 90° | 16 mL |
| 7 | 120 RPM | 90° | 30 mL |
| 8 | 160 RPM | 45° | 10 mL |
| 9 | 188 RPM | 30° | 2 mL |
| 10 | 188 RPM | 60° | 30 mL |
| 11 | 188 RPM | 90° | 16 mL |
| 12 | 255 RPM | 30° | 2 mL |
| 13 | 255 RPM | 30° | 16 mL |
| 14 | 255 RPM | 30° | 30 mL |
| 15 | 255 RPM | 60° | 2 mL |
| 16 | 255 RPM | 90° | 2 mL |
| 17 | 255 RPM | 90° | 16 mL |
| 18 | 255 RPM | 90° | 30 mL |

The viable cell density profiles of the 14-day cultures of cells expressing and secreting recombinant human $\alpha$-galactosidase maintained in CD CHO under different parameters are shown in Figure 20A. Starting from day 3, the growth patterns of different conditions began to diverge with two conditions (conditions #13 and #14) reaching peak viable cell density on day 7 of 14 x $10^6$ cells/mL and 20 x $10^6$ cells/mL, respectively.

Most of the remaining culture conditions reached a peak viable cell density at day 14, with the exception of conditions #10, #17, and #18 reaching a peak viable cell density at day 9 with a viable cell density of 22 x $10^6$ cells/mL and day 11 with peak viable cell densities of 30 x $10^6$ cells/mL and 19 x $10^6$ cells/mL, respectively (Figure 20A). Conditions #5 and #12 exhibited a slower growth rate resulting in a 1.6-fold increase of viable cell density. All conditions supporting cell growth maintained a similar percent viability of 90% to 100% throughout the process run (Figure 20C).

The volumetric productivity rate of culture conditions that failed to support cell growth or maintained a viable cell density below 3 x $10^6$ cells/mL were not submitted for analysis due to concentrations being below the detection threshold. The VPR profiles of the well-performing culture conditions followed their growth profiles. Three conditions had similar maximum VPR of approximately 30,000 U/L/day (Figures 20A-C). Excluding the three conditions with high VPR, most of the culture conditions had similar VPR peaking at approximately 20,000 U/L/day.

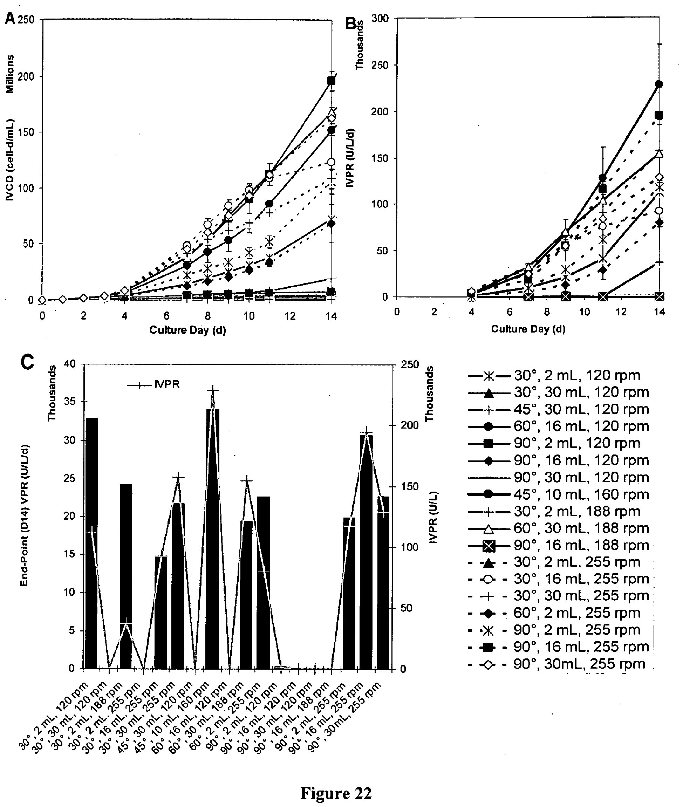

The growth and productivity profiles were integrated for better comparison of conditions. The cumulative profiles allowed analysis of the total growth and productivity over the 14-day process. The integrated viable cell density (IVCD) profiles of the 14-day cultures of cells expressing and secreting recombinant human $\alpha$-galactosidase maintained in CD CHO medium under different parameters are shown in Figure 22A. The end-point analysis of IVCD resulted in similar observations as the growth profile analysis, with the same 10 conditions having significant growth compared to the remaining conditions. Conditions #16 and #17 had the highest peak viable cell densities, however, looking at the entire 14-day process, condition #16 exhibited a lower overall VCD than condition #17. This suggests that condition #17 is better for 14-day processes, whereas condition #16 may be better for longer processes due to its late onset of the exponential growth phase. Conditions #8, #10, and #18 also perform well for overall viable cell density.

The integrated volumetric productivity (IVPR) profiles of the 14-day cultures are shown in Figure 22B. IVPR profiles followed a similar trend to the VPR results. Looking at day 14 for end-point analysis, conditions #1 and #8 exhibit high performance of approximately 30,000 U/L/d, however, condition #1 has low total productivity over the 14 days (Figure 22C). Conditions #8 and #17 have the highest total productivity of approximately 225,000 U/L.

Using the data collected in the JMP experimental design (as described in this Example), the best operating condition was predicted. The operating condition was predicted using two responses: peak viable cell density (VCD) and peak volumetric productivity rate (VPR). All response limits were set to maximize the outcome.

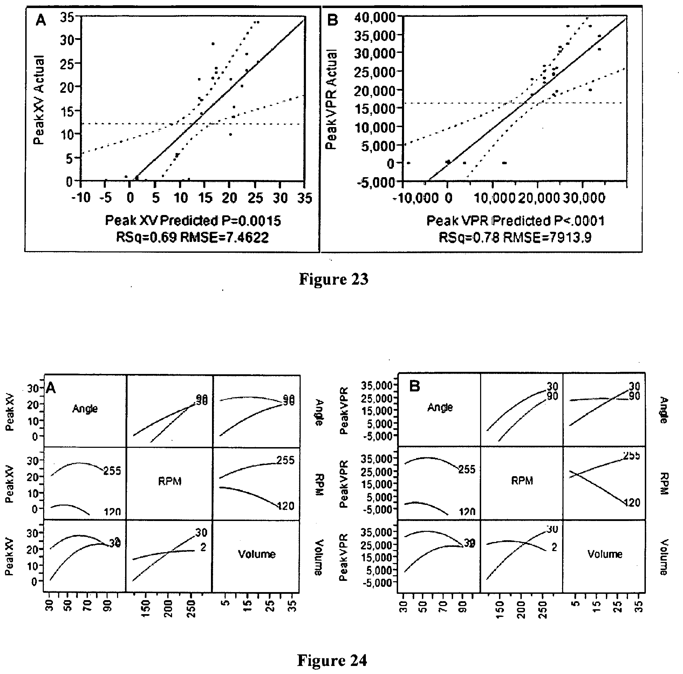

First, the data was fit to the model shown in Figure 23. The data collected for viable cell density (Figure 23A) and volumetric productivity rate (Figure 23B) fit the model well with R-square values of 0.69 and 0.77, respectively. Statistical analysis (t-test) revealed that the angle and the interactions between the angle and shaking speed, and shaking speed and working volume significantly affected the responses.

The parameter interaction profile was used to see trends in the effect of the responses, as well as the interaction between factors (Figures 24A and 24B). Based on the slopes, it was concluded that all conditions have influence on cell growth (Figure 24A). The reactor angle (shaking angle) and the shaking speed have a slight interaction. At all angles increased RPM improves the response (Figure 24A; middle column, top row), with the best results are produced at angles close to the centerpoint (60°) (Figure 24A; left column, middle row). For cultures of all working volumes, higher reactor angles produce better results (Figure 24A; right column, top row). Low volumes produced similar results irrespective of shaking speed, however high volumes are greatly impacted by RPM (Figure 24A; middle column, bottom row). Similar results were observed for the effects on VPR (Figure 24B).

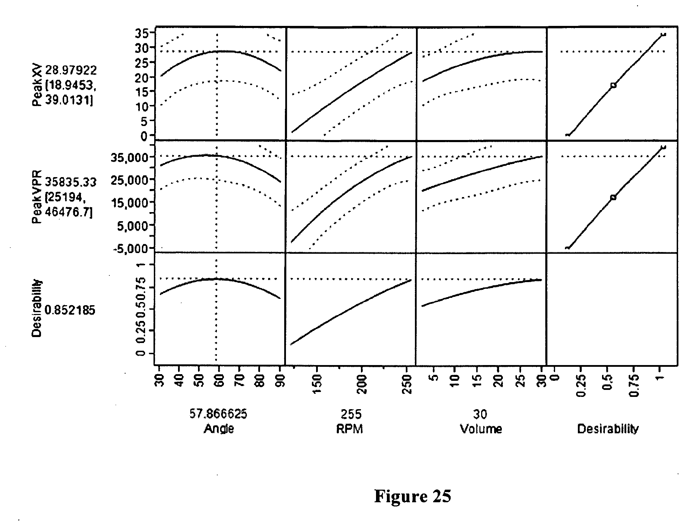

The JMP prediction profiler was used to estimate the best operating conditions. Based on the analysis, the best cell growth was to be obtained when operating with a working volume of 30 mL at 58°, while being agitated at 255 RPM (Figure 25). The profiler was used to determine the best operating ranges: a working volume of 20 to 30 mL at an angle of between 45° and 70°, while being agitated at greater than 200 RPM.

In a separate statistical analyses of the data generated, the experimental conditions were classified as "working" if the culture experiences at minimum a 1.5-fold increase in viable cell density (VCD $\geq 0.7 \times 10^6$ cells/mL with seeding density of $5 \times 10^5$ cells/mL) and maintained a percent cell viability of 85% or greater over the duration of the study (data not shown). The results of three experiments were grouped and categorized by peak viable cell density. A total of six categories were created (Table 18).

**Table 18. Working conditions categorized by peak viable cell densities**

| | | |
|---|---|---|
| **Group #1** | $0.7 \times 10^6$ cells/mL - $3 \times 10^6$ cells/mL | < 5°, 20 rpm, 2 mL |
| | | < 5°, 160 rpm, 2 mL |
| | | < 30°, 255 rpm, 2 mL |
| | | < 45°, 160 rpm, 2 mL |
| | | < 65°, 20 rpm, 2 mL |
| | | |
| **Group #2** | $3.0 \times 10^6$ cells/mL - $10 \times 10^6$ cells/mL | < 30°, 188 rpm, 2 mL |
| | | < 85°, 125 rpm, 2 mL |
| | | |
| **Group #3** | $10.0 \times 10^6$ cells/mL - $15 \times 10^6$ cells/mL | < 30°, 255 rpm, 30 mL |
| | | < 45°, 250 rpm, 35 mL |
| | | < 65°, 330 rpm, 35 mL |
| | | |
| **Group #4** | $15.0 \times 10^6$ cells/mL - $20 \times 10^6$ cells/mL | < 20°, 330 rpm, 20 mL |
| | | < 30°, 120 rpm, 2 mL |
| | | < 30°, 255 rpm, 16 mL |
| | | < 60°, 255 rpm, 2 mL |
| | | |

(continued)

| | | < 5°, 85 rpm, 10 mL |
|---|---|---|
| **Group #5** | 20.0 x 10⁶ cells/mL - 25 x 10⁶ cells/mL | < 45°, 160 rpm, 10 mL |
| | | < 45°, 160 rpm, 10 mL |
| | | < 60°, 188 rpm, 30 mL |
| | | < 65°, 250 rpm, 40 mL |
| | | |
| **Group #6** | 25.0 x 10⁶ cells/mL - 35 x 10⁶ cells/mL | < 5°, 260 rpm, 2 mL |
| | | < 85°, 330 rpm, 20 mL |

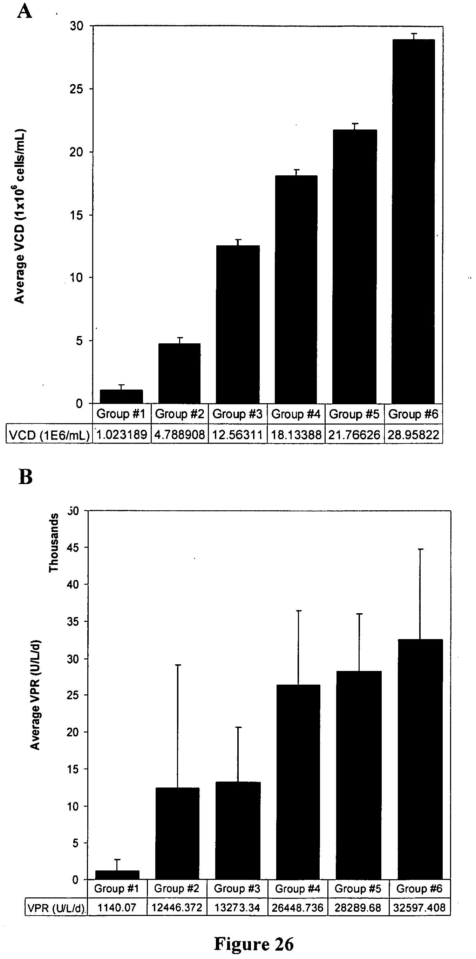

The viable cell density of working conditions range from $0.7 \times 10^6$ cells/mL to $30 \times 10^6$ cells/mL (Figure 26A). Productivity followed a similar trend to viable cell density, but the difference between groups were less pronounced. In general, however, conditions with viable cell density below $5 \times 10^6$ cells/mL experiences low productivity, whereas cultures with high viable cell density had high productivity (Figure 26B). Conditions that obtained a viable cell density above $15 \times 10^6$ cells/mL diminished the relationship- the average performance of the cells plateaued.

In sum, these data show that cultures with high viable cell density and productivity can be achieved over a broad range of different combinations of culture conditions using the methods described herein.

## Claims

**1.** A method of suspension culturing a Chinese hamster ovary (CHO) cell that grows in suspension, the method comprising:

providing a conical container containing a CHO cell that grows in suspension, suspended in a first liquid culture medium that occupies 10% to 30% of the volume of the container;
incubating the container for a period of time at 31 °C to 40 °C, wherein the container is placed at an angle of deviation of 40 degrees to 55 degrees from the horizontal position and with a rotary agitation of 140 revolutions per minute (RPM) to 180 RPM; and
continuously or periodically, during the period of time, removing a first volume of the first liquid culture medium and adding to the first liquid culture medium a second volume of a second liquid culture medium, wherein the first and second volumes are equal,
wherein the container is an elongated vessel that contains at least one end that is cone-shaped or hemispherical, has at least one gas permeable surface and/or vent cap, and has a volume of about 40 mL to about 60 mL;
the method thereby achieving a viable cell density of greater than $10 \times 10^6$ cells/mL in the first liquid culture medium or a combination of the first and second liquid culture medium.

**2.** The method of claim 1, wherein the CHO cell that grows in suspension comprises a nucleic acid that encodes a recombinant protein, and the method further comprises:
recovering the recombinant protein from the CHO cell that grows in suspension or from the first or second culture medium.

**3.** The method of claim 1, wherein the CHO cell that grows in suspension comprises a nucleic acid that encodes a recombinant protein, and the method further comprises:

detecting the recombinant protein in the CHO cell that grows in suspension or in the first or second culture medium; and
comparing the amount of recombinant protein present in the CHO cell that grows in suspension or in the first or second culture medium to a reference level of recombinant protein.

**4.** The method of any one of claims 1-3, wherein the first volume of the first liquid culture medium is substantially free of the CHO cells that grow in suspension.

**5.** The method of claim 1, wherein the CHO cell that grows in suspension comprises a nucleic acid encoding a recombinant protein.

**6.** The method of claim 2 or 3, wherein the recombinant protein is an immunoglobulin, an enzyme, a growth factor, a protein fragment, or an engineered protein.

**7.** The method of any one of claims 1-3, wherein the container is placed at an angle of deviation of 45 degrees from the horizontal position and with a rotary agitation of 160 RPM.

**8.** The method of any one of claims 1-3, wherein the removing of the first volume of the first liquid culture medium and the adding of the second volume of the second liquid culture medium is performed simultaneously.

**9.** The method of any one of claims 1-3, wherein the removing of the first volume of the first liquid culture medium and the adding of the second volume of the second liquid culture medium is performed continuously.

**10.** The method of any one of claims 1-3, wherein the removing of the first volume of the first liquid culture medium and the adding of the second volume of the second liquid culture medium is performed periodically.

**11.** The method of any one of claims 1-3, wherein the first volume of the first liquid culture medium removed and the second volume of the second liquid culture medium added are increased over time.

**12.** The method of claim 11, wherein:

the container is incubated for a period of time greater than 7 days, and on days 1 through 3 of incubation, in each 24-hour period, the first volume of the first liquid culture medium removed and the second volume of the second liquid culture medium added is 50% of the volume of the first liquid culture medium; on days 4 through 6 of incubation, in each 24-hour period, the first volume of the first liquid culture medium removed and the second volume of the second liquid culture medium added is 70% of the volume of the first liquid culture medium; and on day 7 and onwards of incubation, in each 24-hour period, the first volume of the first liquid culture medium removed and the second volume of the second liquid culture medium added is 100% of the volume of the first liquid culture medium.

**13.** The method of any one of claims 1-3, wherein the first liquid culture medium and/or second liquid culture medium is selected from the group consisting of: a chemically-defined liquid culture medium, a serum-free liquid culture medium, a serum-containing liquid culture medium, an animal-derived component-free liquid culture medium, and a protein-free medium.

**14.** The method of claim 13, wherein the first liquid culture medium is a first chemically-defined liquid culture medium and the second liquid culture medium is a second chemically-defined liquid culture medium.

**15.** The method of claim 3, wherein the reference level of recombinant protein is a level of recombinant protein produced using a different culturing method.

**Patentansprüche**

**1.** Verfahren zum Suspensionskultivieren einer Ovarialzelle des chinesischen Hamsters (CHO-Zelle), die in Suspension wächst, wobei das Verfahren umfasst:

Bereitstellen eines kegelförmigen Behälters, der eine CHO-Zelle enthält, die in Suspension wächst, suspendiert in einem ersten flüssigen Kulturmedium, das 10% bis 30 % des Volumens des Behälters einnimmt; Inkubieren des Behälters für einen Zeitraum bei 31 °C bis 40 °C, wobei der Behälter in einem Ablenkungswinkel von 40 Grad bis 55 Grad von der horizontalen Position und mit einer Drehagitation von 140 Umdrehungen pro Minute (U/min) bis 180 U/min platziert wird; und während des Zeitraums kontinuierliches oder periodisches Entfernen eines ersten Volumens des ersten flüssigen Kulturmediums und Hinzufügen eines zweiten Volumens eines zweiten flüssigen Kulturmediums zu dem ersten flüssigen Kulturmedium, wobei das erste und das zweite Volumen gleich sind,

wobei der Behälter ein längliches Gefäß ist, das zumindest ein Ende enthält, das kegelförmig oder halbkugelförmig ist, zumindest eine gasdurchlässige Oberfläche und/oder Lüftungskappe aufweist und ein Volumen von ungefähr 40 ml bis ungefähr 60 ml aufweist;

wobei das Verfahren dadurch eine Dichte lebensfähiger Zellen von mehr als $10 \times 10^6$ Zellen/ml in dem ersten flüssigen Kulturmedium oder eine Kombination aus dem ersten und dem zweiten flüssigen Kulturmedium erzielt.

2. Verfahren nach Anspruch 1, wobei die CHO-Zelle, die in Suspension wächst, eine Nukleinsäure umfasst, die für ein rekombinantes Protein codiert, und wobei das Verfahren ferner umfasst:
Gewinnen des rekombinanten Proteins aus der CHO-Zelle, die in Suspension wächst, oder aus dem ersten oder dem zweiten Kulturmedium.

3. Verfahren nach Anspruch 1, wobei die CHO-Zelle, die in Suspension wächst, eine Nukleinsäure umfasst, die für ein rekombinantes Protein codiert, und wobei das Verfahren ferner umfasst:

Nachweisen des rekombinanten Proteins in der CHO-Zelle, die in Suspension wächst, oder in dem ersten oder dem zweiten Kulturmedium; und
Vergleichen der Menge an rekombinantem Protein, das in der CHO-Zelle, die in Suspension wächst, oder in dem ersten oder dem zweiten Kulturmedium vorhanden ist, mit einem Referenzspiegel rekombinanten Proteins.

4. Verfahren nach einem der Ansprüche 1 bis 3, wobei das erste Volumen des ersten flüssigen Kulturmediums im Wesentlichen keine der CHO-Zellen, die in Suspension wachsen, aufweist.

5. Verfahren nach Anspruch 1, wobei die CHO-Zelle, die in Suspension wächst, eine Nukleinsäure umfasst, die für ein rekombinantes Protein codiert.

6. Verfahren nach Anspruch 2 oder 3, wobei das rekombinante Protein ein Immunglobulin, ein Enzym, ein Wachstumsfaktor, ein Proteinfragment oder ein manipuliertes Protein ist.

7. Verfahren nach einem der Ansprüche 1 bis 3, wobei der Behälter in einem Ablenkungswinkel von 45 Grad von der horizontalen Position und mit einer Drehagitation von 160 U/min platziert wird.

8. Verfahren nach einem der Ansprüche 1 bis 3, wobei das Entfernen des ersten Volumens des ersten flüssigen Kulturmediums und das Hinzufügen des zweiten Volumens des zweiten flüssigen Kulturmediums gleichzeitig durchgeführt werden.

9. Verfahren nach einem der Ansprüche 1 bis 3, wobei das Entfernen des ersten Volumens des ersten flüssigen Kulturmediums und das Hinzufügen des zweiten Volumens des zweiten flüssigen Kulturmediums kontinuierlich durchgeführt werden.

10. Verfahren nach einem der Ansprüche 1 bis 3, wobei das Entfernen des ersten Volumens des ersten flüssigen Kulturmediums und das Hinzufügen des zweiten Volumens des zweiten flüssigen Kulturmediums periodisch durchgeführt werden.

11. Verfahren nach einem der Ansprüche 1 bis 3, wobei das entfernte erste Volumen des ersten flüssigen Kulturmediums und das hinzugefügte zweite Volumen des zweiten flüssigen Kulturmediums mit der Zeit erhöht werden.

12. Verfahren nach Anspruch 11, wobei:

der Behälter für einen Zeitraum von mehr als 7 Tagen inkubiert wird und - an den Tagen 1 bis 3 der Inkubation in jedem 24-Stunden-Zeitraum - das entfernte erste Volumen des ersten flüssigen Kulturmediums und das hinzugefügte zweite Volumen des zweiten flüssigen Kulturmediums 50 % des Volumens des ersten flüssigen Kulturmediums sind;
an den Tagen 4 bis 6 der Inkubation in jedem 24-Stunden-Zeitraum das entfernte erste Volumen des ersten flüssigen Kulturmediums und das hinzugefügte zweite Kulturmedium 70 % des Volumens des ersten flüssigen Kulturmediums sind; und
an Tag 7 der Inkubation und danach in jedem 24-Stunden-Zeitraum das entfernte erste Volumen des ersten flüssigen Kulturmediums und das hinzugefügte zweite Kulturmedium 100 % des Volumens des ersten flüssigen Kulturmediums sind.

13. Verfahren nach einem der Ansprüche 1 bis 3, wobei das erste flüssige Kulturmedium und/oder das zweite flüssige Kulturmedium aus der Gruppe ausgewählt ist, bestehend aus: einem chemisch definierten flüssigen Kulturmedium, einem serumfreien flüssigen Kulturmedium, einem serumhaltigen flüssigen Kulturmedium, einem flüssigen Kulturmedium ohne von Tieren abgeleitete Komponenten und einem proteinfreien Medium.

14. Verfahren nach Anspruch 13, wobei das erste flüssige Kulturmedium ein erstes chemisch definiertes flüssiges Kulturmedium ist und das zweite flüssige Kulturmedium ein zweites chemisch definiertes flüssiges Kulturmedium ist.

15. Verfahren nach Anspruch 3, wobei der Referenzspiegel rekombinanten Proteins ein Spiegel eines rekombinanten Proteins ist, der unter Verwendung eines unterschiedlichen Kultivierungsverfahrens erhalten wird.


**Revendications**

1. Procédé de culture en suspension d'une cellule d'ovaire de hamster chinois (CHO) qui croît en suspension, le procédé comprenant :

   la mise à disposition d'un conteneur conique contenant une cellule CHO qui croît en suspension, mise en suspension dans un premier milieu de culture liquide qui occupe 10 % à 30 % du volume du conteneur ;
   l'incubation du conteneur pendant une certaine période entre 31 °C et 40 °C, où le conteneur est placé à un angle de déviation de 40 degrés à 55 degrés par rapport à la position horizontale et avec une agitation rotative de 140 tours par minutes (tr/min) à 180 tr/min ; et
   de manière continue ou périodique, pendant la période, le retrait d'un premier volume du premier milieu de culture liquide et l'ajout, au premier milieu de culture liquide, d'un second volume d'un second milieu de culture liquide, où les premier et second volumes sont équivalents,
   dans lequel le conteneur est un récipient allongé qui contient au moins une extrémité qui est en forme de cône ou hémisphérique, possède au moins une surface perméable aux gaz et/ou un bouchon de ventilation, et possède un volume d'environ 40 ml à environ 60 ml ;
   le procédé permettant ainsi d'obtenir une densité de cellules viables de plus de $10 \times 10^6$ cellules/ml dans le premier milieu de culture liquide ou une combinaison du premier et du second milieu de culture liquide.

2. Procédé selon la revendication 1, dans lequel la cellule CHO qui croît en suspension comprend un acide nucléique qui code pour une protéine recombinante, et le procédé comprend en outre :
   la récupération de la protéine recombinante à partir de la cellule CHO qui croît en suspension ou à partir du premier ou du second milieu de culture.

3. Procédé selon la revendication 1, dans lequel la cellule CHO qui croît en suspension comprend un acide nucléique qui code pour une protéine recombinante, et le procédé comprend en outre :

   la détection de la protéine recombinante dans la cellule CHO qui croît en suspension ou dans le premier ou le second milieu de culture ; et
   la comparaison de la quantité de protéine recombinante présente dans la cellule CHO qui croît en suspension ou dans le premier ou second milieu de culture avec un taux de référence de la protéine recombinante.

4. Procédé selon l'une quelconque des revendications 1 à 3, dans lequel le premier volume du premier milieu de culture liquide est essentiellement dépourvu des cellules CHO qui croissent en suspension.

5. Procédé selon la revendication 1, dans lequel la cellule CHO qui croît en suspension comprend un acide nucléique codant pour une protéine recombinante.

6. Procédé selon la revendication 2 ou 3, dans lequel la protéine recombinante est une immunoglobuline, une enzyme, un facteur de croissance, un fragment protéique, ou une protéine modifiée.

7. Procédé selon l'une quelconque des revendications 1 à 3, dans lequel le conteneur est placé à un angle de déviation de 45 degrés par rapport à la position horizontale et avec une agitation rotative de 160 tr/min.

8. Procédé selon l'une quelconque des revendications 1 à 3, dans lequel le retrait du premier volume du premier milieu de culture liquide et l'ajout du second volume du second milieu de culture liquide sont réalisés simultanément.

9. Procédé selon l'une quelconque des revendications 1 à 3, dans lequel le retrait du premier volume du premier milieu de culture liquide et l'ajout du second volume du second milieu de culture liquide sont réalisés de manière continue.

10. Procédé selon l'une quelconque des revendications 1 à 3, dans lequel le retrait du premier volume du premier milieu de culture liquide et l'ajout du second volume du second milieu de culture liquide sont réalisés de manière périodique.

11. Procédé selon l'une quelconque des revendications 1 à 3, dans lequel le premier volume du premier milieu de culture liquide retiré et le second volume du second milieu de culture liquide ajouté sont augmentés au cours du temps.

12. Procédé selon la revendication 11, dans lequel :

le conteneur est incubé pendant une période de plus de 7 jours, et aux jours 1 à 3 de l'incubation, dans chaque période de 24 heures, le premier volume du premier milieu de culture liquide retiré et le second volume du second milieu de culture liquide ajouté est 50 % du volume du premier milieu de culture liquide ;
aux jours 4 à 6 de l'incubation, dans chaque période de 24 heures, le premier volume du premier milieu de culture liquide retiré et le second volume du second milieu de culture liquide ajouté est 70 % du volume du premier milieu de culture liquide ; et
au jour 7 de l'incubation et par la suite, dans chaque période de 24 heures, le premier volume du premier milieu de culture liquide retiré et le second volume du second milieu de culture liquide ajouté est 100 % du volume du premier milieu de culture liquide.

13. Procédé selon l'une quelconque des revendications 1 à 3, dans lequel le premier milieu de culture liquide et/ou le second milieu de culture liquide est sélectionné dans le groupe consistant en : un milieu de culture liquide chimiquement défini, un milieu de culture liquide dépourvu de sérum, un milieu de culture liquide contenant du sérum, un milieu de culture liquide dépourvu de composants d'origine animale, et un milieu dépourvu de protéines.

14. Procédé selon la revendication 13, dans lequel le premier milieu de culture liquide est un premier milieu de culture liquide chimiquement défini et le second milieu de culture liquide est un second milieu de culture liquide chimiquement défini.

15. Procédé selon la revendication 3, dans lequel le taux de référence de la protéine recombinante est un taux de protéine recombinante produite en utilisant un procédé de culture différent.

FIG. 1

Figure 2

**Figure 3**

50

**Figure 4**

Figure 5

**Figure 6**

Figure 7

**Figure 8**

**Figure 9**

Figure 10

Figure 11

**Figure 12**

Figure 13

Figure 14

**Figure 15**

**Figure 16**

Figure 17

Figure 18

Figure 19

Figure 20

Figure 21

**Figure 22**

Figure 23

Figure 24

**Figure 25**

**A**

| | Group #1 | Group #2 | Group #3 | Group #4 | Group #5 | Group #6 |
|---|---|---|---|---|---|---|
| VCD (1E6/mL) | 1.023189 | 4.788908 | 12.56311 | 18.13388 | 21.76626 | 28.95822 |

**B**

| | Group #1 | Group #2 | Group #3 | Group #4 | Group #5 | Group #6 |
|---|---|---|---|---|---|---|
| VPR (U/L/d) | 1140.07 | 12446.372 | 13273.34 | 26448.736 | 28289.68 | 32597.408 |

**Figure 26**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61717486
- WO 2006138143 A
- WO 2009034186 A
- US 20120164066

**Non-patent literature cited in the description**

- **JURE STRNAD.** *Biotechnol. Prog.,* 2010, vol. 26 (3), 653-663
- **ROBERT A. GRANGER.** Fluid Mechanics. Dover Publications, Inc, 1995
- **BRUCE R. MUNSON et al.** Fundamentals of Fluid Mechanics. John Wiley & Sons, Inc, 2009
- **GEBAUER et al.** *Current Opin. Chem. Biol.,* 2009, vol. 13, 245-255
- Multitron Shaker incubator. Appropriate Technical Resources, Inc,
- Beckman Coulter Vi-Cell Cell Viability Analyzer. Beckman Coulter, Inc,
- YSI Biochemistry Analyzer. Yellow Springs Instruments, Inc,
- Osmometer. Advanced Instruments, Inc,
- Blood gas analyzer. Bayer AG